(19) Europäisches Patentamt
European Patent Office
Office européen des brevets

(11) EP 4 273 149 A1

(12) EUROPEAN PATENT APPLICATION
published in accordance with Art. 153(4) EPC

(43) Date of publication:
**08.11.2023 Bulletin 2023/45**

(21) Application number: **21915825.0**

(22) Date of filing: **29.12.2021**

(51) International Patent Classification (IPC):
***C07D 487/04*** *(2006.01)* ***A61K 31/519*** *(2006.01)*
***A61P 35/00*** *(2006.01)* ***C07D 495/04*** *(2006.01)*

(52) Cooperative Patent Classification (CPC):
**A61K 31/519; A61P 35/00; C07D 487/04; C07D 495/04**

(86) International application number:
**PCT/KR2021/020165**

(87) International publication number:
**WO 2022/146027 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.12.2020 KR 20200186082**

(71) Applicant: **Voronoi Inc.**
**Incheon 21984 (KR)**

(72) Inventors:
- **KO, Yi Kyung**
  **Incheon 21984 (KR)**
- **HAN, Ah Reum**
  **Incheon 21984 (KR)**
- **PARK, Jin Hee**
  **Incheon 21984 (KR)**
- **LEE, Yeong Deok**
  **Incheon 21984 (KR)**
- **IM, Hye Rim**
  **Incheon 21984 (KR)**
- **KIM, Kyun Eun**
  **Incheon 21984 (KR)**
- **HWANG, Dong Keun**
  **Incheon 21984 (KR)**
- **NAM, Su Been**
  **Incheon 21984 (KR)**
- **HEO, Myung Hoe**
  **Incheon 21984 (KR)**
- **CHO, Se Rin**
  **Incheon 21984 (KR)**
- **KO, Eun Hwa**
  **Incheon 21984 (KR)**
- **KIM, Sung Hwan**
  **Incheon 21984 (KR)**
- **CHOI, Hwan Geun**
  **Incheon 21984 (KR)**

(74) Representative: **Viering, Jentschura & Partner mbB**
**Patent- und Rechtsanwälte**
**Am Brauhaus 8**
**01099 Dresden (DE)**

# (54) HETEROARYL DERIVATIVE COMPOUND AND USE THEREOF

(57) The present invention relates to a heteroaryl derivative compound and a use thereof. Since the heteroaryl derivative of the present invention exhibits excellent inhibitory activity against EGFR, the heteroaryl derivative can be usefully used as a therapeutic agent for EGFR-associated diseases.



Processed by Luminess, 75001 PARIS (FR)

## Description

[Technical Field]

The present invention relates to a heteroaryl derivative compound and a medicinal use thereof. Specifically, the present invention relates to a heteroaryl derivative compound having inhibitory activity against EGFR.

[Background Art]

Protein kinases act as molecular switches to participate in signal transduction pathways, and the transition between active and inactive states of target proteins by kinases in cells should be smoothly controlled. If the transition between the active and inactive states is abnormally controlled, intracellular signal transduction is excessively activated or deactivated to induce uncontrollable cell division and proliferation. In particular, abnormal activation by mutation, amplification and/or overexpression of protein kinase genes causes the development and progression of various tumors or plays a crucial role in the development of various diseases such as inflammatory diseases, degenerative brain diseases, and autoimmune diseases.

In particular, the epidermal growth factor receptor (EGFR), which is a receptor tyrosine kinase of ErbB family, has been known to be abnormally active in many epithelial cell tumors, including non-small cell lung carcinoma (NSCLC), breast cancer, glioma, squamous cell carcinoma of the head and neck, colorectal cancer, rectal adenocarcinoma, head and neck cancer, stomach cancer and prostate cancer, and the activation of the EGFR-tyrosine kinase causes continuous cell proliferation, invasion of surrounding tissues, distant metastasis, and angiogenesis, and increases cell survival.

Meanwhile, it has been known that EGFR_del19 or EGFR_L858R, the EGFR mutation, is a major cause of non-small cell lung cancer and head and neck cancer, and therapeutic agents thereof, Iressa and Tarceva, have been developed and are currently used in clinical practice. However, when these drugs are used in patients, acquired resistance, which causes EGFR secondary mutations based on the structure of the drug, was observed, and it was also found that this resistance is a major cause of actual drug resistance. When first-generation EGFR inhibitors are used for about 10 months on average, acquired resistance, called the T790M mutation located in the gatekeeper of EGFR kinase, occurs, and thus the first-generation EGFR inhibitors show no drug efficacy. In other words, EGFR_del19_T790M or EGFR_L858R_T790M double mutations occur, preventing the existing therapeutic agents from exhibiting drug efficacy. In this regard, Osimertinib, a third-generation EGFR-TKI target drug that shows high reactivity against drug resistance due to the T790M mutation, has been developed, but this drug has also been reported to have drug resistance (Clin Cancer Res, 2015, 17:21) . The EGFR C797S mutation has been suggested as one of the main mechanisms that cause drug resistance to Osimertinib, and about 40% of clinical trial patients have been reported to have the C797S mutation (Nature Medicine, 2015, 21:560-562).

As described above, there is an increasing unmet need for novel compounds capable of being usefully utilized in the treatment of EGFR-associated diseases by modulating EGFR activity.

**[Disclosure]**

[Technical Problem]

An object of the present invention is to provide a heteroaryl derivative having a novel structure, an optical isomer thereof, or a pharmaceutically acceptable salt thereof.

Another object of the present invention is to provide a method for preparing the heteroaryl derivative compound.

Still another object of the present invention is to provide a pharmaceutical use of the heteroaryl derivative compound, specifically, a pharmaceutical composition for treating or preventing EGFR-associated diseases comprising the heteroaryl derivative compound as an active ingredient, a use of the compound for treatment or prevention of EGFR-associated diseases, or a method for treating or preventing EGFR-associated diseases comprising administering the compound.

[Technical Solution]

In order to achieve the above object, the present inventors have made research efforts, and as a result, completed the present invention by confirming that the heteroaryl derivative compounds represented by the following Chemical Formula 1 inhibited the proliferation of epidermal growth factor receptor (EGFR)-activated cells.

**Heteroaryl Derivative Compound**

The present invention provides a compound represented by the following Chemical Formula 1, an optical isomer thereof, or a pharmaceutically acceptable salt thereof:

[Chemical Formula 1]

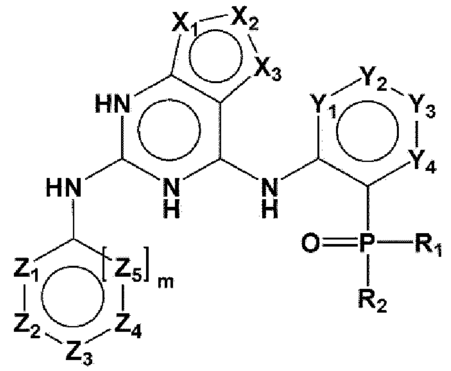

in Chemical Formula 1 above,

$X_1$ to $X_3$ are each independently $CR_aR_b$, $NR_c$, O, or S;

$R_a$ to $R_c$ are each independently -H, -$C_{1-6}$alkyl, -$C_{1-6}$aminoalkyl, -$C_{1-6}$hydroxyalkyl, -$C_{1-6}$haloalkyl, -CN, -$NH_2$, -OH, -O-$C_{1-6}$alkyl, -halo, -$C_{3-6}$cycloalkyl, or null;

$Y_1$ to $Y_4$ are each independently $CR_d$ or $NR_e$ {wherein any one of $Y_1$-$Y_2$, $Y_2$-$Y_3$, and $Y_3$-$Y_4$ may be linked to each other to form a 5-6 membered aryl, a 5-6 membered heteroaryl, or a 5-6 membered heterocycloalkyl [wherein at least one H of the aryl, heteroaryl, or heterocycloalkyl may be substituted with -$C_{1-6}$alkyl, -$C_{1-6}$aminoalkyl, -$C_{1-6}$hydroxyalkyl, -$C_{1-6}$haloalkyl, -CN, -$NR_3R_4$, -$OR_5$, -halo, or -$C_{3-6}$cycloalkyl]};

$R_d$ and $R_e$ are each independently -H, -$C_{1-6}$alkyl, -$C_{1-6}$aminoalkyl, -$C_{1-6}$hydroxyalkyl, -$C_{1-6}$haloalkyl, -CN, -$NR_3R_4$, - $OR_5$, -halo, -$C_{3-6}$cycloalkyl, or null;

$Z_1$ to $Z_5$ are each independently $CR_f$, $NR_g$ or N {wherein any one of $Z_1$-$Z_2$, $Z_2$-$Z_3$, $Z_3$-$Z_4$, and $Z_4$-$Z_5$ may be linked to each other to form a 5-6 membered aryl, a 5-6 membered heteroaryl or a 5-6 membered heterocycloalkyl [wherein at least one H of the aryl, heteroaryl, or heterocycloalkyl may be substituted with -$C_{1-6}$alkyl, -$C_{1-6}$aminoalkyl, -$C_{1-6}$hydroxyalkyl, -$C_{1-6}$haloalkyl, -CN, -$NR_3R_4$, -$OR_5$, -halo, or -$C_{3-6}$cycloalkyl]};

$R_f$ and $R_g$ are each independently -H, -$C_{1-6}$alkyl, -$C_{2-\varepsilon}$alkenyl, -$C_{2-6}$alkynyl, -$C_{1-6}$aminoalkyl, -$C_{1-6}$hydroxyalkyl, -$C_{1-6}$haloalkyl, -CN, -$NR_3R_4$, -$NO_2$, -$OR_5$, -halo, aryl, heteroaryl, -$C_{3-6}$cycloalkyl, or heterocycloalkyl {wherein at least one H of the aryl or heteroaryl may be substituted with -$C_{1-6}$alkyl, -$C_{2-6}$alkenyl, -$C_{2-6}$alkynyl, -$C_{1-6}$aminoalkyl, -$C_{1-6}$hydroxyalkyl, -$C_{1-6}$haloalkyl, -$C_{3-6}$cycloalkyl, heterocycloalkyl, phenyl, or heteroaryl, and at least one H of the -$C_{3-6}$cycloalkyl or heterocycloalkyl may be substituted with -$C_{1-6}$alkyl, -$C_{1-6}$aminoalkyl, -$C_{1-6}$hydroxyalkyl, -$C_{1-6}$haloalkyl, -$C_{3-6}$cycloalkyl, -$C_{1-6}$alkyl-$C_{3-6}$cycloalkyl, -C(=O)-$C_{3-6}$cycloalkyl, or heterocycloalkyl (wherein at least one H of the -$C_{3-6}$cycloalkyl, -$C_{1-6}$alkyl-$C_{3-6}$cycloalkyl, -C(=O)-$C_{3-6}$cycloalkyl, or heterocycloalkyl ring may be substituted with -$C_{1-6}$alkyl, -$C_{1-6}$aminoalkyl, -$C_{1-6}$hydroxyalkyl, -$C_{1-6}$haloalkyl, -$C_{3-6}$cycloalkyl, or -halo)]};

m is 0 or 1;

$R_1$ and $R_2$ may each independently be -H, -$C_{1-6}$alkyl, -$C_{1-6}$alkoxy, -$C_{1-6}$alkylamino, -$C_{2-12}$dialkylamino, or $C_{3-6}$cycloalkyl, or the $R_1$ and $R_2$ may be linked to each other to form a 4- to 7-membered ring together with the P atom;

$R_3$ and $R_4$ are each independently -H, -$C_{1-6}$alkyl, -$C_{1-6}$alkyl-$NR_6R_7$, -$C_{1-6}$alkyl-O-$C_{1-6}$alkyl, -C(=O)-$C_{1-6}$alkyl, or -C(=O)-$C_{2-6}$alkenyl;

$R_5$ is -H, -$C_{1-6}$alkyl, -$C_{1-6}$alkyl-$NR_6R_7$, -$C_{1-6}$aminoalkyl, - $C_{1-6}$hydroxyalkyl, or -$C_{1-6}$haloalkyl; and

$R_6$ and $R_7$ are each independently -H or -$C_{1-6}$alkyl.

According to an embodiment of the present invention, the compound represented by Chemical Formula 1, an optical isomer thereof, or a pharmaceutically acceptable salt thereof may be in the following ranges:

$X_1$ to $X_3$ are each independently $CR_aR_b$, $NR_c$, or S;

$R_a$ to $R_c$ are each independently -H, -$C_{1-6}$alkyl, -$C_{1-6}$haloalkyl, -CN, -halo, -$C_{3-6}$cycloalkyl, or null;

$Y_1$ to $Y_4$ are each independently $CR_d$ or $NR_e$ {wherein any one of $Y_1$-$Y_2$, $Y_2$-$Y_3$, and $Y_3$-$Y_4$ may be linked to each

other to form a 5-6 membered aryl, a 5-6 membered heteroaryl, or a 5-6 membered heterocycloalkyl [wherein at least one H of the aryl, heteroaryl, or heterocycloalkyl may be substituted with -$C_{1-6}$alkyl, -$C_{1-6}$haloalkyl, -halo, or -$C_{3-6}$cycloalkyl]};

$R_d$ and $R_e$ are each independently -H, -$C_{1-6}$alkyl, or null;

$Z_1$ to $Z_5$ are each independently $CR_f$, $NR_g$ or N {wherein any one of $Z_1$-$Z_2$, $Z_2$-$Z_3$, $Z_3$-$Z_4$, and $Z_4$-$Z_5$ may be linked to each other to form a 5-6 membered heterocycloalkyl [wherein at least one H of the heterocycloalkyl may be substituted with -$C_{1-6}$alkyl, -$C_{1-6}$haloalkyl, -halo, or -$C_{3-6}$cycloalkyl]};

$R_f$ and $R_g$ are each independently -H, -$C_{1-6}$alkyl, -$C_{2-\varepsilon}$alkenyl, -$C_{2-6}$alkynyl, -$C_{1-6}$haloalkyl, -CN, -$NR_3R_4$, -$NO_2$, -$OR_5$, -halo, aryl, heteroaryl, -$C_{3-6}$cycloalkyl, or heterocycloalkyl {wherein at least one H of the aryl or heteroaryl may be substituted with -$C_{1-6}$alkyl, -$C_{1-6}$haloalkyl, or -$C_{3-6}$cycloalkyl, and at least one H of the -$C_{3-6}$cycloalkyl or heterocycloalkyl may be substituted with -$C_{1-6}$alkyl, -$C_{1-6}$hydroxyalkyl, -$C_{1-6}$haloalkyl, -$C_{3-6}$cycloalkyl, -$C_{1-6}$alkyl-$C_{3-6}$cycloalkyl, -C(=O)-$C_{3-6}$cycloalkyl, or heterocycloalkyl (wherein at least one H of the -$C_{3-6}$cycloalkyl, -$C_{1-6}$alkyl-$C_{3-6}$cycloalkyl, -C(=O)-$C_{3-6}$cycloalkyl, or heterocycloalkyl ring may be substituted with -$C_{1-6}$alkyl, -$C_{3-6}$cycloalkyl, or -halo)]};

m is 0 or 1;

$R_1$ and $R_2$ are each independently -$C_{1-6}$alkyl;

$R_3$ and $R_4$ are each independently -H, -$C_{1-6}$alkyl, -$C_{1-6}$alkyl-$NR_6R_7$, -$C_{1-6}$alkyl-O-$C_{1-6}$alkyl, -C (=O)-$C_{1-6}$alkyl, or -C(=O)-$C_{2-6}$alkenyl;

$R_5$ is -H, -$C_{1-6}$alkyl, -$C_{1-6}$alkyl-$NR_6R_7$, or -$C_{1-6}$haloalkyl; and

$R_6$ and $R_7$ are each independently -H or -$C_{1-6}$alkyl.

According to another embodiment of the present invention, the compound represented by Chemical Formula 1, an optical isomer thereof, or a pharmaceutically acceptable salt thereof may be in the following ranges:

$X_1$ is $CR_aR_b$, $NR_c$, or S;

$X_2$ is $CR_aR_b$;

$X_3$ is $CR_aR_b$, $NR_c$, or S; and

$R_a$ to $R_c$ are each independently -H, -$C_{1-6}$haloalkyl, -CN, -halo, -$C_{3-6}$cycloalkyl, or null.

According to another embodiment of the present invention, the compound represented by Chemical Formula 1, an optical isomer thereof, or a pharmaceutically acceptable salt thereof may be in the following ranges:

$Y_1$ to $Y_4$ are each independently $CR_d$ or $NR_e$ {wherein any one of $Y_1$-$Y_2$, $Y_2$-$Y_3$, and $Y_3$-$Y_4$ may be linked to each other to form a 5-6 membered aryl, a 5-6 membered heteroaryl, or a 5-6 membered heterocycloalkyl [wherein at least one H of the aryl, heteroaryl, or heterocycloalkyl may be substituted with -$C_{1-6}$alkyl, -$C_{1-6}$haloalkyl, -halo, or -$C_{3-6}$cycloalkyl]}; and

$R_d$ and $R_e$ are each independently -H, -$C_{1-6}$alkyl, or null.

According to another embodiment of the present invention, the compound represented by Chemical Formula 1, an optical isomer thereof, or a pharmaceutically acceptable salt thereof may be in the following ranges:

$Z_1$ to $Z_5$ are each independently $CR_f$, $NR_g$ or N {wherein $Z_1$-$Z_2$ may be linked to each other to form a 5-6 membered heterocycloalkyl [wherein at least one H of the heterocycloalkyl may be substituted with -$C_{1-6}$alkyl, -$C_{1-6}$haloalkyl, or -halo]};

$R_f$ and $R_g$ are each independently -H, -$C_{1-6}$alkyl, -$C_{2-6}$alkenyl, -$C_{2-6}$alkynyl, -$C_{1-6}$haloalkyl, -CN, -$NR_3R_4$, -$NO_2$, -$OR_5$, -halo, aryl, heteroaryl, -$C_{3-6}$cycloalkyl, or heterocycloalkyl {wherein at least one H of the aryl or heteroaryl may be substituted with -$C_{1-6}$alkyl, -$C_{1-6}$haloalkyl, -$C_{3-6}$cycloalkyl, heterocycloalkyl, phenyl, or heteroaryl, and at least one H of the -$C_{3-6}$cycloalkyl or heterocycloalkyl may be substituted with -$C_{1-6}$alkyl, -$C_{1-6}$hydroxyalkyl, -$C_{1-6}$haloalkyl, -$C_{3-6}$cycloalkyl, -$C_{1-6}$alkyl-$C_{3-6}$cycloalkyl, -C(=O)-$C_{3-6}$cycloalkyl, or heterocycloalkyl (wherein at least one H of the -$C_{3-6}$cycloalkyl, -$C_{1-6}$alkyl-$C_{3-6}$cycloalkyl, -C(=O)-$C_{3-6}$cycloalkyl, or heterocycloalkyl ring may be substituted with -$C_{1-6}$alkyl, -$C_{3-6}$cycloalkyl, or -halo)]};

$R_3$ and $R_4$ are each independently -H, -$C_{1-6}$alkyl, -$C_{1-6}$alkyl-$NR_6R_7$, -$C_{1-6}$alkyl-O-$C_{1-6}$alkyl, -C(=O)-$C_{1-6}$alkyl, or -C(=O)-$C_{2-6}$alkenyl;

$R_5$ is -$C_{1-6}$alkyl, -$C_{1-6}$alkyl-$NR_6R_7$, or -$C_{1-6}$haloalkyl; and

$R_6$ and $R_7$ are each independently -H or -$C_{1-6}$alkyl.

According to another embodiment of the present invention, the compound represented by Chemical Formula 1, an optical isomer thereof, or a pharmaceutically acceptable salt thereof may be in the following ranges:

$R_1$ and $R_2$ are each independently -$C_{1-6}$alkyl.

According to another embodiment of the present invention, the compound represented by Chemical Formula 1 may be selected from the group consisting of compounds listed in Table 1 described below.

In the present invention, "alkyl" may refer to a straight-chain or branched-chain acyclic, cyclic, or saturated hydrocarbon to which they are bonded, unless otherwise specified. For example, "$C_{1-6}$ alkyl" may mean an alkyl containing 1 to 6 carbon atoms. The acyclic alkyl may include, for example, methyl, ethyl, n-propyl, n-butyl, isopropyl, sec-butyl, isobutyl, tert-butyl, or the like, but is not limited thereto. The cyclic alkyl may be used interchangeably with "cycloalkyl" in the present specification, and may include, as an example, cyclopropyl, cyclobutyl, cyclopentyl, cyclohexyl, cycloheptyl, cyclooctyl, or the like, but is not limited thereto.

In the present invention, "alkoxy" may refer to -(O-alkyl) as an alkyl ether group, wherein the alkyl is the same as defined above. For example, "$C_{1-6}$ alkoxy" may mean an alkoxy containing $C_{1-6}$ alkyl, i.e. -(O-$C_{1-6}$ alkyl), and as an example, the alkoxy may include, but is not limited to, methoxy, ethoxy, n-propoxy, isopropoxy, n-butoxy, isobutoxy, sec-butoxy, tert-butoxy, or the like.

In the present invention, "halo" may be F, Cl, Br, or I.

In the present invention, "haloalkyl" may refer to a straight-chain or branched-chain alkyl (hydrocarbon) having carbon atoms substituted with at least one halo as defined herein. Examples of the haloalkyl include methyl, ethyl, propyl, isopropyl, isobutyl or n-butyl independently substituted with at least one halogen such as F, Cl, Br, or I, but are not limited thereto.

In the present invention, "aminoalkyl" may refer to a straight-chain or branched-chain alkyl (hydrocarbon) having carbon atoms substituted with amino-(NR'R"). Here, R' and R" may each independently be selected from the group consisting of hydrogen and $C_{1-6}$ alkyl, and the selected R' and R" may each independently be substituted or unsubstituted.

In the present invention, "heterocycloalkyl" may refer to a ring containing 1 to 5 heteroatoms selected from N, O and S as atoms forming the ring, and may be saturated or partially unsaturated. Here, when unsaturated, the heterocycloalkyl may be referred to as a heterocycloalkene. Unless otherwise stated, the heterocycloalkyl may be a single ring or multiple rings such as spiro rings, bridged rings or fused rings. Further, "3- to 12-membered heterocycloalkyl" may mean a heterocycloalkyl containing 3 to 12 atoms forming a ring, and as an example, the heterocycloalkyl may include pyrrolidine, piperidine, imidazolidine, pyrazolidine, butyrolactam, valerolactam, imidazolidinone, hydantoin, dioxolane, phthalimide, piperidine, pyrimidin-2,4(1H,3H)-dione, 1,4-dioxane, morpholine, thiomorpholine, thiomorpholine-S-oxide, thiomorpholine-*S,S*-oxide, piperazine, pyran, pyridone, 3-pyrroline, thiopyran, pyrone, tetrahydrofuran, tetrahydrothiophene, quinuclidine, tropane, 2-azaspiro[3.3]heptane, (1R,5S)-3-azabicyclo[3.2.1]octane, (1*s*,4*s*)-2-azabicyclo[2.2.2]octane, (1R,4R)-2-oxa-5-azabicyclo[2.2.2]octane, or the like, but are not limited thereto.

In the present invention, "arene" may refer to an aromatic hydrocarbon ring. The arene may be monocyclic arene or polycyclic arene. The number of carbon atoms forming the arene ring may be 5 or more and 30 or less, 5 or more and 20 or less, or 5 or more and 15 or less. Examples of arene may include benzene, naphthalene, fluorene, anthracene, phenanthrene, bibenzene, terbenzene, quaterbenzene, quinquebenzene, sexibenzene, triphenylene, pyrene, benzofluoranthene, chrysene, and the like, but are not limited thereto. In the present specification, a residue obtained by removing one hydrogen atom from the above "arene" is referred to as "aryl".

In the present invention, "heteroarene" may be a ring containing one or more of O, N, P, Si, and S as heterogeneous elements. The number of carbon atoms forming the heteroarene ring may be 2 or more and 30 or less, or 2 or more and 20 or less. The heteroarene may be a monocyclic heteroarene or a polycyclic heteroarene. The polycyclic heteroarene may have, for example, a bicyclic or tricyclic structure. Examples of the heteroarene include thiophene, purine, pyrrole, pyrazole, imidazole, thiazole, oxazole, isothiazole, oxadiazole, triazole, pyridine, bipyridyl, triazine, acridyl, pyridazine, pyrazine, quinoline, quinazoline, quinoxaline, phenoxazine, phthalazine, pyrimidine, pyridopyrimidine, pyridopyrazine, pyrazinopyrazine, isoquinoline, indole, carbazole, imidazopyridazine, imidazopyridine, imidazopyrimidine, pyrazolopyrimidine, imidazopyrazine or pyrazolopyridine, *N*-arylcarbazole, *N*-heteroarylcarbazole, *N*-alkylcarbazole, benzoxazole, benzoimidazole, benzothiazole, benzocarbazole, benzothiophene, dibenzothiophene, thienothiophene, benzofuran, phenanthroline, isoxazole, oxadiazole, thiadiazole, benzothiazole, tetrazole, phenothiazine, dibenzosilole, dibenzofuran, and the like, but not limited thereto. In an embodiment of the present invention, the heteroarene may also include a bicyclic heterocyclo-arene including an arene ring fused to a heterocycloalkyl ring or a heteroarene fused to a cycloalkyl ring. In the present specification, a residue obtained by removing one hydrogen atom from the above "heteroarene" is referred to as "heteroaryl".

In the present invention, the term "optical isomer (enantiomer)" means a compound of the present invention or a salt thereof, having the same chemical formula or molecular formula but sterically different. Each of these optical isomers and mixtures thereof are also included within the scope of the present invention. Unless otherwise specified, a solid bond (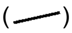) connected to an asymmetric carbon atom may include the solid wedge (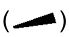) or dashed wedge (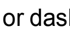) representing the absolute arrangement of stereocenters.

The compound of Chemical Formula 1 of the present invention may be present in the form of a "pharmaceutically

acceptable salt". As the salt, an acid addition salt formed by a pharmaceutically acceptable free acid is useful. The term "pharmaceutically acceptable salt" of the present invention refers to a concentration having a relatively nontoxic and harmless effective effect on patients, which includes any organic acid or inorganic acid addition salt of the compound represented by Chemical Formula 1 in which side effects caused by these salts do not reduce the beneficial efficacy of the compound.

Acid addition salts are prepared by conventional methods, for example, by dissolving a compound in an excess of an aqueous acid solution and precipitating the salt using a water-miscible organic solvent, such as methanol, ethanol, acetone or acetonitrile. Here, the acid or alcohol in water and the compound in equimolar amounts may be heated, and then the mixture may be evaporated to dryness, or the precipitated salt may be suction filtered.

Here, the free acid may be an organic acid or an inorganic acid, wherein the inorganic acid may be hydrochloric acid, phosphoric acid, sulfuric acid, nitric acid, or the like, and the organic acid may be methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, or hydroiodic acid, or the like. However, they are not limited thereto.

Further, a pharmaceutically acceptable metal salt may be prepared using a base. The alkali metal salt or alkaline earth metal salt is obtained, for example, by dissolving the compound in an excess of alkali metal hydroxide or alkaline earth metal hydroxide solution, filtering the undissolved compound salt, and then evaporating and drying the filtrate. Here, as the metal salt, sodium, potassium, or calcium salt is particularly suitable in a pharmaceutical aspect, but the metal salt is not limited thereto. In addition, the corresponding silver salt may be obtained by reacting an alkali metal or alkaline earth metal salt with a suitable silver salt (for example, silver nitrate).

The pharmaceutically acceptable salt of the present invention, unless otherwise indicated, includes salts of acidic or basic groups that may be present in the compounds of Chemical Formula 1 above. For example, the pharmaceutically acceptable salts may include sodium, calcium, and potassium salts, and the like of hydroxy groups, and may include other pharmaceutically acceptable salts of amino groups such as hydrobromide, sulfate, hydrogen sulfate, phosphate, hydrogen phosphate, dihydrogen phosphate, acetate, succinate, citrate, tartrate, lactate, mandelate, methanesulfonate (mesylate), and p-toluenesulfonate (tosylate) salts, and the like, which may be prepared through salt preparation methods known in the art.

**<u>Use of heteroaryl derivative compound</u>**

The present invention provides use of a compound represented by the following Chemical Formula 1, an optical isomer thereof, or a pharmaceutically acceptable salt thereof:

[Chemical Formula 1]

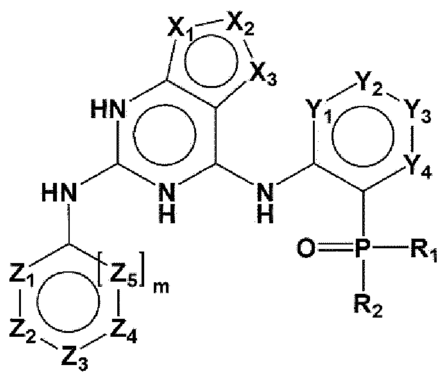

the Chemical Formula 1 is the same as defined above.

The compound represented by Chemical Formula 1 of the present invention, an optical isomer thereof, or a pharmaceutically acceptable salt thereof exhibits inhibitory activity against various kinases (Experimental Example 1).

According to an embodiment of the present invention, the heteroaryl derivative represented by Chemical Formula 1 may exhibit excellent inhibitory activity against EGFR (Epidermal Growth Factor Receptor) among several kinases, thereby being usefully employed for treatment or prevention of EGFR-associated diseases, in particular, cancer. Specifically, the compound of Chemical Formula 1 may inhibit EGFR wild-type or mutant kinase, which is supported by Experimental Examples to be described below. The EGFR mutation may be one or more selected from the group consisting of EGFR Del19/T790M, EGFR Del19/T790M/C797S, EGFR L858R/T790M, EGFR L858R, EGFR Exon20

ins NPH, EGFR Exon20 ins SVD, EGFR Exon20 ins FQEA, EGFR Exon20 ins H, and EGFR Exon20 ins ASV, but is not limited thereto.

In the present invention, the cancer includes all cancers capable of exhibiting therapeutic or preventive efficacy due to inhibition of EGFR activity, and may be solid cancer or hematological cancer. For example, the cancer may be one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of vater cancer, bladder cancer, peritoneal cancer, parathyroid cancer, adrenal cancer, sinonasal cancer, non-small cell lung cancer, tongue cancer, astrocytoma, small cell lung cancer, childhood brain cancer, childhood lymphoma, childhood leukemia, small intestine cancer, meningioma, esophageal cancer, glioma, renal pelvic cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoma, gastrointestinal stromal cancer, Wilms' cancer, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoma, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleural cancer, hematological cancer, and thymic cancer, but is not limited thereto. In addition, the cancer includes not only primary cancer but also metastatic cancer.

According to an embodiment of the present invention, the present invention provides a pharmaceutical composition for treating or preventing EGFR-associated diseases, comprising the compound represented by Chemical Formula 1, an optical isomer thereof, or a pharmaceutically acceptable salt thereof as an active ingredient. Specifically, the EGFR-associated disease may be cancer. The type of cancer is as described above.

The pharmaceutical composition of the present invention may further include at least one active ingredient exhibiting the same or similar efficacy in addition to the compound represented by Chemical Formula 1, an optical isomer thereof, or a pharmaceutically acceptable salt thereof.

In addition, according to an embodiment of the present invention, provided is a method for treating or preventing EGFR-associated diseases, comprising administering a therapeutically effective amount of the compound represented by Chemical Formula 1, an optical isomer thereof, or a pharmaceutically acceptable salt thereof to a subject in need thereof. The subject may be a mammal including a human.

The term "therapeutically effective amount" used in the present invention refers to an amount of the compound represented by Chemical Formula 1 effective for the treatment or prevention of EGFR-associated diseases. Specifically, the "pharmaceutically effective amount" means an amount sufficient to treat a disease at a reasonable benefit/risk ratio applicable to medical treatment, and the effective dose level may be determined depending on factors including the type and severity of the subject, age, sex, the type of disease, the activity of the drug, the sensitivity to the drug, the time of administration, the route of administration and the rate of excretion, the duration of treatment, drugs used concurrently, and other factors well known in the medical field. The pharmaceutical composition of the present invention may be administered as an individual therapeutic agent or in combination with other therapeutic agents, or may be administered sequentially or simultaneously with a commercially available therapeutic agent. In addition, the pharmaceutical composition of the present invention may be administered singly or in multiple doses. In consideration of all of the above factors, it is important to administer an amount capable of obtaining the maximum effect with the minimum amount without side effects, which may be easily determined by those skilled in the art. The administration dose of the pharmaceutical composition of the present invention may be determined by specialists depending on various factors such as the patient's condition, age, sex, complications, and the like. Since the active ingredient of the pharmaceutical composition of the present invention has excellent safety, the active ingredient may be used even above the determined administration dose.

In addition, according to an embodiment of the present invention, provided is use of the compound represented by Chemical Formula 1, an optical isomer thereof, or a pharmaceutically acceptable salt thereof for use in the manufacture of a medicament for use in the treatment or prevention of EGFR-associated diseases. The compound represented by Chemical Formula 1 for the manufacture of medicaments may be mixed with acceptable adjuvants, diluents, carriers, and the like, and may be prepared as a combined preparation with other active agents to have a synergistic effect of the active ingredients.

Matters described in the use, composition, and treatment method of the present invention are equally applied unless they contradict each other.

Embodiments of the present invention may be modified in various other forms, and the scope of the present invention is not limited to the embodiments described below. In addition, the embodiments of the present invention are

provided to more completely explain the present invention to those skilled in the art. Further, "including" a component throughout the specification does not mean excluding other components, but rather it means that other components may be further included, unless otherwise stated.

[Advantageous Effects]

The heteroaryl derivative compound of the present invention may exhibit excellent inhibitory activity against EGFR, which may be usefully employed for the treatment or prevention of EGFR-associated diseases.

[Best Mode]

Hereinafter, the present invention will be described in detail by Examples and Experimental Examples. However, the following Examples and Experimental Examples are only provided to illustrate the present invention, and the scope of the present invention is not limited thereto.

**<Analysis and purification conditions>**

1. HPLC analysis conditions

(a) Instrument : Waters e2695

Column : Xbridge C18, 4.6 x 150 mm, 5 um, 40°C
Mobile phase : 20% -> 95% acetonitrile/$H_2O$ + 0.1% TFA
Analysis time : 10 minutes, flow rate : 1 mL/min
UV detector: 254 nm

(b) Instrument : Waters e2695

Column : YMC-Pack ODS-AM, 150 x 4.6 mm, 3 um, 12 nm, 40°C
Mobile phase : 10 % -> 90 % acetonitrile/$H_2O$ + 0.1 % TFA
Analysis time : 20 minutes, flow rate : 1 mL/min
UV detector: 254 nm

2. LC-MS analysis conditions

Instrument : Waters AQUITY UPLC
Column : AQUITY UPLC® BEH C18, 50 x 2.1 mm, 5 um, 40°C
Mobile phase : acetonitrile/$H_2O$ + 0.1% TFA
Flow rate : 0.6 mL/min
UV detector: 254 nm

3. MPLC analysis conditions

Instrument : CombiFlash® Rf+
UV detector: 254 nm

4. Prep-HPLC purification conditions (A)

Instrument : Waters GX-281, waters 2555 pump, waters 2998 photodiod array detector
Column : XBridge® Prep Shield RP18, 250 x 30 mm, 10 um
Mobile phase : acetonitrile/0.1% TFA $H_2O$
Flow rate : 30 mL/min
UV detector: 254 nm

5, Prep-HPLC purification conditions (B)

Instrument : ACCQPrep HP125
Column : XBridge® Prep Shield RP18, 250 x 19 mm, 10 um
Mobile phase : acetonitrile/0.1% TFA $H_2O$
Flow rate : 25 mL/min
UV detector: 254 nm

6. Prep-HPLC purification conditions (C)

Instrument : ACCQPrep HP125
Column : XBridge® Prep Shield RP18, 250 x 19 mm, 10 um
Mobile phase : acetonitrile/0.1 % FA $H_2O$
Flow rate : 25 mL/min
UV detector: 254 nm

7. $^1H$ NMR

Instrument : Bruker Ascend™ 400 (400 MHz)
Commercial reagents used in the experiments were used without further purification. In the present invention, ambient temperature means a temperature of about 20 ~ 25°C, and room temperature means a temperature of 15 ~ 25°C. Concentration under reduced pressure or solvent distillation removal was performed using a rotary evaporator.

**Preparation Example 1: Preparation of 5-(1-methyl-1H-pyrazol-4-yl)-4-(4-methylpiperazin-1-yl)-2-(2,2,2-trifluoroethoxy)aniline**

The title compound was prepared by the method shown in Reaction Scheme 1 below.

[Reaction Scheme 1]

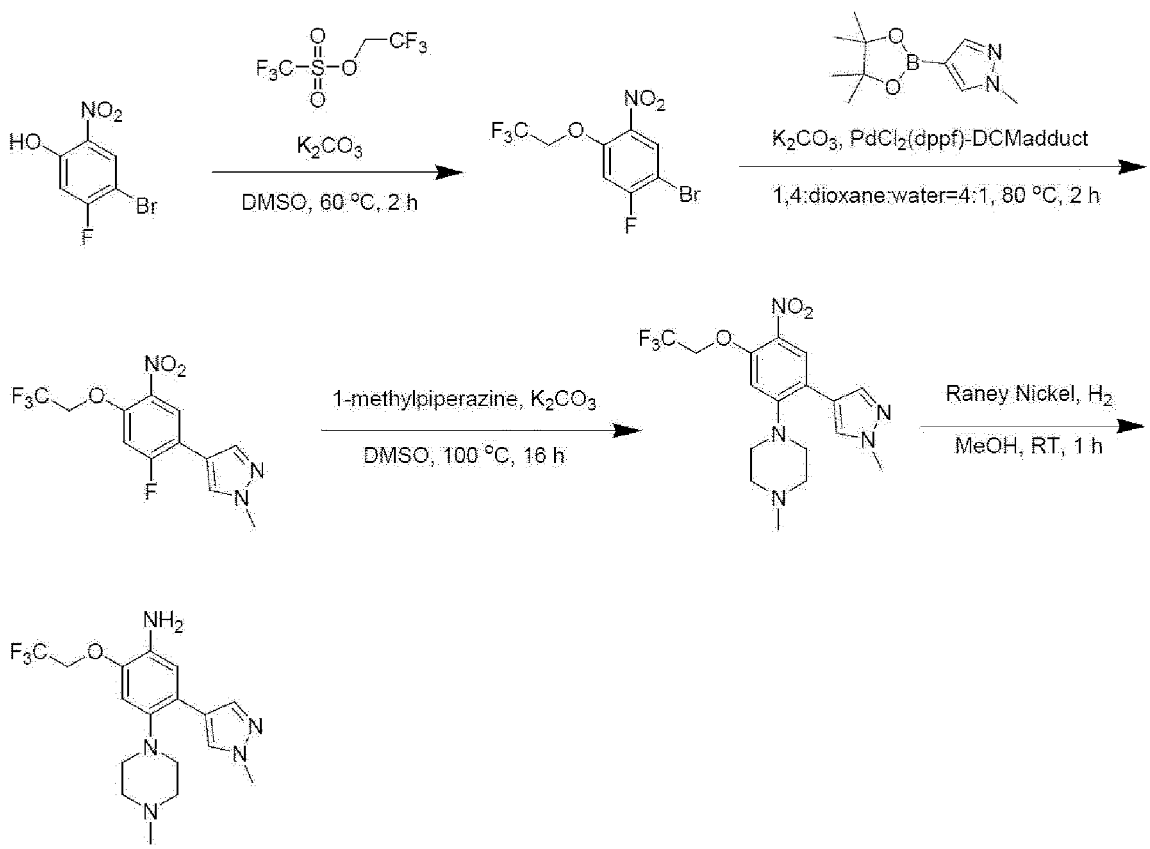

Step 1: Preparation of 1-bromo-2-fluoro-5-nitro-4-(2,2,2-trifluoroethoxy)benzene

4-Bromo-5-fluoro-2-nitrophenol (1 eq.), 2,2,2-trifluoroethyl trifluoromethanesulfonate (1.5 eq.), and $K_2CO_3$ (2 eq.) were dissolved in DMSO (0.4 M), and stirred at 60°C for 2 hours. Water and brine were added, organic materials were extracted with EtOAc, and the collected organic layers were concentrated by removing remaining water using $Na_2SO_4$. The reaction mixture was purified through MPLC (Hexane:EtOAc) to obtain the target compound as a yellow liquid (yield: 75%, MS (ESI): m/z 317 $[M+H]^+$).

Step 2: Preparation of 4-(2-fluoro-5-nitro-4-(2,2,2-trifluoroethoxy)phenyl)-1-methyl-1H-pyrazole

1-Bromo-2-fluoro-5-nitro-4-(2,2,2-trifluoroethoxy) benzene (1 eq.) prepared in step 1, 1-methyl-4-(4,4,5,5-tetramethyl-1,3,2-dioxaborolan-2-yl)-1H-pyrazole (1.5 eq.), and $K_2CO_3$ (2 eq.) were dissolved in 1,4-dioxane:water = 4:1 (0.2 M), and $PdCl_2$(dppf)-DCM adduct(0.1 eq.) was added under nitrogen, and stirred at 80°C for 2 hours. The stirred mixture was filtered through celite and then concentrated. The reaction mixture was purified through MPLC (DCM:MeOH) to obtain the target compound as a yellow solid (yield: 66%, MS (ESI): m/z 320 $[M+H]^+$).

Step 3: Preparation of 1-methyl-4-(2-(1-methyl-1H-pyrazol-4-yl)-4-nitro-5-(2,2,2-trifluoroethoxy)phenyl)piperazine

4-(2-Fluoro-5-nitro-4-(2,2,2-trifluoroethoxy)phenyl)-1-methyl-1H-pyrazole (1 eq.) prepared in step 2, and 1-methylpiperazine (2 eq.) and $K_2CO_3$ (6 eq.) were dissolved in DMSO (0.17 M), and stirred at 100°C for 16 hours. Water and NaHCOs were added, and organic materials were extracted with EtOAc. The collected organic layers were concentrated by removing remaining water using $Na_2SO_4$. The target compound of the obtained yellow liquid was used in the next reaction without further purification (yield: 100%, MS (ESI): m/z 332 $[M+H]^+$).

Step 4: Preparation of 5-(1-methyl-1H-pyrazol-4-yl)-4-(4-methylpiperazin-1-yl)-2-(2,2,2-trifluoroethoxy)aniline

1-Methyl-4-(2-(1-methyl-1H-pyrazol-4-yl)-4-nitro-5-(2,2,2-trifluoroethoxy)phenyl)piperazine (1 eq.) prepared in step 3, and Raney Ni (5 eq.) were dissolved in MeOH (0.2 M), followed by stirring under hydrogen at room temperature for 1 hour. The stirred mixture was filtered through celite, and the filtrate was concentrated. The target compound of the obtained purple liquid was used in the next reaction without further purification (yield: 95%, MS (ESI): m/z 370 $[M+H]^+$).

**Preparation Example 2: Preparation of 4-(9-((1-fluorocyclopropyl)methyl)-3,9-diazaspiro[5.5]undecan-3-yl)-2-methoxy-5-(1-methyl-1H-pyrazol-4-yl)aniline**

The title compound was prepared by the method shown in Reaction Scheme 2 below.

[Reaction Scheme 2]

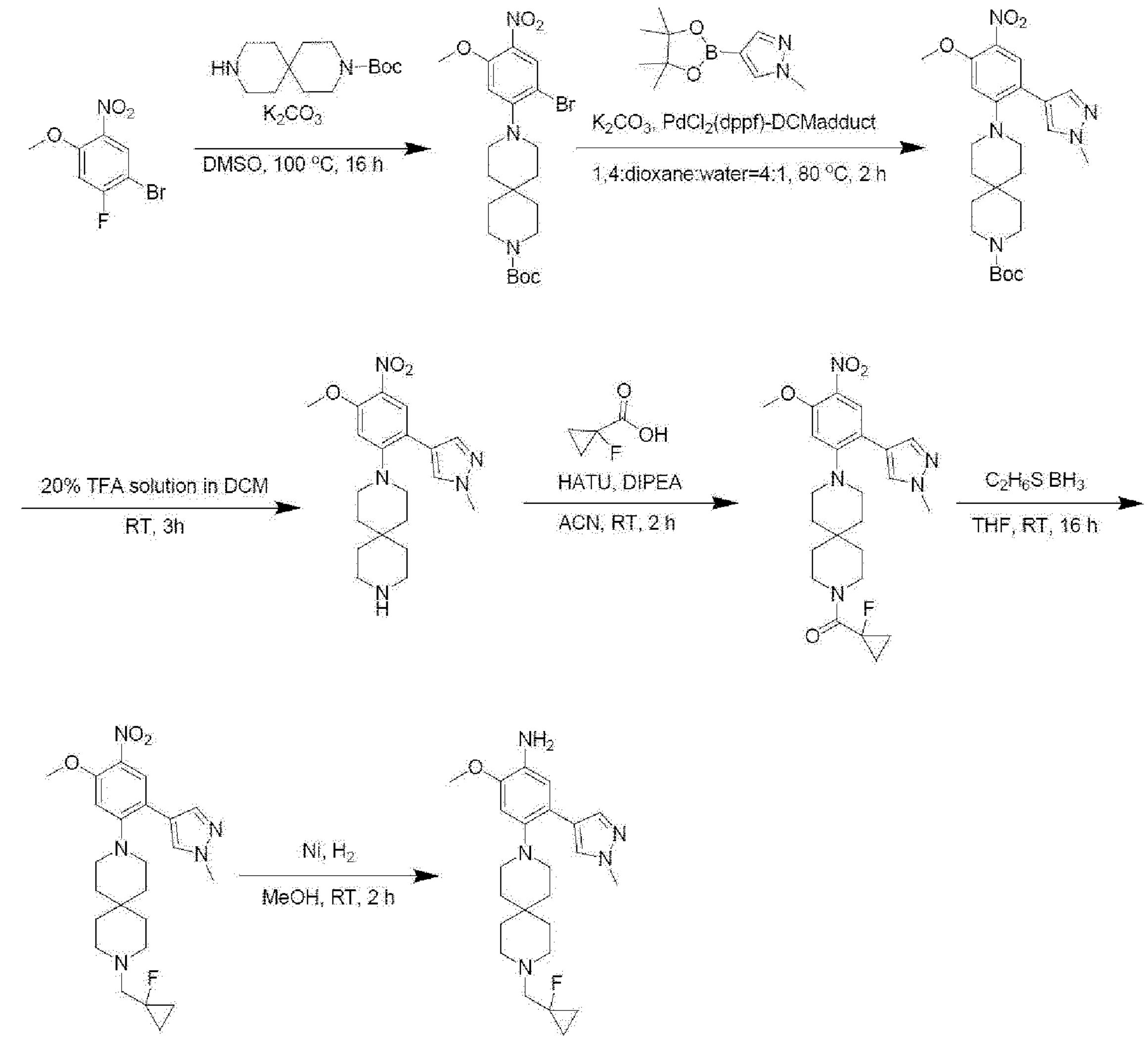


Step 1: Preparation of tert-butyl 9-(2-bromo-5-methoxy-4-nitrophenyl)-3,9-diazaspiro[5.5]undecan-3-carboxylate

1-Bromo-2-fluoro-4-methoxy-5-nitrobenzene (1 eq.), tert-butyl 3,9-diazaspiro[5.5]undecan-3-carboxylate (2 eq.), and $K_2CO_3$ (5 eq.) was dissolved in DMSO (0.17 M), and stirred at 100°C for 1 hour. The yellow solid obtained by adding water was used in the next reaction without further purification (yield: 97%, MS (ESI): m/z 484 $[M+H]^+$).

Step 2: Preparation of tert-butyl 9-(5-methoxy-2-(1-methyl-1H-pyrazol-4-yl)-4-nitrophenyl)-3,9-diazaspiro[5.5]undecan-3-carboxylate

tert-Butyl 9-(2-bromo-5-methoxy-4-nitrophenyl)-3,9-diazaspiro[5.5]undecan-3-carboxylate (1 eq.) prepared in step 1, 1-methyl-4-(4,4,5,5-tetramethyl-1,3,2-dioxaborolan-2-yl)-1H-pyrazole (1.5 eq.), and $K_2CO_3$ (2 eq.) were dissolved in 1,4-dioxane:water = 4:1 (0.2 M), and $PdCl_2$(dppf)-DCM adduct(0.1 eq.) was added under nitrogen, and stirred at 100°C for 2 hours. The stirred mixture was filtered through celite and then concentrated. The reaction mixture was purified through MPLC (DCM:EtOAc) to obtain the target compound as a yellow liquid (yield: 85%, MS (ESI) : m/z 486 $[M+H]^+$).

Step 3: Preparation of 3-(5-methoxy-2-(1-methyl-1H-pyrazol-4-yl)-4-nitrophenyl)-3,9-diazaspiro[5.5]undecane

tert-Butyl 9-(5-methoxy-2-(1-methyl-1H-pyrazol-4-yl)-4-nitrophenyl)-3,9-diazaspiro[5.5]undecan-3-carboxylate (1 eq.) prepared in step 2 was dissolved in DCM (0.13 M), and TFA (20 eq.) was added and stirred at room temperature for 1 hour. After concentrating the mixture, DCM and ether were added to obtain a yellow solid. The obtained yellow solid was used in the next reaction without further purification (yield: 100%, MS (ESI) : m/z 386 $[M+H]^+$).

Step 4: Preparation of (1-fluorocyclopropyl) (9-(5-methoxy-2-(1-methyl-1H-pyrazol-4-yl)-4-nitrophenyl)-3,9-diazaspiro[5.5]undecan-3-yl)methanone

3-(5-Methoxy-2-(1-methyl-1H-pyrazol-4-yl)-4-nitrophenyl)-3,9-diazaspiro[5.5]undecane (1 eq.) prepared in step 3 was dissolved in acetonitrile (0.34 M), and then 1-fluorocyclopropane-1-carboxylic acid (1.3 eq.), DIPEA (5 eq.), and HATU (2 eq.) were added and stirred at room temperature for 3 hours. The reaction mixture was concentrated and purified through MPLC (DCM:MeOH) to obtain the target compound as a yellow liquid (yield: 100%, MS (ESI): m/z 472 $[M+H]^+$).

Step 5: Preparation of 3-((1-fluorocyclopropyl)methyl)-9-(5-methoxy-2-(1-methyl-1H-pyrazol-4-yl)-4-nitrophenyl)-3,9-diazaspiro[5.5]undecane

To the (1-fluorocyclopropyl) (9-(5-methoxy-2-(1-methyl-1H-pyrazol-4-yl)-4-nitrophenyl)-3,9-diazaspiro[5.5]undecan-3-yl)methanone prepared in step 4, dimethyl sulfide borane (in THF) (2 M) was added and stirred at room temperature for 16 hours. Methanol (0.51 M) was added at 0°C and stirred for 20 minutes. The reaction mixture was concentrated and purified through MPLC (DCM:MeOH) to obtain the target compound as a yellow solid (yield: 34%, MS (ESI): m/z 458 $[M+H]^+$).

Step 6: Preparation of 4-(9-((1-fluorocyclopropyl)methyl)-3,9-diazaspiro[5.5]undecan-3-yl)-2-methoxy-5-(1-methyl-1H-pyrazol-4-yl)aniline

3-((1-Fluorocyclopropyl)methyl)-9-(5-methoxy-2-(1-methyl-1H-pyrazol-4-yl)-4-nitrophenyl)-3,9-diazaspiro [5.5]undecane (1 eq.) prepared in step 5, and Raney Ni (1 eq.) were dissolved in THF (0.03 M), followed by stirring under hydrogen at room temperature for 30 minutes. The stirred mixture was filtered through celite, and the filtrate was concentrated. The target compound of the obtained gray liquid was used in the next reaction without further purification (yield: 91%, MS (ESI): m/z 428 $[M+H]^+$).

Preparation Example 3: Preparation of 5-(1-methyl-1H-pyrazol-4-yl)-4-morpholino-2,3-dihydrobenzofuran-7-amine

[Reaction Scheme 3]

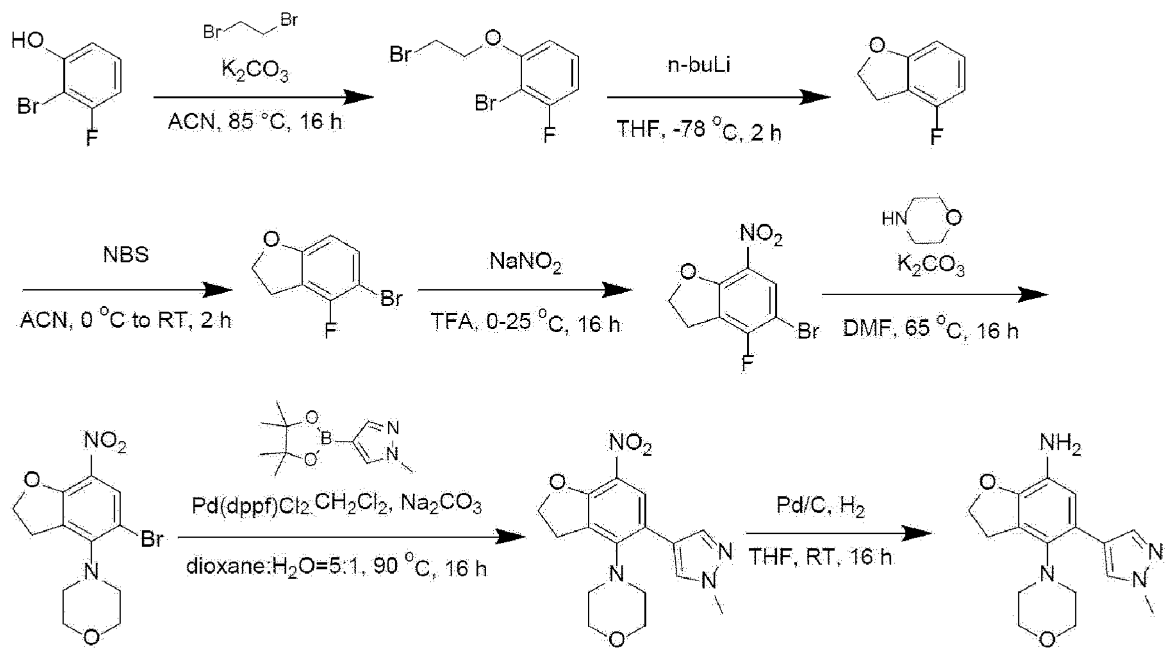

Step 1: Preparation of 2-bromo-1-(2-bromoethoxy)-3-fluorobenzene

2-Bromo-3-fluorophenol (1 eq.), 1,2-dibromoethane (5 eq.), and $K_2CO_3$ (3 eq.) were dissolved in acetonitrile (0.57 M), followed by stirring at 85°C for 16 hours. The reaction mixture was filtered and concentrated, and then purified through column chromatography (Petroleum ether:EtOAc) to obtain the target compound as a colorless liquid (yield: 82%) . $^1$H NMR (400 MHz, $CDCl_3$) δ 7.25-7.23 (m, 1H), 6.84-6.80 (dt, J = 6.8 Hz, 1H), 6.71-6.69 (d, J = 8.4 Hz, 1H), 4.36 (t, J= 6.4 Hz, 2H), 3.70 (t, J= 6.4 Hz, 2H).

Step 2: Preparation of 4-fluoro-2,3-dihydrobenzofuran

2-Bromo-1-(2-bromoethoxy)-3-fluorobenzene (1 eq.) prepared in step 1 was dissolved in THF (0.26 M) and then n-BuLi (2.5 M, 1.1 eq.) was slowly added at -78°C, followed by stirring for 2 hours. Water and $NH_4Cl$ were added to the reaction mixture, organic materials were extracted with MTBE, and the collected organic layers were concentrated by removing remaining water using $Na_2SO_4$. The reaction mixture was purified through column chromatography (Petroleum ether:MTBE) to obtain the target compound as a colorless liquid (yield: 70%). $^1$H NMR (400 MHz, $CDCl_3$) δ 7.11-7.05 (m, 1H), 6.61-6.55 (m, 2H), 4.63 (t, J = 8.4 Hz, 2H), 3.26 (t, J = 8.4 Hz, 2H).

Step 3: Preparation of 5-bromo-4-fluoro-2,3-dihydrobenzofuran

4-Fluoro-2,3-dihydrobenzofuran (1 eq.) prepared in step 2 was dissolved in acetonitrile (0.7 M), and then NBS (1.1 eq.) was added at 0°C, followed by stirring at room temperature for 2 hours. The reaction mixture was concentrated and purified through column chromatography (Petroleum ether:MTBE) to obtain the target compound as a colorless liquid (yield: 94%). $^1$H NMR (400 MHz, $CDCl_3$) δ 7.29-7.25 (m, 1H), 6.52-6.50 (d, J = 8.4 Hz, 1H), 4.67 (t, J= 8.8 Hz, 2H), 3.30 (t, J= 8.8 Hz, 2H).

Step 4: Preparation of 5-bromo-4-fluoro-7-nitro-2,3-dihydrobenzofuran

5-Bromo-4-fluoro-2,3-dihydrobenzofuran (1 eq.) prepared in step 3 was dissolved in TFA (0.55 eq.), and then $NaNO_2$(2 eq.) was added at 0°C, followed by stirring at room temperature for 16 hours. To the reaction mixture, a saturated NaHCOs solution was added, organic materials were extracted with EtOAc, and the collected organic layers were concentrated by removing remaining water using $Na_2SO_4$. The reaction mixture was purified through column chromatography (Petroleum ether:EtOAc) to obtain the target compound as a yellow solid (yield: 55%). $^1$H NMR (400 MHz, $CDCl_3$) δ 8.22-8.20 (d, J = 6.0 Hz, H), 4.95 (t, J = 8.8 Hz, 2H), 3.41 (t, *J* = 8.8 Hz, 2H).

Step 5: Preparation of 4-(5-bromo-7-nitro-2,3-dihydrobenzofuran-4-yl)morpholine

5-Bromo-4-fluoro-7-nitro-2,3-dihydrobenzofuran (1 eq.) prepared in step 4 was dissolved in DMF, and then $K_2CO_3$ (2 eq.) and morpholine (1.5 eq.) were added and stirred at 65°C for 16 hours. To the reaction mixture, water was added, organic layers were extracted with EtOAc, and the collected organic layers were concentrated by removing remaining water using $Na_2SO_4$. The reaction mixture was purified through column chromatography (Petroleum ether:EA) to obtain the target compound as a yellow solid (yield: 54%). $^1$H NMR (400 MHz, $CDCl_3$) δ 8.20 (s, 1H), 4.83 (t, J = 8.8 Hz, 2H), 3.87 (m, 4H), 3.43 (t, J = 8.8 Hz, 2H), 3.25 (m, 4H).

Step 6: Preparation of 4-(5-(1-methyl-1H-pyrazol-4-yl)-7-nitro-2,3-dihydrobenzofuran-4-yl)morpholine

4-(5-Bromo-7-nitro-2,3-dihydrobenzofuran-4-yl)morpholine (1 eq.) prepared in step 5 was dissolved in 1,4-dioxane:water = 5:1 (0.36 M), then 1-methyl-4-(4,4,5,5-tetramethyl-1,3,2-dioxaborolan-2-yl)-1H-pyrazole (1.3 eq.), Pd(dppf)DCM adduct(0.1 eq.), and $Na_2CO_3$ (2 eq.) were added under nitrogen, and stirred at 90°C for 16 hours. To the reaction mixture, water was added, organic layers were extracted with EtOAc, and the collected organic layers were concentrated by removing remaining water using $Na_2SO_4$. The reaction mixture was purified through column chromatography (DCM:MeOH), and then EtOAc was added, followed by filtration to obtain the target compound as a yellow solid (yield: 42%) . $^1$H NMR (400 MHz, $CDCl_3$) δ 7.85 (s, 1H), 7.79 (s, 1H), 7.63 (s, 1H), 4.83 (t, J = 8.8 Hz, 2H), 3.97 (s, 3H), 3.70 (m, 4H), 3.45 (t, J = 8.8 Hz, 2H), 3.09 (m, 4H).

Step 7: 5-(1-Methyl-1H-pyrazol-4-yl)-4-morpholino-2,3-dihydrobenzofuran-7-amine

4-(5-(1-Methyl-1H-pyrazol-4-yl)-7-nitro-2,3-dihydrobenzofuran-4-yl)morpholine (1 eq.) prepared in step 6 was dissolved in THF (0.06 M), then Pd/C (10% purity, 0.05 eq.) was added under nitrogen, and the reaction mixture was

stirred under hydrogen for 16 hours. The stirred mixture was filtered through celite, and the filtrate was concentrated. The compound of the obtained yellow solid was used in the next reaction without further purification (yield: 92%). $^1$H NMR (400 MHz, $CDCl_3$): δ 7.78 (s, 1H), 7.72 (s, 1H), 6.63 (s, 1H), 4.59 (t, J = 8.8 Hz, 2H), 3.94 (s, 3H), 3.74 (m, 4H), 3.50 (s, 2H), 3.40 (t, J = 8.4 Hz, 2H), 2.97 (s, 4H) .

**Preparation Example 4: Preparation of 6-(1-methyl-1H-pyrazol-4-yl)-7-(4-methylpiperazin-1-yl)benzo[d][1,3]dioxol-4-amine**

[Reaction Scheme 4]

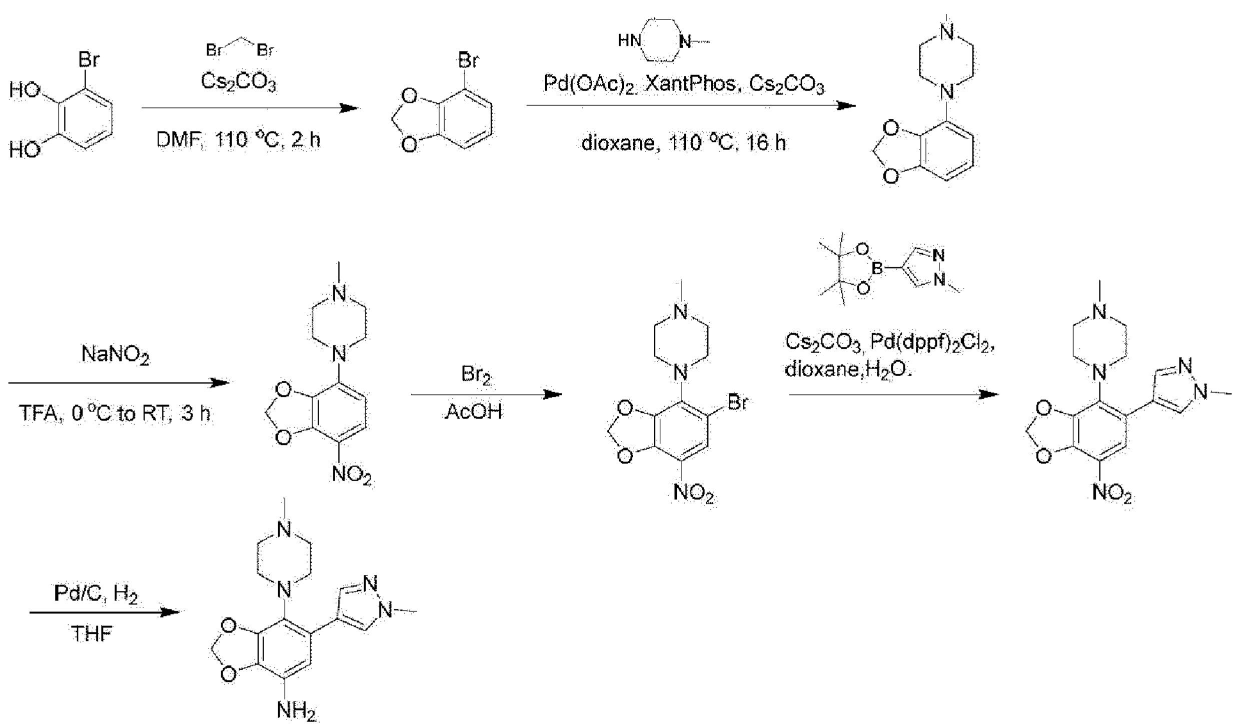


Step 1: Preparation of 4-bromobenzo[d][1,3]dioxole

3-Bromobenzene-1,2-diol (1 eq.), $Cs_2CO_3$ (1.5 eq.), and dibromomethane (1.5 eq.) were dissolved in DMF (0.5 M), followed by stirring at 110°C for 2 hours. Water and brine were added, and organic materials were extracted with EtOAc. The collected organic layers were concentrated by removing remaining water using $Na_2SO_4$, and then purified through column chromatography (Petroleum ether:EtOAc) to obtain the target compound as a colorless liquid (yield: 54%). $^1$H NMR (400 MHz, $CDCl_3$) δ 6.99-6.96 (m, 1H), 6.78-6.73 (m, 3H), 6.03 (s, 2H).

Step 2: Preparation of 1-(benzo[d][1,3]dioxol-4-yl)-4-methylpiperazine

4-Bromobenzo[d] [1,3]dioxole (1 eq.) prepared in step 1 was dissolved in 1,4-dioxane (0.3 M), and then 1-methylpiperazine (2.0 eq.), Pd(OAc)$_2$ (0.1 eq.), XantPhos (0.2 eq.), and $Cs_2CO_3$ (2.5 eq.) were added under nitrogen, and stirred at 110°C for 16 hours. The stirred mixture was filtered through celite and then concentrated. The reaction mixture was purified through a reverse phase column (0.1% TFA) to obtain the target compound as a brown liquid (yield: 58%). $^1$H NMR (400 MHz, $CDCl_3$) δ 6.97 (dd, J= 1.6, 8.4 Mz. 1H), 6.78-6.71 (m, 2H), 6.03 (s, 2H).

Step 3: Preparation of 1-methyl-4-(7-nitrobenzo[d][1,3]dioxol-4-yl)piperazine

1-(Benzo[d][1,3]dioxol-4-yl)-4-methylpiperazine (1 eq.) prepared in step 2 was dissolved in TFA (0.23 M), and then $NaNO_2$ (2.5 eq.) was added at 0°C, followed by stirring at room temperature for 3 hours. Ice water was added to the reaction mixture, the pH was adjusted to 8 using 6 M NaOH, and the organic materials were extracted with EtOAc.

The collected organic layers were concentrated by removing remaining water using $Na_2SO_4$, and then purified through prep-HPLC (TFA in water:acetonitrile) to obtain the target compound as a yellow solid (yield: 45%). $^1H$ NMR (400 MHz, $CDCl_3$) δ 7.63 (d, *J* = 9.6 Mz. 1H), 6.42 (d, *J* = 9.6 Mz. 1H), 6.16 (s, 2H), 3.48 (t, J= 4.8 Mz. 4H), 2.66 (br s, 4H), 2.43 (s, 3H).

Step 4: Preparation of 1-(5-bromo-7-nitrobenzo[d] [1,3]dioxol-4-yl)-4-methylpiperazine

1-Methyl-4-(7-nitrobenzo[d][1,3]dioxol-4-yl)piperazine (1 eq.) prepared in step 3 was dissolved in acetic acid (0.35 M), and then $Br_2$ (2 eq.) was added and stirred at room temperature for 5 hours. The reaction mixture was filtered, and then the organic materials were extracted with EtOAc and concentrated. The target compound of the obtained yellow solid was used in the next reaction without further purification (yield: 91%). $^1H$ NMR (400 MHz, $CD_3CN$) δ 7.86 (s, 1H), 6.24 (s, 2H), 3.53-3.41 (m, 6H), 3.28-3.18 (m, 2H), 2.87 (d, *J* = 3.2 Mz. 3H).

Step 5: Preparation of 1-methyl-4-(5-(1-methyl-1H-pyrazol-4-yl)-7-nitrobenzo[d] [1,3]dioxol-4-yl)piperazine

1-(5-Bromo-7-nitrobenzo[d][1,3]dioxol-4-yl)-4-methylpiperazine (1 eq.) prepared in step 4, 1-methyl-4-(4,4,5,5-tetramethyl-1,3,2-dioxaborolan-2-yl)-1H-pyrazole (1.1 eq.), and $Cs_2CO_3$ (3 eq.) were dissolved in 1,4-dioxane:$H_2O$ = 5:1 (0.12 M), then Pd(dppf)$Cl_2$.DCM adduct (0.1 eq.) was added, and the reaction mixture was stirred at 80°C for 2 h under nitrogen. Ice water was added to the reaction mixture, the pH was adjusted to 8 using 6 M NaOH, and the organic materials were extracted with EtOAc. The collected organic layers were concentrated by removing remaining water using $Na_2SO_4$, and then purified through prep-HPLC (TFA in water:acetonitrile) to obtain the target compound as a yellow solid (yield: 32%). $^1H$ NMR (400 MHz, $CDCl_3$) δ 7.83 (s, 1H), 7.68 (s, 1H), 7.57 (s, 1H), 6.17 (s, 2H), 3.97 (s, 3H), 3.22 (s, 4H), 2.52 (s, 4H), 2.38 (s, 3H).

Step 6: Preparation of 6-(1-methyl-1H-pyrazol-4-yl)-7-(4-methylpiperazin-1-yl)benzo[d][1,3]dioxol-4-amine

1-Methyl-4-(5-(1-methyl-1H-pyrazol-4-yl)-7-nitrobenzo[d][1,3]dioxol-4-yl)piperazine (1 eq.) prepared in step 5 was dissolved in THF (0.15 M), and Pd/C (10% purity, 0.1 eq.) was added under nitrogen. The reaction mixture was stirred under hydrogen for 16 hours. The stirred mixture was filtered through celite, and the filtrate was concentrated. The compound of the obtained colorless solid was used in the next reaction without further purification (yield: 89%, MS (ESI): m/z 316 $[M+H]^+$).

**Example 1: Preparation of (6-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)quinoxalin-5-yl)dimethylphosphine oxide**

[Reaction Scheme 5]

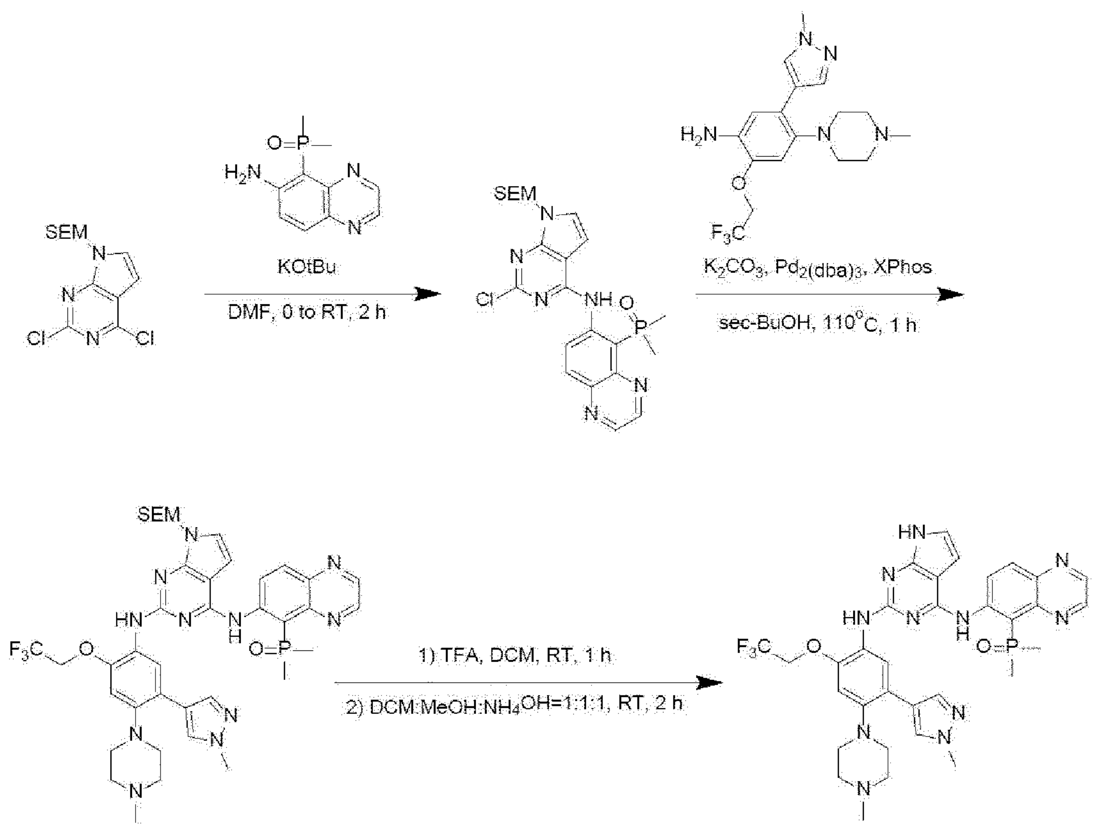


Step 1: Preparation of (6-((2-chloro-7-((2-(trimethylsilyl)ethoxy)methyl)-7H-pyrrolo[2,3-d]pyrimidin-4-yl)amino)quinoxalin-5-yl)dimethylphosphine oxide

2,4-Dichloro-7-((2-(trimethylsilyl)ethoxy)methyl)-7H-pyrrolo[2,3-d]pyrimidine (1 eq.), (6-aminoquinoxalin-5-yl)dimethylphosphine oxide (1.15 eq.) were dissolved in DMF (0.2 M), then KOtBu (1.5 eq.) was added at 0°C, and stirred at room temperature for 2 hours. Water and brine were added, and organic materials were extracted with EtOAc. The collected organic layers were concentrated by removing remaining water using $Na_2SO_4$. DCM and ether were added to the reaction mixture to obtain the target compound as a yellow solid (yield: 48%, MS (ESI): m/z 503 $[M]^+$).

Step 2: Preparation of dimethyl(6-((2-((5-(1-methyl-1H-pyrazol-4-yl)-4-(4-methylpiperazin-1-yl)-2-(2,2,2-trifluoroethoxy)phenyl)amino)-7-((2-(trimethylsilyl)ethoxy)methyl)-7H-pyrrolo[2,3-d]pyrimidin-4-yl)amino)quinoxalin-5-yl)phosphine oxide

(6-((2-Chloro-7-((2-(trimethylsilyl) ethoxy) methyl) - 7H-pyrrolo[2,3-d]pyrimidin-4-yl)amino)quinoxalin-5-yl)dimethylphosphine oxide (1 eq.) prepared in step 1, 5-(1-methyl-1H-pyrazol-4-yl)-4-(4-methylpiperazin-1-yl)-2-(2,2,2-trifluoroethoxy)aniline (1.2 eq.) and $K_2CO_3$ (3 eq.) were dissolved in sec-BuOH (0.1 M), then $Pd_2(dba)_3$ (0.1 eq.) and Xphos (0.1 eq.) were added under nitrogen, and stirred at 110°C for 1 hour. The stirred mixture was filtered through celite and then concentrated. The reaction mixture was purified through MPLC (DCM:MeOH) to obtain the target compound as a brown liquid (yield: 74%, MS (ESI): m/z 836 $[M+H]^+$).

Step 3: Preparation of dimethyl(6-((2-((5-(1-methyl-1H-pyrazol-4-yl)-4-(4-methylpiperazin-1-yl)-2-(2,2,2-trifluoroethoxy)phenyl)amino)-7H-pyrrolo[2,3-d]pyrimidin-4-yl)amino)quinoxalin-5-yl)phosphine oxide

Dimethyl(6-((2-((5-(1-methyl-1H-pyrazol-4-yl)-4-(4-methylpiperazin-1-yl)-2-(2,2,2-trifluoroethoxy)phenyl)amino)-7-((2-(trimethylsilyl)ethoxy)methyl)-7H-pyrrolo[2,3-d]pyrimidin-4-yl)amino)quinoxalin-5-yl)phosphine oxide (1 eq.) prepared in step 2 was dissolved in DCM (0.1 M), and an equal amount of TFA was added and stirred at room temperature for 1 hour. The reaction mixture was concentrated and dissolved in DCM:MeOH:$NH_4OH$ = 1:1:1, followed by stirring at room temperature for 2 hours. Water and brine were added, organic materials were extracted with EtOAc, and the collected organic layers were concentrated by removing remaining water using $Na_2SO_4$. The reaction mixture was purified through prep-HPLC, and the target compound was obtained as a yellow solid (yield: 82%, MS (ESI): m/z 706

[M+H]+).

**Preparation of Compounds of Examples 2 to 142**

Compounds of Examples 2 to 142 were prepared in a similar manner to Example 1 above. Chemical structures, compound names, NMR, yields, and HPLC analysis results of the compounds of Examples 1 to 142 are summarized in Table 1 below.

[Table 1]

| | Structure | Compound name | $^1$H NMR, MS | Yield (%) | HPLC r.t.(min), Purity(%), (meth |
|---|---|---|---|---|---|

| | | | | | |
|---|---|---|---|---|---|
| | | | | | od) |
| 1 | 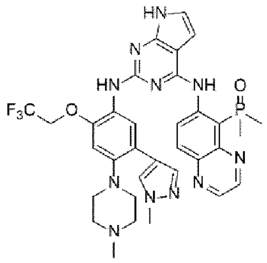 | dimethyl(6-((2-((5-(1-methyl-1H-pyrazol-4-yl)-4-(4-methylpiperazin-1-yl)-2-(2,2,2-trifluoroethoxy)phenyl)amino)-7H-pyrrolo[2,3-d]pyrimidin-4-yl)amino)quinoxalin-5-yl)phosphine oxide | $^{1}$H NMR (400 MHz, TFA salt, Methanol-$d_4$) δ 9.26-9.24 (m, 1H), 8.89 (d, *J* = 2.0 Hz, 1H), 8.84 (d, *J* = 2.0 Hz, 1H), 8.12 (s, 1H), 7.87 (s, 1H), 7.82 (s, 1H), 7.70-7.67 (m, 1H), 7.10 (d, *J* = 3.6 Hz, 1H), 7.04 (s, 1H), 6.77 (d, *J* = 3.6 Hz, 1H), 4.74-4.67 (m, 1H), 3.91 (s, 3H), 3.64-3.61 (m, 2H), 3.47-3.32 (m, 4H), 3.17-3.12 (m, 2H), 3.05 (s, 3H), 2.22 (s, 3H), 2.18 (s, 3H); MS(ESI): 706 m/z [M+H]$^{+}$ | 82 | 8.60, 98, (b) |

| | | | | | |
|---|---|---|---|---|---|
| 2 | 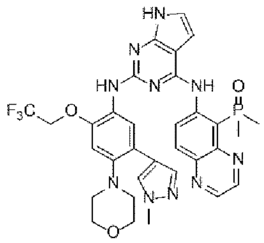 | dimethyl(6-((2-((5-(1-methyl-1H-pyrazol-4-yl)-4-morpholino-2-(2,2,2-trifluoroethoxy)phenyl)amino)-7H-pyrrolo[2,3-d]pyrimidin-4-yl)amino)quinoxalin-5-yl)phosphine oxide | $^1$H NMR (400 MHz, FA salt, DMSO-$d_6$) δ 13.25 (s, 1H), 11.41 (s, 1H), 9.63 (dd, $J$ = 9.40 Hz, 1H), 8.85 (d, $J$ = 1.79 Hz, 1H), 8.80 (d, $J$ = 1.79 Hz, 1H), 8.53 (s, 0.1H), 8.19 (s, 1H), 8.08 (s, 1H), 7.91 (s, 1H), 7.70 (s, 1H), 7.68 (d, $J$ = 8.93 Hz, 1H), 7.03-7.00 (m, 2H), 6.47 (s, 1H), 4.88 (q, 2H), 3.84 (s, 3H), 3.77 (t, 4H) 2.87 (t, 4H), 2.09 (s, 3H), 2.06 (s, 3H); MS(ESI): m/z 693 [M+H]$^+$ | 29 | 10.78, 97, (b) |
| 3 | 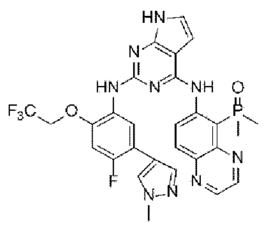 | (6-((2-((4-fluoro-5-(1-methyl-1H-pyrazol-4-yl)-2- | $^1$H NMR (400 MHz, FA salt, DMSO-$d_6$) δ 13.26 (s, 1H), 11.46 (s, 1H), 9.60 (dd, $J$ = 9.40 Hz, 1H), 8.85 (d, | 86 | 11.42, 98, (b) |

| | | | | | |
|---|---|---|---|---|---|
| | | (2,2,2-trifluoroethoxy)phenyl)amino)-7H-pyrrolo[2,3-d]pyrimidin-4-yl)amino)quinoxalin-5-yl)dimethylphosphine oxide | $J$ = 1.81 Hz, 1H), 8.80 (d, $J$ = 1.82 Hz, 1H), 8.49 (s, 0.2H), 8.32 (d, $J$ = 8.44 Hz, 1H), 8.07 (d, $J$ = 1.68 Hz, 1H), 7.82 (d, $J$ = 2.16 Hz, 1H), 7.76 (d, $J$ = 9.80 Hz, 1H), 7.05 (q, 1H), 6.48 (q, 1H), 4.89 (q, 2H), 3.84 (s, 3H), 2.09 (s, 3H), 2.06 (s, 3H); MS(ESI): m/z 626 $[M+H]^+$ | | |
| 4 | 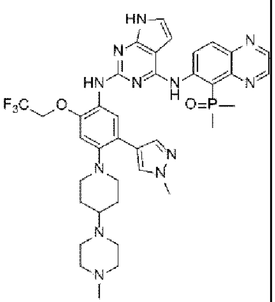 | dimethyl(6-((2-((5-(1-methyl-1H-pyrazol-4-yl)-4-(4-(4-methylpiperazin-1-yl)piperidin-1-yl)-2-(2,2,2- | $^1$H NMR (400 MHz, TFA salt, $CD_3OD$) δ 9.34 (d, $J$ = 5.2 Hz, 1H), 8.87 (d, $J$ = 2.0 Hz, 1H), 8.81 (d, $J$ = 2.0 Hz, 1H), 8.05 (s, 1H), 7.86 (s, 1H), 7.82 (s, 1H), 7.71 (d, $J$ = 9.2 Hz, 1H), 7.08 (d, $J$ = 3.2 Hz, 1H), 6.99 (s, | 51 | 8.22, 97, (b) |

| | | | | | |
|---|---|---|---|---|---|
| | | trifluoroethoxy)phenyl)amino)-7H-pyrrolo[2,3-d]pyrimidin-4-yl)amino)quinoxalin-5-yl)phosphine oxide | 1H), 6.74 (d, *J* = 3.6 Hz, 1H), 4.69 (q, *J* = 8.4 Hz, 2H), 3.86 (s, 3H), 3.38-3.33 (m, 4H), 3.24-3.20 (m, 4H), 2.92 (s, 1H), 2.86 (s, 3H), 2.79 (t, *J* = 11.2 Hz, 2H), 2.21 (s, 3H), 2.17 (s, 3H), 2.12 (d, *J* = 12.0 Hz, 2H), 1.87-1.79 (m, 2H), 1.33 (t, *J* = 7.2 Hz, 2H); MS(ESI): m/z 789 $[M+H]^+$ | | |
| 5 | 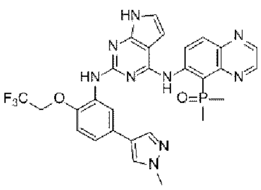 | dimethyl(6-((2-((5-(1-methyl-1H-pyrazol-4-yl)-2-(2,2,2-trifluoroethoxy)phenyl)amino)-7H-pyrrolo[2,3- | $^1H$ NMR (400 MHz, TFA salt, Methanol-$d_4$) δ 9.58-9.55 (m, 1H), 8.71 (d, *J* = 1.6 Hz, 1H), 8.63 (d, *J* = 1.6 Hz, 1H), 8.51 (d, *J* = 2.5 Hz, 1H), 7.73 (s, 1H), 7.62-7.58 (m, 2H), 7.16-7.13 (m, 1H), 7.05 (d, *J* = | 56 | 6.78 100 (b) |

| | | | | | |
|---|---|---|---|---|---|
| | | d]pyrimidin-4-yl)amino)quinoxalin-5-yl)phosphine oxide | 8.4 Hz, 1H), 6.98 (d, *J* = 3.6 Hz, 1H), 6.54 (d, *J* = 3.6 Hz, 1H), 4.68-4.61 (m, 2H), 3.76 (s, 3H), 2.16 (s, 3H), 2.12 (s, 3H); MS(ESI): m/z 608 $[M+H]^+$ | | |
| 6 | 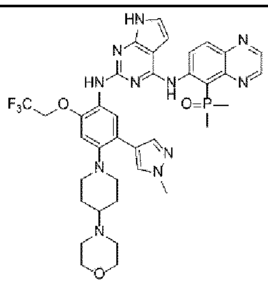 | dimethyl(6-((2-((5-(1-methyl-1H-pyrazol-4-yl)-4-(4-morpholinopiperidin-1-yl)-2-(2,2,2-trifluoroethoxy)phenyl)amino)-7H-pyrrolo[2,3-d]pyrimidin-4-yl)amino)qui | $^1H$ NMR (400 MHz, TFA salt, Methanol-$d_4$) δ 9.26-9.24 (m, 1H), 8.89 (d, *J* = 2.0 Hz, 1H), 8.84 (d, *J* = 2.0 Hz, 1H), 8.12 (s, 1H), 7.87 (s, 1H), 7.82 (s, 1H), 7.70-7.67 (m, 1H), 7.10 (d, *J* = 3.6 Hz, 1H), 7.04 (s, 1H), 6.77 (d, *J* = 3.6 Hz, 1H), 4.74-4.67 (m, 1H), 3.91 (s, 3H), 3.64-3.61 (m, 2H), 3.47-3.32 (m, 4H), 3.17-3.12 (m, 2H), | 48 | 8.79, 100, (b) |

| | | | | | |
|---|---|---|---|---|---|
| | | noxalin-5-yl)phosphine oxide | 3.05 (s, 3H), 2.22 (s, 3H), 2.18 (s, 3H); MS(ESI): m/z 776 [M+H]+ | | |
| 7 | 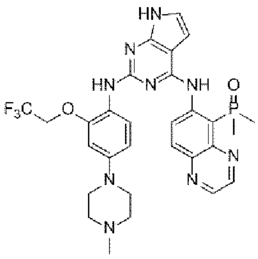 | dimethyl(6-((2-((4-(4-methylpiperazin-1-yl)-2-(2,2,2-trifluoroethoxy)phenyl)amino)-7H-pyrrolo[2,3-d]pyrimidin-4-yl)amino)quinoxalin-5-yl)phosphine oxide | $^1$H NMR (400 MHz, TFA salt, Methanol-$d_4$) δ 9.37-9.32 (m, 1H), 8.89 (d, *J* = 1.9 Hz, 1H), 8.83 (d, *J* = 1.9 Hz, 1H), 7.97 (d, *J* = 9.6 Hz, 1H), 7.57 (d, *J* = 8.6 Hz, 1H), 7.06 (d, *J* = 3.6 Hz, 1H), 6.92 (d, *J* = 2.5 Hz, 1H), 6.87 (dd, *J* = 8.7, 2.6 Hz, 1H), 6.73 (d, *J* = 3.5 Hz, 1H), 4.60 (q, *J* = 8.4 Hz, 2H), 4.08-3.96 (m, 4H), 3.75-3.64 (m, 4H), 3.04 (s, 3H), 2.21 (s, 3H), 2.17 (s, 3H); MS(ESI): m/z 626 [M+H]+ | 63 | 8.25, 100, (b) |

| | | | | | |
|---|---|---|---|---|---|
| 8 | 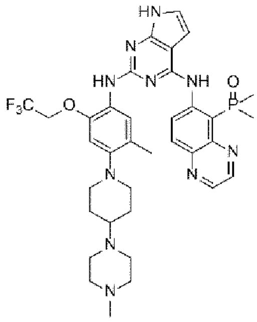 | dimethyl(6-((2-((5-methyl-4-(4-(4-methylpiperazin-1-yl)piperidin-1-yl)-2-(2,2,2-trifluoroethoxy)phenyl)amino)-7H-pyrrolo[2,3-d]pyrimidin-4-yl)amino)quinoxalin-5-yl)phosphine oxide | $^1$H NMR (400 MHz, TFA salt, Methanol-$d_4$) δ 9.40-9.25 (m, 1H), 8.89 (d, $J$ = 1.6 Hz, 1H), 8.84 (d, $J$ = 1.6 Hz, 1H), 7.98 (d, $J$ = 9.2 Hz, 1H), 7.46 (s, 1H), 7.07 (d, $J$ = 3.6 Hz, 1H), 6.96 (s, 1H), 6.76 (d, $J$ = 3.6 Hz, 1H), 4.62-4.50 (m, 2H), 3.60-6.40 (m, 9H), 3.30-3.20 (m, 2H), 3.00-2.82 (m, 5H), 2.33 (s, 3H), 2.28-2.12 (m, 8H), 2.10-1.92 (m, 2H); MS(ESI): m/z 723 [M+H]+ | 46 | 100* |
| 9 | 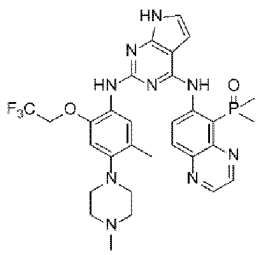 | dimethyl(6-((2-((5-methyl-4-(4-methylpiperazin-1-yl)-2- | $^1$H NMR (400 MHz, FA salt, Methanol-$d_4$) δ 9.72-9.69 (m, 1H), 8.85 (d, $J$ = 2.0 Hz, 1H), 8.76 (d, $J$ = 2.0 | 20 | 8.67, 100, (b) |

| | | | | | |
|---|---|---|---|---|---|
| | | (2,2,2-trifluoroethoxy)phenyl)amino)-7H-pyrrolo[2,3-d]pyrimidin-4-yl)amino)quinoxalin-5-yl)phosphine oxide | Hz, 1H), 8.42 (s, 1H), 8.09 (s, 1H), 8.04 (d, $J$ = 9.6 Hz, 1H), 7.01 (d, $J$ = 3.6 Hz, 1H), 6.92 (s, 1H), 6.63 (d, $J$ = 3.6 Hz, 1H), 4.67-4.61 (m, 2H), 3.24-3.16 (m, 8H), 2.83 (s, 3H), 2.32 (s, 3H), 2.22 (s, 3H), 2.19 (s, 3H); MS(ESI): m/z 640 $[M+H]^+$ | | |
| 10 | 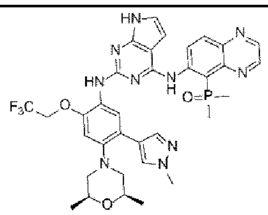 | (6-((2-((4-((2R,6S)-2,6-dimethylmorpholino)-5-(1-methyl-1H-pyrazol-4-yl)-2-(2,2,2-trifluoroethoxy)phenyl)amino)-7H- | $^1$H NMR (400 MHz, TFA salt, Methanol-$d_4$) δ 9.30 (dd, $J$ = 7.2, 3.4 Hz, 1H), 8.86 (d, $J$ = 1.8 Hz, 1H), 8.82 (d, $J$ = 1.8 Hz, 1H), 8.09 (s, 1H), 7.84 (s, 1H), 7.78 (s, 1H), 7.74 (d, $J$ = 9.0 Hz, 1H), 7.07 (d, $J$ = 3.6 Hz, 1H), 6.97 (s, 1H), 6.75 (d, $J$ = 3.5 Hz, 1H), 4.67 | 56 | 11.73, 98, (b) |

| | | | | | |
|---|---|---|---|---|---|
| | | pyrrolo[2,3-d]pyrimidin-4-yl)amino)quinoxalin-5-yl)dimethylphosphine oxide | (dd, *J* = 16.9, 8.4 Hz, 2H), 3.95-3.88 (m, 2H), 3.86 (s, 3H), 3.09 (d, *J* = 10.9 Hz, 2H), 2.50-2.41 (m, 2H), 2.21 (s, 3H), 2.17 (s, 3H); MS(ESI): m/z 721 $[M+H]^+$ | | |
| 11 | 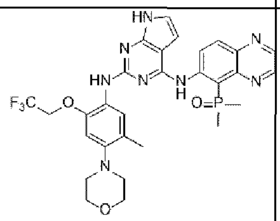 | dimethyl(6-((2-((5-methyl-4-morpholino-2-(2,2,2-trifluoroethoxy)phenyl)amino)-7H-pyrrolo[2,3-d]pyrimidin-4-yl)amino)quinoxalin-5-yl)phosphine oxide | $^1H$ NMR (400 MHz, FA salt, DMSO-$d_6$) δ 13.26 (s, 1H), 11.37 (s, 1H), 9.67-9.63 (m, 1H), 8.87 (d, *J* = 2.0 Hz, 1H), 8.82 (d, *J* = 2.0 Hz, 1H), 8.02 (d, *J* = 9.6 Hz, 1H), 7.82 (s, 1H), 7.66 (s, 1H), 7.01 (t, *J* = 2.4 Hz, 1H), 6.94 (s, 1H), 6.47-6.45 (m, 1H), 4.81 (q, *J* = 9.2 Hz, 2H), 3.78 (t, *J* = 4.0 Hz, 4H), 2.89 (t, *J* = | 20 | 11.41, 99, (b) |

| | | | | | |
|---|---|---|---|---|---|
| | | | 4.0 Hz, 4H), 2.33 (s, 3H), 2.32 (d, $J$ = 2.0 Hz, 6H); MS(ESI): m/z 627 $[M+H]^+$ | | |
| 12 | 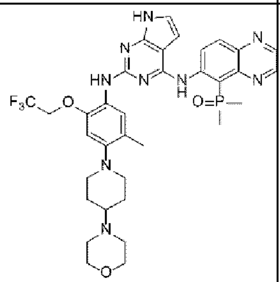 | dimethyl(6-((2-((5-methyl-4-(4-morpholinopiperidin-1-yl)-2-(2,2,2-trifluoroethoxy)phenyl)amino)-7H-pyrrolo[2,3-d]pyrimidin-4-yl)amino)quinoxalin-5-yl)phosphine oxide | $^1H$ NMR (400 MHz, FA salt, DMSO-$d_6$) δ 13.26 (s, 1H), 11.36 (s, 1H), 9.67-9.63 (m, 1H), 8.87 (d, $J$ = 1.6 Hz, 1H), 8.82 (d, $J$ = 1.6 Hz, 1H), 8.20 (s, 1H), 8.01 (d, $J$ = 9.6 Hz, 1H), 7.79 (s, 1H), 7.64 (s, 1H), 7.01 (t, $J$ = 3.2 Hz, 1H), 6.90 (s, 1H), 6.46 (t, $J$ = 3.2 Hz, 1H), 4.80 (q, $J$ = 8.8 Hz, 2H), 3.61-3.59 (m, 4H), 3.16-3.11 (m, 4H), 2.67-2.63 (m, 4H), 2.28 (s, 1H), 2.22 (s, 3H), 2.10 (s, 3H), 2.06 (s, 3H), 1.91-1.88 (m, | 41 | 8.81, 98, (b) |

| | | | | | |
|---|---|---|---|---|---|
| | | | 2H), 1.61-1.57 (m, 2H); MS(ESI): m/z 710 $[M+H]^+$ | | |
| 13 | 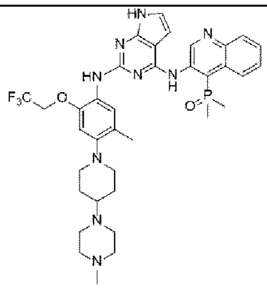 | dimethyl(3-((2-((5-methyl-4-(4-(4-methylpiperazin-1-yl)piperidin-1-yl)-2-(2,2,2-trifluoroethoxy)phenyl)amino)-7H-pyrrolo[2,3-d]pyrimidin-4-yl)amino)quinolin-4-yl)phosphine oxide | $^1H$ NMR (400 MHz, TFA salt, Methanol-$d_4$) δ 9.86 (d, *J* = 2.4 Hz, 1H), 8.33 (d, *J* = 8.4 Hz, 1H), 8.13 (d, *J* = 8.0 Hz, 1H), 7.83-7.74 (m, 2H), 7.45 (s, 1H), 7.09 (d, *J* = 3.2 Hz, 1H), 6.89 (s, 1H), 6.72 (d, *J* = 3.6 Hz, 1H), 4.65-4.58 (m, 2H), 3.50-3.29 (m, 10H), 3.16-3.15 (m, 1H), 2.84 (s, 3H), 2.83-2.81 (m, 2H), 2.13 (s, 3H), 2.12 (s, 3H), 2.11 (s, 3H), 2.11-2.10 (m, 2H), 1.89-1.81 (m, 2H); MS(ESI): m/z 722 $[M+H]^+$ | 75 | 7.84, 100, (b) |

| | | | | | |
|---|---|---|---|---|---|
| 14 | 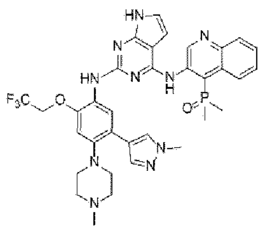 | dimethyl(3-((2-((5-(1-methyl-1H-pyrazol-4-yl)-4-(4-methylpiperazin-1-yl)-2-(2,2,2-trifluoroethoxy)phenyl)amino)-7H-pyrrolo[2,3-d]pyrimidin-4-yl)amino)quinolin-4-yl)phosphine oxide | $^1$H NMR (400 MHz, TFA salt, Methanol-$d_4$) δ 10.18 (d, *J* = 4.8 Hz, 1H), 8.35 (s, 1H), 8.12-8.10 (m, 1H), 7.94-7.91 (m, 1H), 7.80 (s, 1H), 7.73-7.67 (m, 3H), 7.02 (d, *J* = 3.6 Hz, 1H), 6.93 (s, 1H), 6.55 (d, *J* = 3.6 Hz, 1H), 4.73-4.65 (m, 2H), 3.54 (s, 3H), 3.16-3.15 (m, 4H), 2.99-2.81 (m, 4H), 2.53 (s, 3H), 1.34 (s, 3H), 1.31 (s, 3H); MS(ESI): m/z 705 [M+H]$^+$ | 34 | 8.25, 100, (b) |
| 15 | 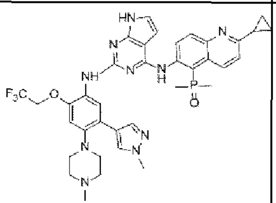 | (2-cyclopropyl-6-((2-((5-(1-methyl-1H-pyrazol-4-yl)-4-(4- | $^1$H NMR (400 MHz, FA salt, MeOD-$d_4$) δ 9.09-9.05 (m, 1H), 8.47 (s, 1H), 8.39 (d, *J* = 9.2 Hz, 1H), 8.31 (s, 1H), 7.83 (s, 1H), 7.78 (s, | 19 | 8.24, 98, (b) |

| | | | | | |
|---|---|---|---|---|---|
| | | methylpiperazin-1-yl)-2-(2,2,2-trifluoroethoxy)phenyl)amino)-7H-pyrrolo[2,3-d]pyrimidin-4-yl)amino)quinolin-5-yl)dimethylphosphine oxide | 1H), 7.59 (d, *J* = 9.6 Hz, 1H), 7.29 (d, *J* = 9.2 Hz, 1H), 6.95-6.92 (m, 2H), 6.50 (d, *J* = 3.6 Hz, 1H), 4.70-4.64 (m, 2H), 3.74 (s, 3H), 3.05 (s, 4H), 3.01 (s, 4H), 2.67 (s, 3H), 2.29-2.24 (m, 1H), 2.15 (s, 3H), 2.12 (s, 3H), 1.17-1.11 (m, 4H); MS(ESI): m/z 639 $[M+H]^+$ | | |
| 16 | 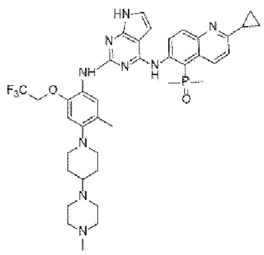 | (2-cyclopropyl-6-((2-((5-methyl-4-(4-(4-methylpiperazin-1-yl)piperidin-1-yl)-2-(2,2,2- | $^1$H NMR (400 MHz, TFA salt, Methanol-$d_4$) δ 8.93-8.85 (m, 1H), 8.74 (d, *J* = 9.0 Hz, 1H), 8.00 (d, *J* = 9.5 Hz, 1H), 7.49-7.41 (m, 2H), 7.04 (d, *J* = 3.5 Hz, 1H), 6.88 (s, 1H), 6.67 (d, *J* = 3.3 Hz, 1H), 4.56 (q, *J* = | 51 | 8.07, 99, (b) |

| | | | | | |
|---|---|---|---|---|---|
| | | trifluoroethoxy)phenyl)amino)-7H-pyrrolo[2,3-d]pyrimidin-4-yl)amino)quinolin-5-yl)dimethylphosphine oxide | 8.4 Hz, 2H), 3.28-3.25 (m, 5H), 3.23-3.15 (m, 2H), 2.92 (t, *J* = 12.1 Hz, 1H), 2.87-2.79 (m, 5H), 2.40-2.34 (m, 1H), 2.17 (s, 3H), 2.15-2.11 (m, 9H), 1.91-1.82 (m, 2H), 1.32-1.26 (m, 3H), 1.26-1.18 (m, 2H); MS(ESI): m/z 762 $[M+H]^+$ | | |
| 17 | 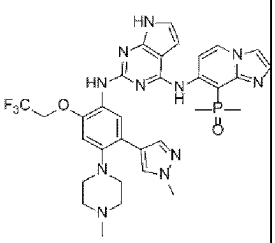 | dimethyl(7-((2-((5-(1-methyl-1H-pyrazol-4-yl)-4-(4-methylpiperazin-1-yl)-2-(2,2,2-trifluoroethoxy)phenyl)amino)-7H- | $^1H$ NMR (400 MHz, FA salt, Methanol-$d_4$) δ 8.74 (dd, *J* = 7.7, 3.5 Hz, 1H), 8.46 (s, 1H), 8.30 (s, 2H), 7.98 (s, 1H), 7.90 (s, 1H), 7.78 (d, *J* = 7.7 Hz, 1H), 7.60 (t, *J* = 1.4 Hz, 1H), 7.45 (d, *J* = 1.3 Hz, 1H), 6.98 (d, *J* = 3.6 Hz, 1H), 6.96 | 45 | 7.80, 99, (b) |

| | | | | | |
|---|---|---|---|---|---|
| | | pyrrolo[2,3-d]pyrimidin-4-yl)amino)imidazo[1,2-a]pyridin-8-yl)phosphine oxide | (s, 1H), 6.54 (d, *J* = 3.6 Hz, 1H), 4.71 (q, *J* = 8.5 Hz, 2H), 3.76 (s, 3H), 3.23-3.09 (m, 8H), 2.83 (s, 3H), 2.15 (s, 3H), 2.12 (s, 3H); MS(ESI): m/z 694 $[M+H]^+$ | | |
| 18 | 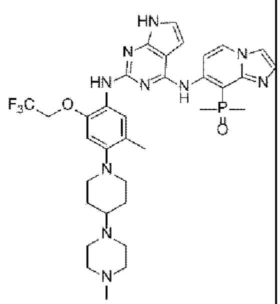 | dimethyl(7-((2-((5-methyl-4-(4-(4-methylpiperazin-1-yl)piperidin-1-yl)-2-(2,2,2-trifluoroethoxy)phenyl)amino)-7H-pyrrolo[2,3-d]pyrimidin-4-yl)amino)imi | $^1$H NMR (400 MHz, TFA salt, Methanol-$d_4$) δ 8.91-8.89 (m, 1H), 8.59 (d, *J* = 7.6 Hz, 1H), 8.01 (s, 1H), 7.78 (d, *J* = 1.6 Hz, 1H), 7.68 (s, 1H), 7.07 (d, *J* = 3.6 Hz, 1H), 6.99 (s, 1H), 6.67 (d, *J* = 3.6 Hz, 1H), 4.68-4.62 (m, 2H), 3.58-3.54 (m, 8H), 3.51-3.50 (m, 2H), 3.35-3.34 (m, 1H), 2.99 (s, 3H), 2.97-2.94 (m, 2H), | 7 | 96* |

| | | | | | |
|---|---|---|---|---|---|
| | | dazo[1,2-a]pyridin-8-yl)phosphine oxide | 2.36 (s, 3H), 2.28-2.25 (m, 2H), 2.18 (s, 3H), 2.15 (s, 3H), 2.07-2.01 (m, 2H); MS(ESI): m/z 711 [M+H]$^+$ | | |
| 19 | 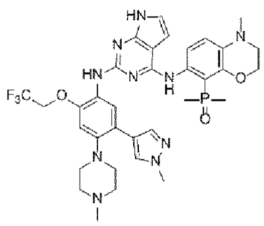 | dimethyl(4-methyl-7-((2-((5-(1-methyl-1H-pyrazol-4-yl)-4-(4-methylpiperazin-1-yl)-2-(2,2,2-trifluoroethoxy)phenyl)amino)-7H-pyrrolo[2,3-d]pyrimidin-4-yl)amino)-3,4-dihydro-2H-benzo[b][1,4 | MS(ESI): m/z 725 [M+H]$^+$ | | |

| | | | | | |
|---|---|---|---|---|---|
| | | ]oxazin-8-yl)phosphine oxide | | | |
| 20 | 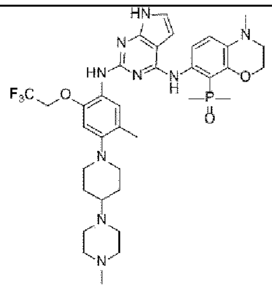 | dimethyl(4-methyl-7-((2-((5-methyl-4-(4-(4-methylpiperazin-1-yl)piperidin-1-yl)-2-(2,2,2-trifluoroethoxy)phenyl)amino)-7H-pyrrolo[2,3-d]pyrimidin-4-yl)amino)-3,4-dihydro-2H-benzo[b][1,4]oxazin-8-yl)phosphine | MS(ESI): m/z 742 $[M+H]^+$ | | |

| | | | | | |
|---|---|---|---|---|---|
| | | oxide | | | |
| 21 | 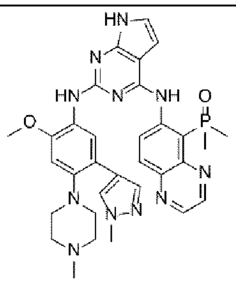 | (6-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)quinoxalin-5-yl)dimethylphosphine oxide | $^1$H NMR (400 MHz, TFA salt, Metan ol-$d_4$) δ 9.34 (dd, *J* = 9.32 Hz, 1H), 8.87 (d, *J* = 1.89 Hz, 1H), 8.82 (d, *J* = 1.90 Hz, 1H), 8.04 (s, 1H), 7.95 (s, 1H), 7.77 (s, 1H), 7.62 (d, *J* = 10.10 Hz, 1H), 7.08 (d, *J* = 3.55 Hz, 1H), 6.94 (s, 1H), 6.75 (d, *J* = 3.54 Hz, 1H), 3.97 (s, 3H), 3.86 (s, 3H), 3.61 (d, *J* = 12.11 Hz, 2H), 3.45-3.35 (m, 4H), 3.14-3.06 (m, 2H), 3.03 (s, 3H), 2.21 (s, 3H), 2.17 (s, 3H); MS(ESI): m/z 638 [M+H]$^+$ | 80 | 7.85, 99, (b) |

| | | | | | |
|---|---|---|---|---|---|
| 22 | 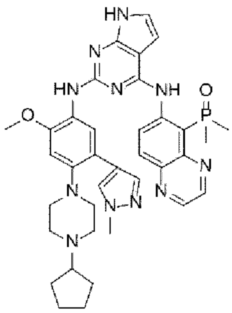 | (6-((2-((4-(4-cyclopentylpiperazin-1-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1$H NMR (400 MHz, FA salt, Metanol-$d_4$) δ 9.60 (dd, $J$ = 9.66 Hz, 1H), 8.80 (d, $J$ = 1.82 Hz, 1H), 8.72 (d, $J$ = 1.84 Hz, 1H), 8.38 (s, 1H), 8.37 (s, 1H), 7.96 (s, 1H), 7.77 (s, 1H), 7.57 (d, $J$ = 9.50 Hz, 1H), 6.99 (d, $J$ = 3.58 Hz, 1H), 6.86 (s, 1H), 6.60 (d, $J$ = 3.55 Hz, 1H), 3.98 (s, 3H), 3.81 (s, 3H), 3.56 (t, 1H), 3.43-3.35 (m, 3H), 3.22-3.11 (m, 4H), 2.23-2.15 (m, 9H), 1.88 (s, 2H), 1.72 (s, 4H); MS(ESI): m/z 692 [M+H]$^+$ | 47 | 8.49, 98, (b) |

| | | | | | |
|---|---|---|---|---|---|
| 23 | 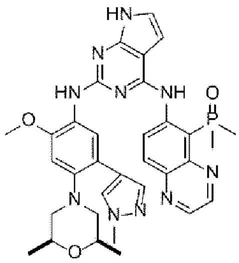 | (6-((2-((4-((2S,6R)-2,6-dimethylmorpholino)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1$H NMR (400 MHz, TFA salt, Methanol-$d_4$) δ 9.60-9.57 (m, 1H), 8.78 (d, *J* = 2.0 Hz, 1H), 8.71 (d, *J* = 2.0 Hz, 1H), 8.33 (s, 1H), 8.00 (s, 1H), 7.79 (s, 1H), 7.57 (d, *J* = 9.6 Hz, 1H), 6.98 (d, *J* = 3.6 Hz, 1H), 6.84 (s, 1H), 6.59 (d, *J* = 3.6 Hz, 1H), 3.97 (s, 3H), 3.90-3.86 (m, 2H), 3.80 (s, 3H), 3.00 (d, *J* = 10.8 Hz, 2H), 2.42 (t, *J* = 10.8, 10.8 Hz, 2 H), 2.20 (s, 3H), 2.16 (s, 3H), 1.19 (s, 3H), 1.17 (s, 3H); MS(ESI): m/z 653 [M+H]$^+$ | 17 | 6.41, 93, (b) |

| | | | | | |
|---|---|---|---|---|---|
| 24 | 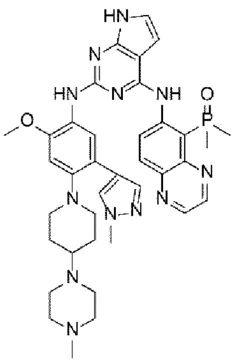 | (6-((2-((2-methoxy-5-(1-methyl-1H-pyrazol-4-yl)-4-(4-(4-methylpiperazin-1-yl)piperidin-1-yl)phenyl)amino)-7H-pyrrolo[2,3-d]pyrimidin-4-yl)amino)quinoxalin-5-yl)dimethylphosphine oxide | $^1H$ NMR (400 MHz, FA salt, MeOD-$d_4$) δ 9.60 (dd, *J* = 9.55 Hz, 1H), 8.78 (d, *J* = 1.83 Hz, 1H), 8.70 (d, *J* = 1.87 Hz, 1H), 8.29 (s, 1H), 8.25 (s, 3H), 7.92 (s, 1H), 7.81 (s, 1H), 7.56 (d, *J* = 9.56 Hz, 1H), 6.98 (d, *J* = 3.58 Hz, 1H), 6.84 (s, 1H), 6.59 (d, *J* = 3.54 Hz, 1H), 3.94 (s, 3H), 3.78 (s, 3H), 3.25 (d, *J* = 11.68 Hz, 2H), 3.14-2.96 (m, 8H), 2.71-2.67 (m, 6H), 2.18 (s, 3H), 2.15 (s, 3H), 2.03 (d, *J* = 16.73 Hz 2H), 1.77-1.69 (m, 2H): MS(ESI): m/z 721 [M+H]$^+$ | 66 | 7.53, 99, (b) |

| | | | | | |
|---|---|---|---|---|---|
| 25 | 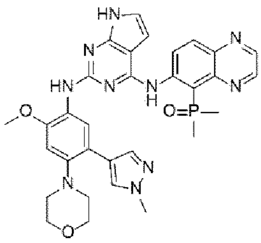 | (6-((2-((2-methoxy-5-(1-methyl-1H-pyrazol-4-yl)-4-morpholinophenyl)amino)-7H-pyrrolo[2,3-d]pyrimidin-4-yl)amino)quinoxalin-5-yl)dimethylphosphine oxide | $^1$H NMR (400 MHz, free, DMSO-$d_6$) δ 13.25 (s, 1H), 11.39 (s, 1H), 9.69 (dd, *J* = 9.6, 4.1 Hz, 1H), 8.85 (d, *J* = 1.9 Hz, 1H), 8.80 (d, *J* = 1.9 Hz, 1H), 8.19 (s, 1H), 8.16 (s, 1H), 7.91 (s, 1H), 7.79 (d, *J* = 9.2 Hz, 1H), 7.67 (s, 1H), 7.01 (dd, *J* = 3.4, 2.2 Hz, 1H), 6.85 (s, 1H), 6.49 (dd, *J* = 3.4, 1.8 Hz, 1H), 3.89 (s, 3H), 3.85 (s, 3H), 3.79-3.71 (m, 4H), 2.95-2.80 (m, 4H), 2.10 (s, 3H), 2.06 (s, 3H); MS(ESI): m/z 625 [M+H]$^+$ | 16 | 9.76, 99, (b) |

| | | | | | |
|---|---|---|---|---|---|
| 26 | 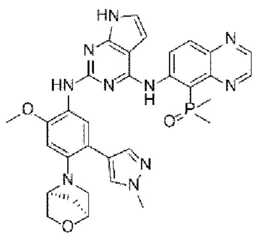 | (6-((2-((4-((1S,4S)-2-oxa-5-azabicyclo[2.2.1]heptan-5-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^{1}H$ NMR (400 MHz, Methanol-$d_4$) δ 9.60 (dd, $J$ = 9.6, 4.4 Hz, 1H), 8.76 (d, $J$ = 1.9 Hz, 1H), 8.70 (d, $J$ = 1.9 Hz, 1H), 8.11 (s, 1H), 7.75 (s, 1H), 7.58-7.53 (m, 2H), 6.94 (d, $J$ = 3.6 Hz, 1H), 6.73 (s, 1H), 6.56 (d, $J$ = 3.6 Hz, 1H), 4.60-4.51 (m, 2H), 4.18-4.16 (m, 1H), 4.05 (d, $J$ = 7.5 Hz, 1H), 3.95 (s, 3H), 3.82 (s, 3H), 3.15-3.02 (m, 2H), 2.17 (s, 3H), 2.14 (s, 3H), 2.04-2.00 (m, 1H), 1.88-1.84 (m, 1H); MS(ESI): m/z 637 [M+H]$^{+}$ | 8 | 9.29, 98, (b) |

| | | | | | |
|---|---|---|---|---|---|
| 27 | 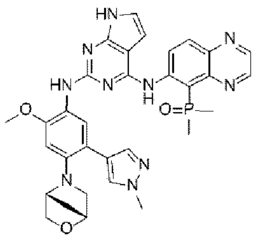 | (6-((2-((4-((1R,4R))-2-oxa-5-azabicyclo[2.2.1]heptan-5-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1$H NMR (400 MHz, free, Methanol-$d_4$) δ 9.63 (dd, $J$ = 9.6, 4.5 Hz, 1H), 8.79 (d, $J$ = 1.9 Hz, 1H), 8.72 (d, $J$ = 1.8 Hz, 1H), 8.11 (s, 1H), 7.77 (s, 1H), 7.59 (d, $J$ = 7.1 Hz, 2H), 6.95 (d, $J$ = 3.7 Hz, 1H), 6.75 (s, 1H), 6.59 (d, $J$ = 3.6 Hz, 1H), 4.58 (s, 1H), 4.54 (s, 1H), 4.19 (s, 1H), 4.06 (d, $J$ = 7.6 Hz, 1H), 3.96 (s, 3H), 3.83 (s, 3H), 3.76 (d, $J$ = 7.4 Hz, 1H), 3.14 (s, 1H), 3.07 (d, $J$ = 10.3 Hz, 1H), 2.19 (s, 3H), 2.15 (s, 3H), 2.03 (d, $J$ = 7.8 Hz, 1H), 1.88 (d, $J$ = 8.2 Hz, 1H); MS(ESI): m/z 637 [M+H]$^+$ | 31 | 9.30, 99, (b) |

| | | | | | |
|---|---|---|---|---|---|
| 28 | 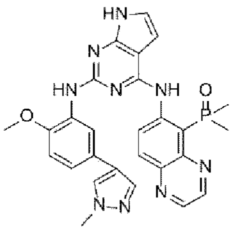 | (6-((2-((2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1$H NMR (400 MHz, TFA salt, Methanol-$d_4$) δ 9.62-9.58 (m, 1H), 8.82 (d, *J* = 1.6 Hz, 1H), 8.74 (d, *J* = 1.6 Hz, 1H), 8.57 (d, *J* = 2.0 Hz, 1H), 7.77 (s, 1H), 7.74 (d, *J* = 9.2 Hz, 1H), 7.61 (s, 1H), 7.23-7.20 (m, 1H), 7.06 (s, 1H), 7.04-7.03 (m, 1H), 6.64 (d, *J* = 3.6 Hz, 1H), 3.99 (s, 3H), 3.80 (s, 3H), 2.23 (s, 3H), 2.19 (s, 3H); MS(ESI): m/z 540 $[M+H]^+$ | 21 | 10.02, 100, (b) |
| 29 | 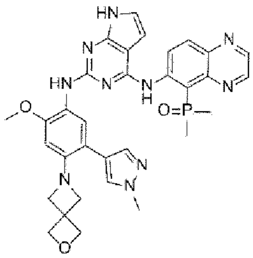 | (6-((2-((2-methoxy-5-(1-methyl-1H-pyrazol-4-yl)-4-(2-oxa-6-azaspiro[3.3 | $^1$H NMR (400 MHz, FA salt, Methanol-$d_4$) δ 9.60 (dd, *J* = 9.7, 4.4 Hz, 1H), 8.79 (d, *J* = 1.9 Hz, 1H), 8.74 (d, *J* = 1.9 Hz, 1H), 8.51 (s, 1H, FA), 7.95 (s, | 49 | 9.06, 99, (b) |

| | | | | | |
|---|---|---|---|---|---|
| | | ]heptan-6-yl)phenyl)amino)-7H-pyrrolo[2,3-d]pyrimidin-4-yl)amino)quinoxalin-5-yl)dimethylphosphine oxide | 1H), 7.66 (s, 1H), 7.60 (d, *J* = 9.4 Hz, 1H), 7.49 (s, 1H), 6.94 (d, *J* = 3.5 Hz, 1H), 6.57 (d, *J* = 3.6 Hz, 1H), 6.39 (s, 1H), 4.79 (s, 4H), 3.95 (s, 3H), 3.88 (s, 3H), 3.80 (s, 4H), 2.18 (s, 3H), 2.15 (s, 3H); MS(ESI): m/z 637 $[M+H]^+$ | | |
| 30 | 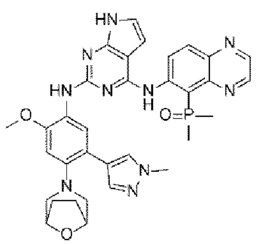 | (6-((2-((4-((1R,5S)-8-oxa-3-azabicyclo[3.2.1]octan-3-yl)-2-methoxy-5-(1-methyl-1H-pyrazol-4-yl)phenyl)amino)-7H- | $^1$H NMR (400 MHz, FA salt, DMSO-$d_6$) δ 13.27 (s, 1H), 11.39 (s, 1H), 9.72-9.68 (m, 1H), 8.87 (d, *J* = 2.0 Hz, 1H), 8.82 (d, *J* = 1.6 Hz, 1H), 8.29 (s, 1H), 8.01 (s, 1H), 7.91 (s, 1H), 7.81 (d, *J* = 2.4 Hz, 1H), 7.71 (s, 1H), 7.69 (s, 1H), 7.00 (d, *J* = 2.4 Hz, | 48 | 10.11, 100, (b) |

| | | | | | |
|---|---|---|---|---|---|
| | | pyrrolo[2,3-d]pyrimidin-4-yl)amino)quinoxalin-5-yl)dimethylphosphine oxide | 1H), 6.89 (s, 1H), 6.49 (d, *J* = 1.6 Hz, 1H), 4.27 (s, 2H), 3.89 (s, 3H), 3.87 (s, 3H), 2.92-2.91 (m, 2H), 2.85-2.83 (m, 2H), 2.10 (s, 3H), 2.07 (s, 3H), 1.94-1.92 (m, 2H), 1.91-1.88 (m, 2H); MS(ESI): m/z 651 $[M+H]^+$ | | |
| 31 | 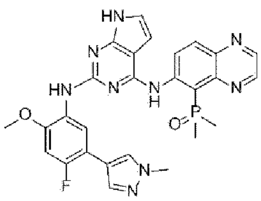 | (6-((2-((4-fluoro-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1H$ NMR (400 MHz, FA salt, DMSO-$d_6$) δ 13.23 (s, 1H), 11.43 (s, 1H), 9.67-9.63 (m, 1H), 8.80 (d, *J* = 2.0 Hz, 1H), 8.75 (d, *J* = 2.0 Hz, 1H), 8.45 (s, 1H), 8.39 (d, *J* = 8.0 Hz, 1H), 7.98 (d, *J* = 2.0 Hz, 1H), 7.83 (d, *J* = 9.6 Hz, 1H), 7.77 (s, 1H), 7.75 (s, 1H), | 37 | 10.33, 100, (b) |

| | | | | | |
|---|---|---|---|---|---|
| | | noxalin-5-yl)dimethylphosphine oxide | 7.04 (s, 1H), 7.01-6.98 (m, 1H), 6.44-6.43 (m, 1H), 3.83 (s, 3H), 3.80 (s, 3H), 2.05 (s, 3H), 2.04 (s, 3H); MS(ESI): m/z 558 [M+H]$^+$ | | |
| 32 | 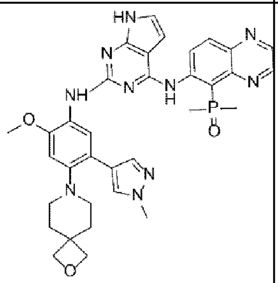 | (6-((2-((2-methoxy-5-(1-methyl-1H-pyrazol-4-yl)-4-(2-oxa-7-azaspiro[3.5]nonane-7-yl)phenyl)amino)-7H-pyrrolo[2,3-d]pyrimidin-4-yl)amino)quinoxalin-5-yl)dimethylphosphine | $^1$H NMR (400 MHz, TFA salt, Methanol-$d_4$) δ 9.56-9.53 (m, 1H), 8.82 (d, $J$ = 1.8 Hz, 1H), 8.76 (d, $J$ = 1.8 Hz, 1H), 8.16-8.12 (m, 1H), 8.04 (s, 1H), 7.78 (s, 1H), 7.62-7.58 (m, 1H), 7.00 (d, $J$ = 3.5 Hz, 1H), 6.85 (s, 1H), 6.64 (d, $J$ = 3.6 Hz, 1H), 4.53 (s, 4H), 3.94 (s, 3H), 3.82 (s, 3H), 2.89-2.83 (m, 4H), 2.20 (s, 3H), 2.16 (s, 3H), 2.05-2.01 (m, 4H); | 33 | 9.96, 99, (b) |

| | | | | | |
|---|---|---|---|---|---|
| | | oxide | MS(ESI): m/z 665 [M+H]+ | | |
| 33 | 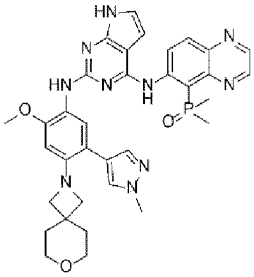 | (6-((2-((2-methoxy-5-(1-methyl-1H-pyrazol-4-yl)-4-(7-oxa-2-azaspiro[3.5]nonane-2-yl)phenyl)amino)-7H-pyrrolo[2,3-d]pyrimidin-4-yl)amino)quinoxalin-5-yl)dimethylphosphine oxide | $^1$H NMR (400 MHz, TFA salt, DMSO-$d_6$) δ 13.20 (s, 1H), 11.27 (s, 1H), 9.71-9.66 (m, 1H), 8.84 (d, *J* = 1.9 Hz, 1H), 8.79 (d, *J* = 1.9 Hz, 1H), 7.80-7.76 (m, 1H), 7.74 (s, 1H), 7.66 (s, 1H), 7.61 (s, 1H), 7.51 (s, 1H), 6.95 (dd, *J* = 3.4, 2.0 Hz, 1H), 6.44 (dd, *J* = 3.2, 1.3 Hz, 1H), 6.33 (s, 1H), 3.86-3.83 (m, 6H), 3.54-3.51 (m, 4H), 3.42-3.41 (m, 4H), 2.09 (s, 3H), 2.05 (s, 3H), 1.72-1.68 (m, 4H); MS(ESI): m/z 665 [M+H]+ | 16 | 9.72, 100, (b) |

| | | | | | |
|---|---|---|---|---|---|
| 34 | 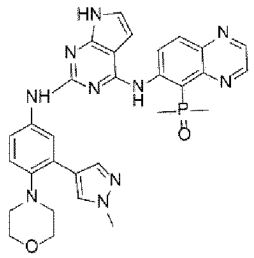 | dimethyl(6-((2-((3-(1-methyl-1H-pyrazol-4-yl)-4-morpholinophenyl)amino)-7H-pyrrolo[2,3-d]pyrimidin-4-yl)amino)quinoxalin-5-yl)phosphine oxide | $^1$H NMR (400 MHz, TFA salt, DMSO-$d_6$) δ 13.29 (s, 1H), 11.42 (s, 1H), 9.94 (dd, $J$ = 10.0, 3.6 Hz, 1H), 9.01 (s, 1H), 8.87 (d, $J$ = 1.8 Hz, 1H), 8.81 (d, $J$ = 1.9 Hz, 1H), 8.24 (s, 1H), 7.95 (dd, $J$ = 5.8, 3.0 Hz, 3H), 7.58 (dd, $J$ = 8.5, 2.5 Hz, 1H), 7.10 (d, $J$ = 8.8 Hz, 1H), 7.02 (dd, $J$ = 3.2, 2.5 Hz, 1H), 6.50 (dd, $J$ = 3.4, 1.8 Hz, 1H), 3.88 (s, 3H), 3.76-3.71 (m, 4H), 2.81-2.76 (m, 4H), 2.11 (s, 3H), 2.07 (s, 3H); MS(ESI): m/z 595 [M+H]$^+$ | 4 | 9.72, 97, (a) |

| | | | | | |
|---|---|---|---|---|---|
| 35 | 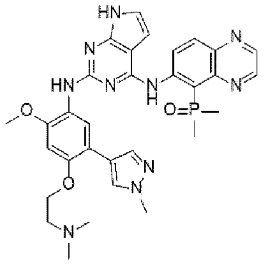 | (6-((2-((4-(2-(dimethylamino)ethoxy)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^{1}$H NMR (400 MHz, TFA salt, Methanol-$d_4$) δ 9.60-9.57 (m, 1H), 8.76 (d, *J* = 1.6 Hz, 1H), 8.68 (d, *J* = 2.0 Hz, 1H), 8.32 (s, 1H), 7.97 (s, 1H), 7.68 (s, 1H), 7.59 (d, *J* = 9.6 Hz, 1H), 6.96 (d, *J* = 3.6 Hz, 1H), 6.81 (s, 1H), 6.57 (d, *J* = 3.6 Hz, 1H), 4.26-4.24 (m, 2H), 3.96 (s, 3H), 3.81 (s, 3H), 3.00-2.97 (m, 2H), 2.47 (s, 6H), 2.18 (s, 3H), 2.01 (s, 3H); MS(ESI): m/z 627 [M+H]$^+$ | 13 | 7.75, 100, (b) |
| 36 | 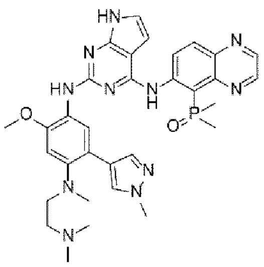 | (6-((2-((4-((2-(dimethylamino)ethyl)(methyl)amino)- | $^{1}$H NMR (400 MHz, FA salt, $CD_3OD$) δ 9.61-9.58 (m, 1H), 8.81 (d, *J* = 1.6 Hz, 1H), 8.73 (d, *J* = 2.0 Hz, 1H), | 31 | 8.23, 99, (b) |

| | | | | | |
|---|---|---|---|---|---|
| | | 2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | 8.50 (s, 1H), 8.39 (s, 1H), 7.90 (s, 1H), 7.68 (s, 1H), 7.61 (d, *J* = 9.6 Hz, 1H), 7.02 (s, 1H), 7.00 (d, *J* = 3.6 Hz, 1H), 6.61 (d, *J* = 3.6 Hz, 1H), 4.00 (s, 3H), 3.81 (s, 3H), 3.16 (t, *J* = 6.8 Hz, 2H), 2.80 (s, 3H), 2.72 (s, 6H), 2.65 (s, 2H), 2.19 (s, 3H), 2.16 (s, 3H); MS(ESI): m/z 640 $[M+H]^+$ | | |
| 37 | 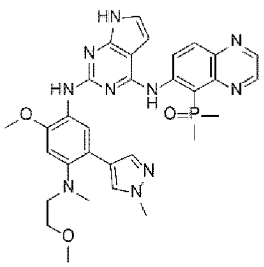 | (6-((2-((2-methoxy-4-((2-methoxyethyl)(methyl)amino)-5-(1-methyl-1H-pyrazol-4-yl)phenyl)am | $^1$H NMR (400 MHz, TFA salt, MeOD-$d_4$) δ 9.44-9.41 (m, 1H), 8.84-8.81 (m, 2H), 8.44 (s, 1H), 7.95 (s, 1H), 7.74 (d, *J* = 9.6 Hz, 1H), 7.60 (s, 1H), 7.29 (s, 1H), 7.06 (d, *J* = 3.6 Hz, 1H), 6.68 | 64 | 9.12, 99, (b) |

| | | | | | |
|---|---|---|---|---|---|
| | | ino)-7H-pyrrolo[2,3-d]pyrimidin-4-yl)amino)quinoxalin-5-yl)dimethylphosphine oxide | (d, $J$ = 3.6 Hz, 1H), 4.06 (s, 3H), 3.95 (s, 3H), 3.70 (s, 2H), 3.45 (s, 2H), 3.25 (s, 3H), 3.18 (s, 3H), 2.20 (s, 3H), 2.16 (s, 3H); MS(ESI): m/z 639 $[M+H]^+$ | | |
| 38 | 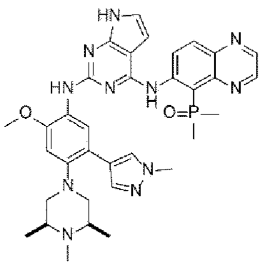 | (6-((2-((2-methoxy-5-(1-methyl-1H-pyrazol-4-yl)-4-((3S,5R)-3,4,5-trimethylpiperazin-1-yl)phenyl)amino)-7H-pyrrolo[2,3-d]pyrimidin-4-yl)amino)qui | $^1H$ NMR (400 MHz, FA salt, Methanol-$d_4$) δ 9.58 (dd, $J$ = 9.6, 4.4 Hz, 1H), 8.80 (d, $J$ = 1.9 Hz, 1H), 8.73 (d, $J$ = 1.9 Hz, 1H), 8.46 (s, 1H), 8.38 (s, 1H), 7.95 (s, 1H), 7.77 (s, 1H), 7.57 (d, $J$ = 9.4 Hz, 1H), 6.99 (d, $J$ = 3.6 Hz, 1H), 6.86 (s, 1H), 6.60 (d, $J$ = 3.6 Hz, 1H), 3.99 (s, 3H), 3.80 (s, 3H), 3.22 (d, $J$ = 11.2 Hz, 2H), | 26 | 8.17, 98, (b) |

| | | | | | |
|---|---|---|---|---|---|
| | | noxalin-5-yl)dimethylphosphine oxide | 2.84-2.73 (m, 5H), 2.19 (s, 3H), 2.16 (s, 3H), 1.33 (s, 3H), 1.31 (s, 3H), 0.93-0.74 (m, 2H); MS(ESI): m/z 666 $[M+H]^+$ | | |
| 39 | 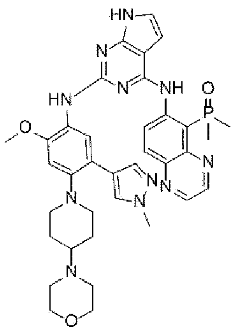 | (6-((2-((2-methoxy-5-(1-methyl-1H-pyrazol-4-yl)-4-(4-morpholinopiperidin-1-yl)phenyl)amino)-7H-pyrrolo[2,3-d]pyrimidin-4-yl)amino)quinoxalin-5-yl)dimethylphosphine oxide | $^1H$ NMR (400 MHz, TFA salt, Methanol-$d_4$) δ 9.37-9.35 (m, 1H), 8.86 (d, $J$ = 1.6 Hz, 1H), 8.80 (d, $J$ = 2.0 Hz, 1H), 7.95 (s, 1H), 7.89 (s, 1H), 7.84 (s, 1H), 7.65 (d, $J$ = 9.2 Hz, 1H), 7.06 (d, $J$ = 3.6 Hz, 1H), 6.93 (s, 1H). 6.73 (d, $J$ = 3.6 Hz, 1H), 4.16 (d, $J$ = 12.4 Hz, 2H), 3.93 (s, 3H), 3.82 (s, 3H), 3.63 (d, $J$ = 12.0 Hz, 2H), 3.48-3.32 (m, 6H), 3.13-3.12 (m, | 23 | 8.14, 99, (b) |

| | | | | | |
|---|---|---|---|---|---|
| | | | 1H), 2.83 (t, *J* = 11.6 Hz, 2H), 2.28 (d, *J* = 11.2 Hz, 2H), 2.20 (s, 3H), 2.16 (s, 3H), 1.93-1.85 (m, 2H); MS(ESI): m/z 708 $[M+H]^+$ | | |
| 40 | 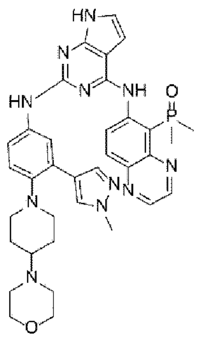 | dimethyl(6-((2-((3-(1-methyl-1H-pyrazol-4-yl)-4-(4-morpholinopiperidin-1-yl)phenyl)amino)-7H-pyrrolo[2,3-d]pyrimidin-4-yl)amino)quinoxalin-5-yl)phosphine oxide | $^1H$ NMR (400 MHz, FA salt, MeOD-$d_4$) δ 9.86 (dd, *J* = 9.59 Hz, 1H), 8.80 (d, *J* = 1.84 Hz, 1H), 8.72 (d, *J* = 1.86 Hz, 1H), 8.29 (s, 2H), 8.06 (s, 1H), 7.95 (s, 1H), 7.88 (d, *J* = 2.52 Hz, 1H), 7.85 (d, *J* = 9.56 Hz, 1H), 7.44 (dd, *J* = 8.59 Hz, 1H), 7.14 (d, *J* = 8.64 1H), 6.97 (d, *J* = 3.56 Hz, 1H), 6.58 (d, *J* = 3.56, 1H) 3.90 (d, 7H), 3.27 (t, 6H), 3.05 (t, 1H), 2.72 (t, | 52 | 8.06, 92, (b) |

| | | | | | |
|---|---|---|---|---|---|
| | | | 2H), 2.20 (d, 8H), 1.83-1.75 (m, 2H); MS(ESI): m/z 678 $[M+H]^+$ | | |
| 41 | 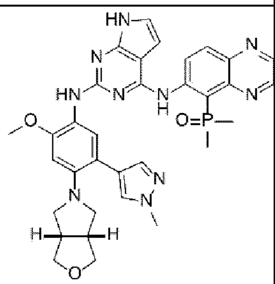 | (6-((2-((2-methoxy-5-(1-methyl-1H-pyrazol-4-yl)-4-((3aR,6aS)-tetrahydro-1H-furo[3,4-c]pyrrole-5(3H)-yl)phenyl)amino)-7H-pyrrolo[2,3-d]pyrimidin-4-yl)amino)quinoxalin-5-yl)dimethylphosphine oxide | $^1$H NMR (400 MHz, FA salt, Methanol-$d_4$) δ 9.61-9.59 (m, 1H), 8.79 (d, $J$ = 2.0 Hz, 1H), 8.72 (d, $J$ = 2.0 Hz, 1H), 8.22 (s, 1H), 8.20 (s, 1H), 8.10 (s, 1H), 7.66 (s, 1H), 7.56 (d, $J$ = 9.6 Hz, 1H), 6.97 (d, $J$ = 3.6 Hz, 1H), 6.83 (s, 1H), 6.59 (d, $J$ = 3.6 Hz, 1H), 3.96 (s, 3H), 3.90-3.84 (m, 2H), 3.76 (s, 3H), 3.75-3.73 (m, 2H), 3.34-3.32 (m, 2H), 3.06-3.04 (m, 2H), 3.00-2.97 (m, 2H), 2.20 (s, 3H), 2.16 (s, 3H); | 14 | 9.89, 95, (b) |

| | | | | | |
|---|---|---|---|---|---|
| | | | MS(ESI): 651 m/z [M+H]+ | | |
| 42 | 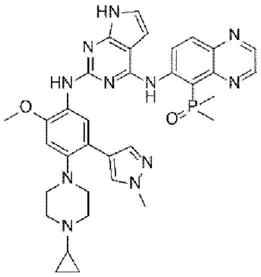 | (6-((2-((4-(4-cyclopropylpiperazin-1-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1$H NMR (400 MHz, TFA salt, $CD_3OD$) δ 9.60-9.57 (m, 1H), 8.81 (d, *J* = 2.0 Hz, 1H), 8.73 (d, *J* = 2.0 Hz, 1H), 8.38 (s, 1H), 8.01 (s, 1H), 7.76 (s, 1H), 7.56 (d, *J* = 9.6 Hz, 1H), 6.99 (d, *J* = 3.6 Hz, 1H), 6.85 (s, 1H), 6.60 (d, *J* = 3.6 Hz, 1H), 3.97 (s, 3H), 3.82 (s, 3H), 3.48-3.47 (m, 5H), 3.13-3.12 (m, 4H), 2.19 (s, 3H), 2.15 (s, 3H), 0.99 (s, 4H); MS(ESI): m/z 644 [M+H]+ | 50 | 4.07, 99, (b) |

| | | | | | |
|---|---|---|---|---|---|
| 43 | 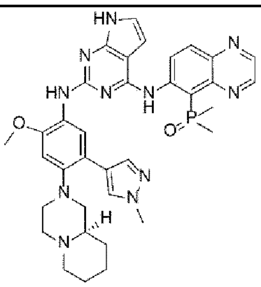 | (R)-(6-((2-((2-methoxy-5-(1-methyl-1H-pyrazol-4-yl)-4-(octahydro-2H-pyrido[1,2-a]pyrazin)-2-yl)phenyl)amino)-7H-pyrrolo[2,3-d]pyrimidin-4-yl)amino)quinoxalin-5-yl)dimethylphosphine oxide | $^1$H NMR (400 MHz, FA salt, Methanol-$d_4$) δ 9.58-9.54 (m, 1H), 8.78 (d, *J* = 2.0 Hz, 1 H), 8.72 (d, J = 1.6 Hz, 1H), 8.40 (s, 1H), 8.28 (s, 1H), 7.96 (s, 1H), 7.77 (s, 1H), 7.55 (d, *J* = 9.6 Hz, 1H), 6.99 (d, *J* = 3.6 Hz, 1H), 6.85 (s, 1H), 6.59 (d, *J* = 3.6 Hz, 1H), 3.99 (s, 3H), 3.82 (s, 3H), 3.48-2.91 (m, 9H), 2.19 (s, 3H), 2.16 (s, 3H), 2.00-1.93 (m, 4H), 1.89-1.86 (m, 2H); MS(ESI): m/z 678 [M+H]$^+$ | 51 | 8.26, 100, (b) |
| 44 | 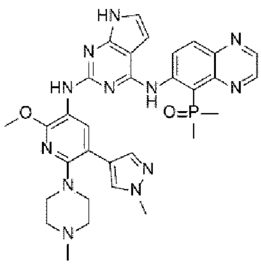 | (6-((2-((2-methoxy-5-(1-methyl-1H-pyrazol- | $^1$H NMR (400 MHz, FA salt, MeOD-$d_4$) δ 9.57-9.53 (m, 1H), 8.79 (d, *J* = 1.6 Hz, 1H), 8.71 | 60 | 8.74, 97, (b) |

| | | | | | |
|---|---|---|---|---|---|
| | | 4-yl)-6-(4-methylpiperazin-1-yl)pyridin-3-yl)amino)-7H-pyrrolo[2,3-d]pyrimidin-4-yl)amino)quinoxalin-5-yl)dimethylphosphine oxide | (d, *J* = 2 Hz, 1H), 8.66 (s, 1H), 8.31 (s, 2H), 7.94 (s, 1H), 7.75 (s, 1H), 7.66 (d, *J* = 9.2 Hz, 1H), 6.99 (d, *J* = 3.6 Hz, 1H), 6.58 (d, *J* = 3.6 Hz, 1H), 4.05 (s, 3H), 3.83 (s, 3H), 3.35 (s, 8H), 2.92 (s, 3H), 2.18 (s, 3H), 2.15 (s, 3H); MS(ESI): m/z 639 [M+H]$^+$ | | |
| 45 | 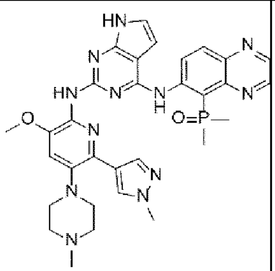 | (6-((2-((3-methoxy-6-(1-methyl-1H-pyrazol-4-yl)-5-(4-methylpiperazin-1-yl)pyridin-2-yl)amino)-7H- | MS(ESI): m/z 639 [M+H]$^+$ | | |

| | | | | | |
|---|---|---|---|---|---|
| | | pyrrolo[2,3-d]pyrimidin-4-yl)amino)quinoxalin-5-yl)dimethylphosphine oxide | | | |
| 46 | 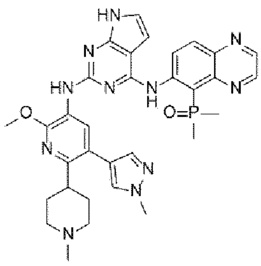 | (6-((2-((2-methoxy-5-(1-methyl-1H-pyrazol-4-yl)-6-(1-methylpiperidin-4-yl)pyridin-3-yl)amino)-7H-pyrrolo[2,3-d]pyrimidin-4-yl)amino)quinoxalin-5-yl)dimethylp | MS(ESI): m/z 638 $[M+H]^+$ | | |

| | | | | | |
|---|---|---|---|---|---|
| | | hosphine oxide | | | |
| 47 | 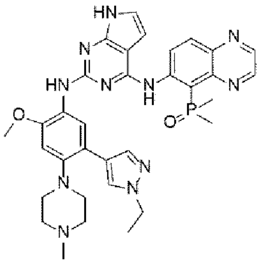 | (6-((2-((5-(1-ethyl-1H-pyrazol-4-yl)-2-methoxy-4-(4-methylpiperazin-1-yl)phenyl)amino)-7H-pyrrolo[2,3-d]pyrimidin-4-yl)amino)quinoxalin-5-yl)dimethylphosphine oxide | $^1$H NMR (400 MHz, FA salt, DMSO-$d_6$) δ 9.66 (dd, *J* = 9.54 Hz, 1H), 8.78 (d, *J* = 1.86 Hz, 1H), 8.70 (d, *J* = 1.88 Hz, 1H), 8.36 (s, 1H), 8.33 (s, 1H), 8.01 (s, 1H), 7.84 (s, 1H), 7.60 (d, *J* = 9.48 Hz, 1H), 6.98 (d, *J* = 3.60 Hz, 1H), 6.87 (s, 1H), 6.59 (d, *J* = 3.56 Hz, 1H), 4.12-4.07 (m, 2H), 3.98 (s, 3H), 3.22-3.08 (m, 4H), 2.90 (s, 3H), 2.19 (s, 3H), 2.15 (s, 3H), 1.35 (t, 3H); MS(ESI): m/z 652 [M+H]$^+$ | 56 | 8.14, 99, (b) |

| | | | | | |
|---|---|---|---|---|---|
| 48 | 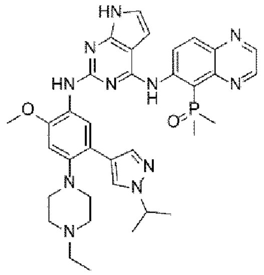 | (6-((2-((4-(4-ethylpiperazin-1-yl)-5-(1-isopropyl-1H-pyrazol-4-yl)-2-methoxyphenyl)amino)-7H-pyrrolo[2,3-d]pyrimidin-4-yl)amino)quinoxalin-5-yl)dimethylphosphine oxide | $^1$H NMR (400 MHz, Neutral, MeOD-$d_4$) δ 9.68-9.65 (m, 1H), (d, $J$ = 1.6 Hz, 1H), 8.67 (d, $J$ = 2 Hz, 1H), 8.27 (s, 1H), 8.07 (s, 1H), 7.84 (s, 1H), 7.59 (d, $J$ = 9.6 Hz, 1H), 6.96 (d, $J$ = 3.6 Hz, 1H), 6.88 (s, 1H), 6.58 (d, $J$ = 3.6 Hz, 1H), 4.44-4.37 (m, 1H), 3.96 (s, 3H), 2.99 (s, 4H), 2.67 (s, 4H), 2.59-2.53 (s, 1H), 2.18 (s, 3H), 2.14 (s, 3H), 1.39 (s, 3H), 1.38 (s, 3H), 1.17 (tr, d = 7.2 Hz, 3H); MS(ESI): m/z 680 $[M+H]^+$ | 33 | 8.44, 98, (b) |

| | | | | | |
|---|---|---|---|---|---|
| 49 | 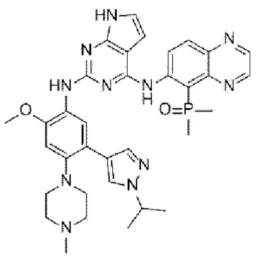 | (6-((2-((5-(1-isopropyl-1H-pyrazol-4-yl)-2-methoxy-4-(4-methylpiperazin-1-yl)phenyl)amino)-7H-pyrrolo[2,3-d]pyrimidin-4-yl)amino)quinoxalin-5-yl)dimethylphosphine oxide | $^{1}$H NMR (400 MHz, FA salt, $CD_3OD$) δ 9.69-9.65 (m, 1H), 8.78 (d, *J* = 2.0 Hz, 1H), 8.70 (d, *J* = 2.0 Hz, 1H), 8.31 (d, *J* = 4.0 Hz, 3H), 7.97 (s, 1H), 7.88 (s, 1H), 7.62 (d, *J* = 9.2 Hz, 1H), 6.98 (d, *J* = 3.6 Hz, 1H), 6.87 (s, 1H), 6.60 (d, *J* = 3.2 Hz, 1H), 4.44-4.38 (m, 1H), 3.97 (s, 3H), 3.25 (s, 4H), 3.15 (s, 4H), 2.86 (s, 3H), 2.18 (s, 3H), 2.14 (s, 3H), 1.36 (s, 3H), 1.35 (s, 3H); MS(ESI): m/z 666 $[M+H]^+$ | 27 | 8.12, 95, (b) |
| 50 | 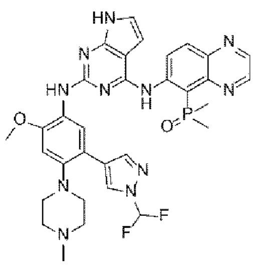 | (6-((2-((5-(1-(difluoromethyl)-1H- | $^{1}$H NMR (400 MHz, FA salt, Methanol-$d_4$) δ 9.58 (dd, *J* = 9.6, 4.3 Hz, 1H), 8.80 (d, *J* = | 20 | 9.70, 100, (b) |

| | | | | | |
|---|---|---|---|---|---|
| | | pyrazol-4-yl)-2-methoxy-4-(4-methylpiperazin-1-yl)phenyl)amino)-7H-pyrrolo[2,3-d]pyrimidin-4-yl)amino)quinoxalin-5-yl)dimethylphosphine oxide | 1.9 Hz, 1H), 8.71 (d, *J* = 1.9 Hz, 1H), 8.43 (s, 1H), 8.41 (s, 1H), 8.28 (s, 1H), 8.10 (s, 1H), 7.71 (d, *J* = 9.6 Hz, 1H), 7.33 (t, *J* = 59.9 Hz, 1H), 6.99 (d, *J* = 3.6 Hz, 1H), 6.92 (s, 1H), 6.60 (d, *J* = 3.6 Hz, 1H), 4.00 (s, 3H), 3.11-2.99 (m, 8H), 2.69 (s, 3H), 2.20 (s, 3H), 2.16 (s, 3H); MS(ESI): m/z 674 $[M+H]^+$ | | |
| 51 | 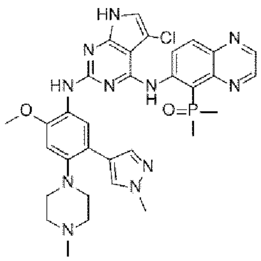 | (6-((5-chloro-2-((2-methoxy-5-(1-methyl-1H-pyrazol-4-yl)-4-(4-methylpiperazin-1- | $^1$H NMR (400 MHz, TFA salt, Methanol-$d_4$) δ 8.88 (d, *J* = 2.0 Hz, 1H), 8.80 (d, *J* = 1.6 Hz, 1H), 8.01-8.57 (m, 1H), 8.22 (s, 1H), 7.79 (s, 1H), 7.67 (d, *J* = 9.2 Hz, 1H), 7.40 | 44 | 8.55, 99, (b) |

| | | | | | |
|---|---|---|---|---|---|
| | | yl)phenyl)amino)-7H-pyrrolo[2,3-d]pyrimidin-4-yl)amino)quinoxalin-5-yl)dimethylphosphine oxide | (s, 1H), 7.06 (s, 1H), 6.84 (s, 1H), 3.97 (s, 3H), 3.72 (s, 3H), 3.55-3.50 (m, 2H), 3.35-3.28 (m, 4H), 3.05-2.98 (m, 5H), 2.21 (s, 3H), 2.17 (s, 3H); MS(ESI): m/z 672 $[M+H]^+$ | | |
| 52 | 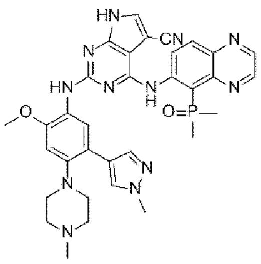 | 4-((5-(dimethylphosphoryl)quinoxalin-6-yl)amino)-2-((2-methoxy-5-(1-methyl-1H-pyrazol-4-yl)-4-(4-methylpiperazin-1)-yl)phenyl)amino)-7H-pyrrolo[2,3- | $^1$H NMR (400 MHz, TFA salt, Methanol-$d_4$) δ 8.87 (d, $J$ = 1.6 Hz, 1H), 8.80 (d, $J$ = 2.0 Hz, 1H), 8.64-8.61 (m, 1H), 8.34 (s, 1H), 7.83 (s, 1H), 7.81 (s, 1H), 7.72 (d, $J$ = 9.6 Hz, 1H), 7.49 (s, 1H), 6.84 (s, 1H), 3.97 (s, 3H), 3.74 (s, 3H), 3.57-3.50 (m, 2H), 3.30-3.27 (m, 4H), 3.04-2.98 (m, 5H), | 39 | 8.34, 100, (b) |

| | | | | | |
|---|---|---|---|---|---|
| | | d]pyrimidin-5-carbonitrile | 2.20 (s, 3H), 2.16 (s, 3H); MS(ESI): m/z 663 [M+H]+ | | |
| 53 | 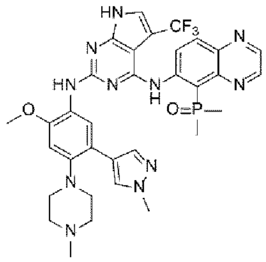 | (6-((2-((2-methoxy-5-(1-methyl-1H-pyrazol-4-yl)-4-(4-methylpiperazin-1-yl)phenyl)amino)-5-(trifluoromethyl)-7H-pyrrolo[2,3-d]pyrimidin-4-yl)amino)quinoxalin-5-yl)dimethylphosphine oxide | $^1$H NMR (400 MHz, FA salt, Methanol-$d_4$) δ 8.84 (d, *J* = 2.0 Hz, 1H), 8.74 (d, *J* = 2.0 Hz, 1H), 8.39 (s, 1H), 8.32 (s, 1H), 8.30-8.27 (m, 1H), 7.75 (s, 1H), 7.68 (d, *J* = 9.6 Hz, 1H), 7.53 (s, 1H), 7.29 (s, 1H), 6.79 (s, 1H), 3.93 (s, 3H), 3.68 (s, 3H), 3.12 (s, 4H), 3.02 (s, 4H), 2.78 (s, 3H), 2.17 (s, 3H), 2.14 (s, 3H); MS(ESI): m/z 706 [M+H]+ | 27 | 9.12, 99, (b) |

| | | | | | |
|---|---|---|---|---|---|
| 54 | 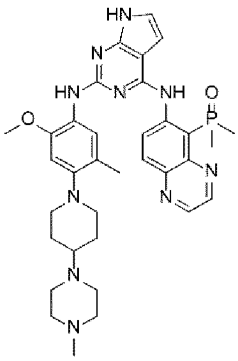 | (6-((2-((2-methoxy-5-methyl-4-(4-(4-methylpiperazin-1-yl)piperidin-1-yl)phenyl)amino)-7H-pyrrolo[2,3-d]pyrimidin-4-yl)amino)quinoxalin-5-yl)dimethylphosphine oxide | $^1$H NMR (400 MHz, TFA salt, DMSO-$d_6$) δ 13.24 (s, 1H), 11.33 (s, 1H), 9.73-9.69 (m, 1H), 8.86 (d, *J* = 1.6 Hz, 1H), 8.81 (d, *J* = 1.6 Hz, 1H), 8.20 (s, 1H), 8.07 (d, *J* = 9.6 Hz, 1H), 7.85 (s, 1H), 7.60 (s, 1H), 6.99 (t, *J* = 3.2 Hz, 1H), 6.74 (s, 1H), 6.47 (t, *J* = 3.2 Hz, 1H), 3.81 (s, 3H), 3.12-3.09 (m, 2H), 2.68-2.62 (m, 4H), 2.60-2.52 (m, 4H), 2.34-2.28 (m, 3H), 2.34 (s, 3H), 2.28 (s, 3H), 2.16 (s, 3H), 2.09 (s, 3H), 1.90-1.85 (m, 2H), 1.63-1.55 (m, 2H); MS(ESI): m/z 655 [M+H]$^+$ | 50 | 4.07, 99, (a) |

| | | | | | |
|---|---|---|---|---|---|
| 55 | 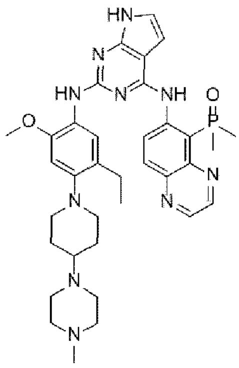 | (6-((2-((5-ethyl-2-methoxy-4-(4-(4-methylpiperazin-1-yl)piperidin-1-yl)phenyl)amino)-7H-pyrrolo[2,3-d]pyrimidin-4-yl)amino)quinoxalin-5-yl)dimethylphosphine oxide | $^1$H NMR (400 MHz, FA salt, DMSO-$d_6$) δ 13.23 (s, 1H), 11.33 (s, 1H), 9.70 (dd, $J$ = 9.5, 4.0 Hz, 1H), 8.86 (d, $J$ = 1.9 Hz, 1H), 8.81 (d, $J$ = 1.9 Hz, 1H), 8.23 (s, 2H), 8.06 (d, $J$ = 9.5 Hz, 1H), 7.91 (s, 1H), 7.63 (s, 1H), 6.99-6.97 (m, 1H), 6.80 (s, 1H), 6.48-6.46 (m, 1H), 3.82 (s, 3H), 3.06-3.00 (m, 2H), 2.74-2.68 (m, 2H), 2.62 (q, $J$ = 7.5 Hz, 2H), 2.56-2.46 (m, 3H), 2.38-2.30 (m, 6H), 2.16 (s, 3H), 2.10 (s, 3H), 2.06 (s, 3H), 1.89-1.83 (m, 2H), 1.64-1.53 (m, 2H), 1.14 (t, $J$ = 7.5 | 49 | 4.38, 98, (a) |

| | | | | | |
|---|---|---|---|---|---|
| | | | Hz, 3H); MS(ESI): m/z 669 [M+H]+ | | |
| 56 | 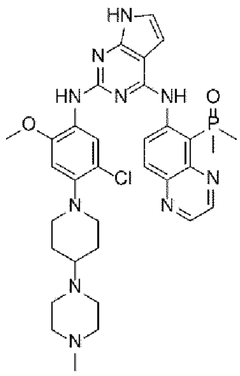 | (6-((2-((5-chloro-2-methoxy-4-(4-(4-methylpiperazin-1-yl)piperidin-1-yl)phenyl)amino)-7H-pyrrolo[2,3-d]pyrimidin-4-yl)amino)quinoxalin-5-yl)dimethylphosphine oxide | $^{1}$H NMR (400 MHz, FA salt, Methanol-$d_4$) δ 9.68 (dd, *J* = 9.6, 4.3 Hz, 1H), 8.82 (d, *J* = 1.9 Hz, 1H), 8.74 (s, 1H), 8.42 (s, 1H), 8.41 (s, 1H), 8.21 (d, *J* = 9.5 Hz, 1H), 6.99 (d, *J* = 3.6 Hz, 1H), 6.82 (s, 1H), 6.61 (d, *J* = 3.6 Hz, 1H), 3.95 (s, 3H), 3.49-3.40 (m, 3H), 3.10-2.95 (m, 7H), 2.80-2.71 (m, 3H), 2.67 (s, 3H), 2.21 (s, 3H), 2.17 (s, 3H), 2.11-2.02 (m, 2H), 1.91-1.77 (m, 2H); MS(ESI): m/z 675 [M+H]+ | 66 | 4.17, 100, (a) |

| | | | | | |
|---|---|---|---|---|---|
| 57 | 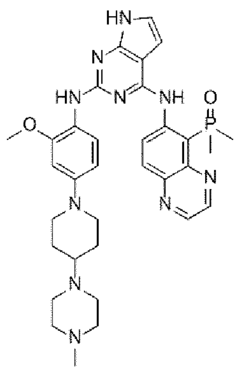 | (6-((2-((2-methoxy-4-(4-(4-methylpiperazin-1-yl)piperidin-1-yl)phenyl)amino)-7H-pyrrolo[2,3-d]pyrimidin-4-yl)amino)quinoxalin-5-yl)dimethylphosphine oxide | $^1$H NMR (400 MHz, FA salt, Methanol-$d_4$) δ 9.64 (dd, *J* = 9.5, 4.2 Hz, 1H), 8.74 (s, 1H), 8.67 (s, 1H), 8.45 (s, 2H), 7.97 (d, *J* = 9.6 Hz, 1H), 7.91 (d, *J* = 8.6 Hz, 1H), 6.92 (d, *J* = 2.9 Hz, 1H), 6.67 (s, 1H), 6.56-6.50 (m, 1H), 3.87 (s, 3H), 3.71-3.62 (m, 2H), 3.14-2.85 (m, 8H), 2.75-2.58 (m, 6H), 2.17 (s, 3H), 2.14 (s, 3H), 2.03-1.96 (m, 2H), 1.76-1.66 (m, 2H); MS(ESI): m/z 641 [M+H]$^+$ | 99 | 3.86, 99, (a) |

| | | | | | |
|---|---|---|---|---|---|
| 58 | 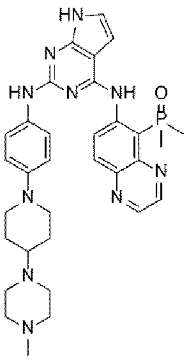 | dimethyl(6-((2-((4-(4-(4-methylpiperazin-1-yl)piperidin-1-yl)phenyl)amino)-7H-pyrrolo[2,3-d]pyrimidin-4-yl)amino)quinoxalin-5-yl)phosphine oxide | $^1$H NMR (400 MHz, FA salt, DMSO-$d_6$) δ 13.26 (s, 1H), 11.35 (s, 1H), 9.96 (dd, $J$ = 9.7, 3.7 Hz, 1H), 8.87 (s, 2H), 8.82 (d, $J$ = 1.7 Hz, 1H), 8.21 (s, 2H), 8.11 (d, $J$ = 9.6 Hz, 1H), 7.62 (d, $J$ = 8.8 Hz, 2H), 7.02-6.95 (m, 1H), 6.92 (d, $J$ = 9.0 Hz, 2H), 6.50-6.42 (m, 1H), 3.63 (d, $J$ = 11.9 Hz, 4H), 2.65-2.55 (m, 5H), 2.30 (d, $J$ = 18.4 Hz, 4H), 2.15 (s, 3H), 2.11 (s, 3H), 2.07 (s, 3H), 1.85 (d, $J$ = 10.8 Hz, 2H), 1.62-1.45 (m, 2H); MS(ESI): m/z 611 [M+H]$^+$ | 32 | 3.79, 99, (a) |

| | | | | | |
|---|---|---|---|---|---|
| 59 | 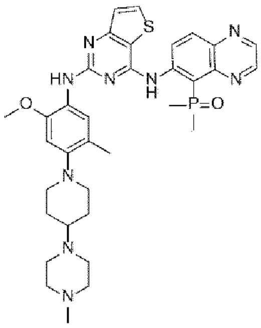 | (6-((2-((2-methoxy-5-methyl-4-(4-(4-methylpiperazin-1-yl)piperidin-1-yl)phenyl)amino)thieno[3,2-d]pyrimidin-4-yl)amino)quinoxalin-5-yl)dimethylphosphine oxide | $^1$H NMR (400 MHz, FA salt, DMSO-$d_6$) δ 13.35 (s, 1H), 9.43-9.41 (m, 1H), 8.89 (s, 1H), 8.86 (s, 1H), 8.13 (d, *J* = 5.2 Hz, 1H), 8.02 (d, *J* = 9.2 Hz, 1H), 7.98 (s, 1H), 7.66 (s, 1H), 7.22 (d, *J* = 5.2 Hz, 1H), 6.77 (s, 1H), 3.80 (s, 3H), 3.14-3.11 (m, 2H), 2.67-2.65 (m, 2H), 2.58 (s, 3H), 2.36-2.32 (m, 9H), 2.19 (s, 3H), 2.10 (s, 3H), 1.89-1.87 (m, 2H), 1.66-1.62 (m, 2H); MS(ESI): m/z 611 [M+H]$^+$ | 39 | 100* |

| | | | | | |
|---|---|---|---|---|---|
| 60 | 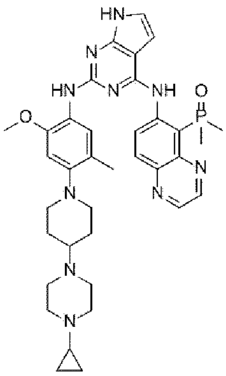 | (6-((2-((4-(4-(4-cyclopropylpiperazin-1-yl)piperidin-1-yl)-2-methoxy-5-methylphenyl)amino)-7H-pyrrolo[2,3-d]pyrimidin-4-yl)amino)quinoxalin-5-yl)dimethylphosphine oxide | $^1$H NMR (400 MHz, FA salt, DMSO-$d_6$) δ 13.24 (s, 1H), 11.33 (s, 1H), 9.71 (dd, $J$ = 9.6, 4.0 Hz, 1H), 8.86 (d, $J$ = 1.9 Hz, 1H), 8.81 (d, $J$ = 1.9 Hz, 1H), 8.18 (s, 1H, FA), 8.06 (d, $J$ = 9.6 Hz, 1H), 7.86 (s, 1H), 7.58 (s, 1H), 6.98 (dd, $J$ = 3.4, 2.3 Hz, 1H), 6.75 (s, 1H), 6.47 (dd, $J$ = 3.4, 1.9 Hz, 1H), 3.82 (s, 3H), 3.35-3.31 (m, 4H), 3.11 (d, $J$ = 10.8 Hz, 3H), 2.73-2.59 (m, 3H), 2.30-2.22 (m, 1H), 2.21 (s, 3H), 2.10 (s, 3H), 2.06 (s, 3H), 1.86 (d, $J$ = 12.4 Hz, 2H), 1.67-1.50 (m, 3H), 0.48-0.32 | 45 | 4.35, 99, (a) |

| | | | | | |
|---|---|---|---|---|---|
| | | | (m, 2H), 0.31-0.22 (m, 2H); MS(ESI): m/z 681 [M+H]+ | | |
| 61 | 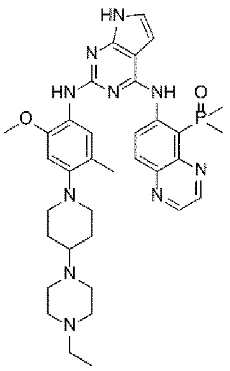 | (6-((2-((4-(4-(4-ethylpiperazin-1-yl)piperidin-1-yl)-2-methoxy-5-methylphenyl)amino)-7H-pyrrolo[2,3-d]pyrimidin-4-yl)amino)quinoxalin-5-yl)dimethylphosphine oxide | $^1$H NMR (400 MHz, TFA salt, DMSO-$d_6$) δ 9.52-9.42 (m, 1H), 8.89 (d, *J* = 2 Hz, 1H), 8.83 (d, *J* = 1.6 Hz, 1H), 8.07 (d, *J* = 9.6 Hz, 1H), 7.60 (s, 1H), 7.06 (d, *J* = 3.6 Hz, 1H), 6.93 (s, 1H), 6.75 (d, *J* = 3.6 Hz, 1H), 3.98 (s, 3H), 3.48-3.10 (m, 10 H), 3.04-3.10 (m, 4H), 2.32 (s, 3H), 2.24-2.10 (m, 9H), 2.00-1.88 (m, 2H), 1.37 (t, *J* = 7.2 Hz, 3H); MS(ESI): m/z 669 [M+H]+ | 41 | 4.15, 99, (a) |

| | | | | | |
|---|---|---|---|---|---|
| 62 | 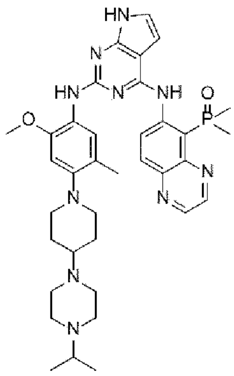 | (6-((2-((4-(4-(4-isopropylpiperazin-1-yl)piperidin-1-yl)-2-methoxy-5-methylphenyl)amino)-7H-pyrrolo[2,3-d]pyrimidin-4-yl)amino)quinoxalin-5-yl)dimethylphosphine oxide | $^1$H NMR (400 MHz, TFA salt, DMSO-$d_6$) δ 9.50-9.40 (m, 1H), 8.89 (d, *J* = 2 Hz, 1H), 8.84 (d, *J* = 2 Hz, 1H), 8.07 (d, *J* = 9.6 Hz, 1H), 7.57 (s, 1H), 7.06 (d, *J* = 3.6 Hz, 1H), 6.94 (s, 1H), 6.75 (d, *J* = 3.6 Hz, 1H), 3.89 (s, 3H), 3.60-3.30 (m, 9 H), 3.10-2.90 (m, 4H), 2.32 (s, 3H), 2.20-2.10 (m, 9H), 2.02-1.98 (m, 2H), 1.39 (s, 3H), 1.38 (s, 3H); MS(ESI): m/z 683 $[M+H]^+$ | 56 | 4.10, 99, (a) |
| 63 | 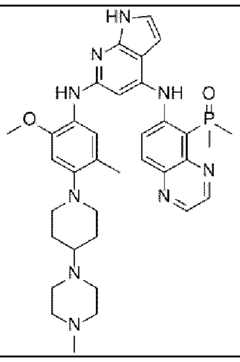 | (6-((6-((2-methoxy-5-methyl-4-(4-(4-methylpipera | $^1$H NMR (400 MHz, FA salt, DMSO-$d_6$) δ 12.12 (s, 1H), 11.11 (s, 1H), 8.81 (d, *J* = 1.9 Hz, 1H), 8.74 (d, *J* = | 53 | 3.96, 99, (a) |

| | | | | | |
|---|---|---|---|---|---|
| | | zin-1-yl)piperidin-1-yl)phenyl)amino)-1H-pyrrolo[2,3-b]pyridin-4-yl)amino)quinoxalin-5-yl)dimethylphosphine oxide | 1.9 Hz, 1H), 8.18-8.07 (m, 3H), 8.04 (s, 1H), 7.64 (s, 1H), 6.97 (dd, *J* = 3.3, 2.5 Hz, 1H), 6.76 (s, 1H), 6.69 (s, 1H), 6.28 (dd, *J* = 3.4, 2.1 Hz, 1H), 3.80 (s, 3H), 3.05 (d, *J* = 11.6 Hz, 2H), 2.61 (t, *J* = 11.6 Hz, 2H), 2.41-2.34 (m, 7H), 2.31-2.25 (m, 2H), 2.20 (s, 3H), 2.17 (s, 3H), 2.05 (s, 3H), 2.02 (s, 3H), 1.84 (d, *J* = 11.4 Hz, 2H), 1.62-1.50 (m, 2H); MS(ESI): m/z 654 $[M+H]^+$ | | |

| | | | | | |
|---|---|---|---|---|---|
| 64 | 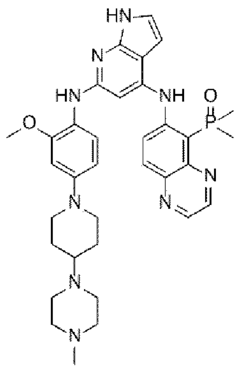 | (6-((6-((2-methoxy-4-(4-(4-methylpiperazin-1-yl)piperidin-1-yl)phenyl)amino)-1H-pyrrolo[2,3-b]pyridin-4-yl)amino)quinoxalin-5-yl)dimethylphosphine oxide | $^1$H NMR (400 MHz, FA salt, Methanol-$d_4$) δ 8.77 (d, $J$ = 2 Hz, 1H), 8.68 (d, $J$ = 2 Hz, 1H), 8.33 (s, 2H), 8.12-8.00 (m, 2H), 6.68 (s, 1H), 6.72-6.38 (m, 3H), 3.98-3.60 (m, 5H), 3.40-3.30 (m, 1H), 3.20-2.88 (m, 8H), 2.80-2.70 (m, 5H), 2.20-2.10 (m, 6H), 2.08-1.98 (m, 2H), 1.80-1.72 (m, 2H); MS(ESI): m/z 640 [M+H]$^+$ | 36 | 3.78, 99, (a) |
| 65 | 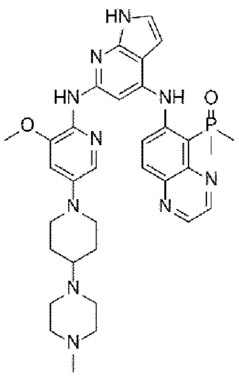 | (6-((6-((3-methoxy-5-(4-(4-methylpiperazin-1-yl)piperidin-1- | $^1$H NMR (400 MHz, FA salt, Methanol-$d_4$) δ 8.82 (d, $J$ = 1.7 Hz, 1H), 8.74 (d, $J$ = 1.8 Hz, 1H), 8.50 (s, 1H), 8.32 (dd, $J$ = 9.4, 4.1 Hz, 1H), 8.19 (d, $J$ = | 5 | 3.84, 97, (a) |

| | | | | | |
|---|---|---|---|---|---|
| | | yl)pyridin-2-yl)amino)-1H-pyrrolo[2,3-b]pyridin-4-yl)amino)quinoxalin-5-yl)dimethylphosphine oxide | 9.4 Hz, 1H), 7.70 (s, 1H), 7.45 (s, 1H), 7.23 (s, 1H), 7.17 (d, *J* = 3.4 Hz, 1H), 6.58 (d, *J* = 3.4 Hz, 1H), 4.01 (s, 3H), 3.72 (d, *J* = 11.7 Hz, 2H), 3.03-2.83 (m, 7H), 2.82-2.72 (m, 2H), 2.65-2.55 (m, 4H), 2.18 (s, 3H), 2.15 (s, 3H), 2.07-2.00 (m, 2H), 1.77-1.64 (m, 2H); MS(ESI): m/z 641 $[M+H]^+$ | | |
| 66 | 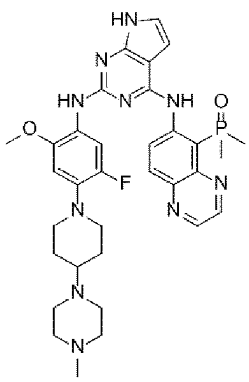 | (6-((2-((5-fluoro-2-methoxy-4-(4-(4-methylpiperazin-1-yl)piperidin-1-yl)phenyl)am | $^1$H NMR (400 MHz, FA salt, DMSO-$d_6$) δ 13.28 (s, 1H), 11.47 (s, 1H), 9.74 (dd, *J* = 9.5, 4.1 Hz, 1H), 8.87 (d, *J* = 1.9 Hz, 1H), 8.83 (d, *J* = 1.9 Hz, 1H), 8.27-8.06 (m, 3H), 7.67 (s, 1H), | 40 | 4.30, 99, (a) |

| | | | | | |
|---|---|---|---|---|---|
| | | ino)-7H-pyrrolo[2,3-d]pyrimidin-4-yl)amino)quinoxalin-5-yl)dimethylphosphine oxide | 7.03 (dd, *J* = 3.4, 2.3 Hz, 1H), 6.72 (d, *J* = 8.3 Hz, 1H), 6.49 (dd, *J* = 3.4, 1.9 Hz, 1H), 3.88 (s, 3H), 3.35-3.31 (m, 6H), 2.70 (t, *J* = 9.3 Hz, 2H), 2.56-2.53 (m, 2H), 2.36-2.33 (m, 2H), 2.32-2.23 (m, 1H), 2.17 (s, 3H), 2.11 (s, 3H), 2.07 (s, 3H), 1.86 (d, *J* = 11.3 Hz, 2H), 1.65-1.51 (m, 2H); MS(ESI): m/z 659 $[M+H]^+$ | | |
| 67 | 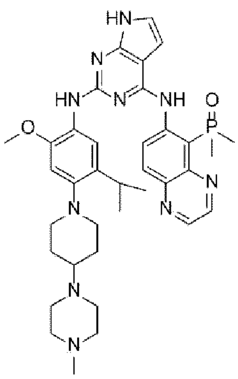 | (6-((2-((5-isopropyl-2-methoxy-4-(4-(4-methylpiperazin-1-yl)piperidin-1- | $^1$H NMR (400 MHz, FA salt, Methanol-$d_4$) δ 9.64 (dd, *J* = 9.6, 4.3 Hz, 1H), 8.81 (d, *J* = 1.9 Hz, 1H), 8.72 (d, *J* = 1.9 Hz, 1H), 8.46 (s, 1H), 8.07-8.02 (m, 2H), 6.96 (d, *J* = | 28 | 8.10, 100, (a) |

| | | | | | |
|---|---|---|---|---|---|
| | | yl)phenyl)amino)-7H-pyrrolo[2,3-d]pyrimidin-4-yl)amino)quinoxalin-5-yl)dimethylphosphine oxide | 3.6 Hz, 1H), 6.85 (s, 1H), 6.59 (d, *J* = 3.6 Hz, 1H), 4.58 (s, 3H), 3.88 (s, 3H), 3.14-3.09 (m, 4H), 3.01-2.88 (m, 4H), 2.86-2.80 (m, 3H), 2.66-2.65 (m, 1H), 2.59-2.55 (m, 2H), 2.20 (s, 3H), 2.16 (s, 3H), 2.09-2.03 (m, 2H), 1.83-1.73 (m, 2H), 1.17 (d, *J* = 6.9 Hz, 6H); MS(ESI): m/z 683 $[M+H]^+$ | | |
| 68 | 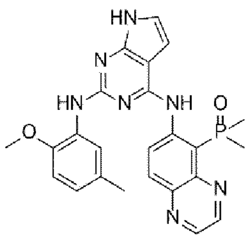 | (6-((2-((2-methoxy-5-methylphenyl)amino)-7H-pyrrolo[2,3-d]pyrimidin-4-yl)amino)quinoxalin-5-yl)dimethylphosphine oxide | $^1H$ NMR (400 MHz, FA salt, Methanol-$d_4$) δ 9.77-9.73 (m, 1H), 8.84 (d, *J* = 2.0 Hz, 1H), 8.76 (d, *J* = 1.6 Hz, 1H), 8.53 (s, 1H), 8.18 (s, 1H), 8.16 (d, *J* = 9.6 Hz, 1H), 7.01 (d, *J* = 3.6 Hz, 1H), | 21 | 11.03, 100, (b) |

| | | | | | |
|---|---|---|---|---|---|
| | | yl)dimethylphosphine oxide | 6.94 (d, *J* = 8.4 Hz, 1H), 6.84 (d, *J* = 8.4 Hz, 1H), 6.63 (d, *J* = 3.6 Hz, 1H), 3.93 (s, 3H), 2.32 (s, 3H), 2.23 (s, 3H), 2.19 (s, 3H); MS(ESI): m/z 474 $[M+H]^+$ | | |
| 69 | 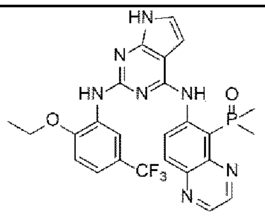 | (6-((2-((2-ethoxy-5-(trifluoromethyl)phenyl)amino)-7H-pyrrolo[2,3-d]pyrimidin-4-yl)amino)quinoxalin-5-yl)dimethylphosphine oxide | $^1H$ NMR (400 MHz, Neutral, MeOD-$d_4$) δ 9.66-9.62 (m, 1H), 8.82 (d, *J* = 2 Hz, 1H), 8.79 (d, *J* = 2 Hz, 1H), 8.75 (d, *J* = 2 Hz, 1H), 8.14 (d, *J* = 9.6 Hz, 1H), 7.25-7.23 (m, 1H), 7.12 (d, *J* = 8.4 Hz, 1H), 7.03 (d, *J* = 3.6 Hz, 1H), 6.64 (d, *J* = 3.6 Hz, 1H), 4.30-4.25 (m, 2H), 2.21 (s, 3H), 2.18 (s, 3H), 1.55 (tr, *J* = 6.8 Hz, 3H), | 80 | 13.96, 99, (b) |

| | | | | | |
|---|---|---|---|---|---|
| | | | 1.32-1.24 (m, 3H); MS(ESI): m/z 542 $[M+H]^+$ | | |
| 70 | 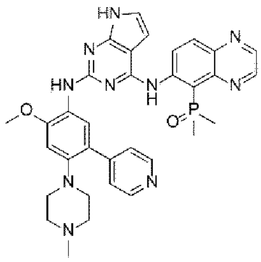 | (6-((2-((2-methoxy-4-(4-methylpiperazin-1-yl)-5-(pyridin-4-yl)phenyl)amino)-7H-pyrrolo[2,3-d]pyrimidin-4-yl)amino)quinoxalin-5-yl)dimethylphosphine oxide | $^1$H NMR (400 MHz, TFA salt, Methanol-$d_4$) δ 9.39-9.36 (m, 1H), 8.71 (d, *J* = 2.0 Hz, 1H), 8.63 (d, *J* = 2.0 Hz, 1H), 8.38 (s, 1H), 8.27 (s, 1H), 8.52 (s, 1H), 7.60-7.59 (m, 3H), 6.89 (d, *J* = 3.6 Hz, 1H), 6.80 (s, 1H), 6.49 (d, *J* = 3.2 Hz, 1H), 4.54-4.53 (m, 2H), 3.94 (s, 3H), 3.03-2.98 (m, 6H), 2.69 (s, 3H), 2.09 (s, 3H), 2.05 (s, 3H); MS(ESI): m/z 635 $[M+H]^+$ | 29 | 7.38, 100, (b) |

| | | | | | |
|---|---|---|---|---|---|
| 71 | 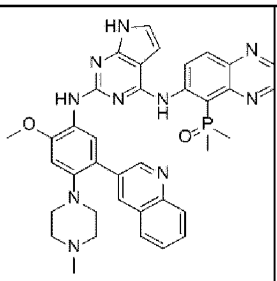 | (6-((2-((2-methoxy-4-(4-methylpiperazin-1-yl)-5-(quinolin-3-yl)phenyl)amino)-7H-pyrrolo[2,3-d]pyrimidin-4-yl)amino)quinoxalin-5-yl)dimethylphosphine oxide | $^1$H NMR (400 MHz, TFA salt, Methanol-$d_4$) δ 9.56 (s, 1H), 9.25-9.22 (m, 1H), 8.67 (d, $J$ = 2.0 Hz, 2H), 8.53 (d, $J$ = 1.6 Hz, 2H), 8.12 (d, $J$ = 8.4 Hz, 1H), 7.90 (t, $J$ = 7.2 Hz, 1H), 7.74 (d, $J$ = 8.0 Hz, 1H), 7.59 (t, $J$ = 7.6 Hz, 1H), 7.49 (d, $J$ = 9.2 Hz, 1H), 7.10 (s, 1H), 7.09 (s, 1H), 6.67 (d, $J$ = 3.6 Hz, 1H), 4.12 (s, 3H), 3.51-3.48 (m, 2H), 3.37-3.35 (m, 2H), 3.18-3.06 (m, 4H), 2.98 (s, 3H), 2.21 (s, 3H), 2.19 (s, 3H); MS(ESI): m/z 685 [M+H]$^+$ | 73 | 7.97, 100, (b) |

| | | | | | |
|---|---|---|---|---|---|
| 72 | 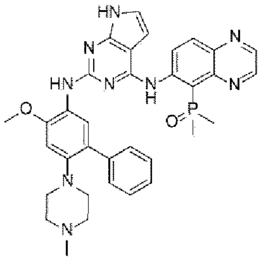 | (6-((2-((4-methoxy-6-(4-methylpiperazin-1-yl)-[1,1'-biphenyl]-3-yl)amino)-7H-pyrrolo[2,3-d]pyrimidin-4-yl)amino)quinoxalin-5-yl)dimethylphosphine oxide | $^1H$ NMR (400 MHz, Neutral, DMSO-$d_6$) δ 13.24 (s, 1H), 11.32 (s, 1H), 9.69-9.66 (m, 1H), 8.84 (d, *J* = 2 Hz, 1H), 8.79 (d, *J* = 2 Hz, 1H), 7.90 (s, 1H), 7.81 (d, *J* = 8.8 Hz, 1H), 7.72 (s, 1H), 7.65 (d, *J* = 7.2 Hz, 2H), 7.36 (tr, *J* = 7.6 Hz, 2H), 7.24 (tr, *J* = 7.2 Hz, 1H), 6.97-6.95 (m, 1H), 6.79 (s, 1H), 6.47-6.46 (m, 1H), 4.13-4.09 (m, 2H), 3.90 (s, 3H), 3.17 (s, 3H), 3.15 (s, 3H), 2.81 (s, 4H), 2.27 (s, 4H), 2.15 (s, 3H), 2.08 (s, 3H), 2.05 (s, 3H); MS(ESI): m/z 634 [M+H]$^+$ | 78 | 8.78, 99, (b) |

| | | | | | |
|---|---|---|---|---|---|
| 73 | 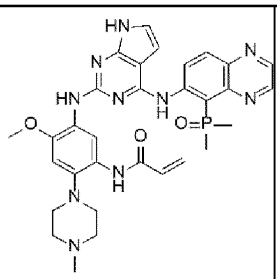 | N-(5-((4-((5-(dimethylphosphoryl)quinoxalin-6-yl)amino)-7H-pyrrolo[2,3-d]pyrimidin-2-yl)amino)-4-methoxy-2-(4-methylpiperazin-1-yl)phenyl)acrylamide | $^1$H NMR (400 MHz, TFA salt, DMSO-$d_6$) δ 13.24 (s, 1H), 11.35 (s, 1H), 9.70-9.67 (m, 1H), 9.09 (s, 1H), 8.85-8.79 (m, 2H), 8.42-8.39 (m, 1H), 8.08 (d, *J* = 9.6 Hz, 1H), 7.75 (s, 1H), 6.97 (s, 1H), 6.87 (s, 1H), 6.64-6.57 (m, 1H), 6.47 (s, 1H), 6.14-6.09 (m, 1H), 5.66 (d, *J* = 11.2 Hz, 1H), 4.13-4.06 (m, 1H), 3.84 (s, 3H), 3.16 (d, *J* = 5.2 Hz, 2H), 2.89-2.87 (m, 2H), 2.26 (s, 3H), 2.09 (s, 3H), 2.05 (s, 3H); MS(ESI): m/z 627 [M+H]$^+$ | 18 | 7.87, 100, (b) |

| | | | | | |
|---|---|---|---|---|---|
| 74 | 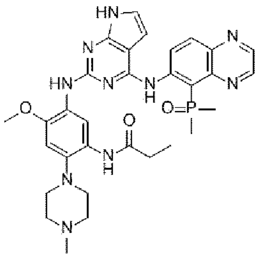 | N-(5-((4-((5-(dimethylphosphoryl)quinoxalin-6-yl)amino)-7H-pyrrolo[2,3-d]pyrimidin-2-yl)amino)-4-methoxy-2-(4-methylpiperazin-1-yl)phenyl)propionamide | MS(ESI): m/z 629 [M+H]$^+$ | | |
| 75 | 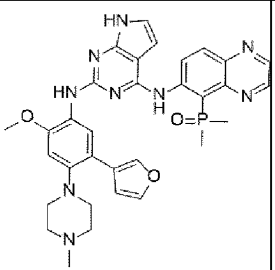 | (6-((2-((5-(furan-3-yl)-2-methoxy-4-(4-methylpiperazin-1-yl)phenyl)am | $^1$H NMR (400 MHz, FA salt, Methanol-$d_4$) δ 9.61 (dd, $J$ = 9.6, 4.3 Hz, 1H), 8.80 (d, $J$ = 1.9 Hz, 1H), 8.71 (d, $J$ = 1.9 Hz, 1H), 8.48 (s, 1H, FA), 8.32 (s, 1H), 7.92 (s, 1H), | 35 | 8.46, 98, (b) |

| | | | | | |
|---|---|---|---|---|---|
| | | ino)-7H-pyrrolo[2,3-d]pyrimidin-4-yl)amino)quinoxalin-5-yl)dimethylphosphine oxide | 7.74 (d, *J* = 9.6 Hz, 1H), 7.37 (t, *J* = 1.6 Hz, 1H), 6.98 (d, *J* = 3.6 Hz, 1H), 6.87 (s, 1H), 6.85 (d, *J* = 1.2 Hz, 1H), 6.60 (d, *J* = 3.6 Hz, 1H), 3.98 (s, 3H), 3.35-3.33 (m, 2H), 3.14-2.99 (m, 6H), 2.68 (s, 3H), 2.19 (s, 3H), 2.16 (s, 3H); MS(ESI): m/z 624 [M+H]+ | | |
| 76 | 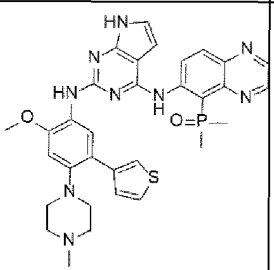 | (6-((2-((2-methoxy-4-(4-methylpiperazin-1-yl)-5-(thiophen-3-yl)phenyl)amino)-7H-pyrrolo[2,3-d]pyrimidin-4- | $^1$H NMR (400 MHz, TFA salt, Methanol-$d_4$) δ 9.57 (dd, *J* = 9.5, 4.3 Hz, 1H), 8.80 (d, *J* = 1.9 Hz, 1H), 8.72 (d, *J* = 1.9 Hz, 1H), 8.37 (s, 1H), 7.63 (d, *J* = 9.6 Hz, 1H), 7.54 (m, 2H), 7.37 (dd, *J* = 4.9, 3.0 Hz, 1H), 6.98 (d, *J* = 3.6 Hz, 1H), | 71 | 8.69, 97, (b) |

| | | | | | |
|---|---|---|---|---|---|
| | | yl)amino)quinoxalin-5-yl)dimethylphosphine oxide | 6.83 (s, 1H), 6.60 (d, *J* = 3.6 Hz, 1H), 4.00 (s, 3H), 3.31-3.15 (m, 8H), 2.94 (s, 3H), 2.19 (s, 3H), 2.15 (s, 3H); MS(ESI): m/z 640 [M+H]$^+$ | | |
| 77 | 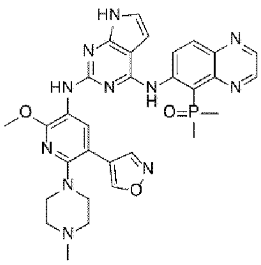 | (6-((2-((5-(isoxazol-4-yl)-2-methoxy-6-(4-methylpiperazin-1-yl)pyridin-3-yl)amino)-7H-pyrrolo[2,3-d]pyrimidin-4-yl)amino)quinoxalin-5-yl)dimethylphosphine | MS(ESI): m/z 626 [M+H]$^+$ | | |

| | | | | | |
|---|---|---|---|---|---|
| | | oxide | | | |
| 78 | 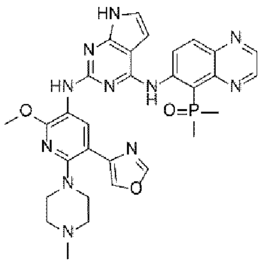 | (6-((2-((2-methoxy-6-(4-methylpiperazin-1-yl)-5-(oxazol-4-yl)pyridin-3-yl)amino)-7H-pyrrolo[2,3-d]pyrimidin-4-yl)amino)quinoxalin-5-yl)dimethylphosphine oxide | MS(ESI): m/z 626 $[M+H]^+$ | | |
| 79 | 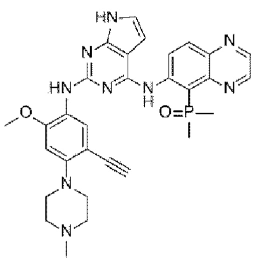 | (6-((2-((5-ethynyl-2-methoxy-4-(4-methylpiperazin-1- | $^1$H NMR (400 MHz, TFA salt, MeOD-$d_4$) δ 9.72-9.69 (m, 1H), 8.84 (d, $J$ = 2.0 Hz, 1H), 8.76 (d, $J$ = 2.0 Hz, 1H), 8.44 (s, 1H), 8.27 (d, | 8 | 8.24, 95, (b) |

| | | | | | |
|---|---|---|---|---|---|
| | | yl)phenyl)amino)-7H-pyrrolo[2,3-d]pyrimidin-4-yl)amino)quinoxalin-5-yl)dimethylphosphine oxide | *J* = 9.6 Hz, 1H), 7.01 (d, *J* = 3.6 Hz, 1H), 6.74 (s, 1H), 6.63 (d, *J* = 3.6 Hz, 1H), 4.01 (s, 3H), 3.63 (s, 1H), 3.51-3.41 (m, 8H), 2.89 (s, 3H), 2.19 (s, 3H), 2.18 (s, 3H); MS(ESI): m/z 582 $[M+H]^+$ | | |
| 80 | 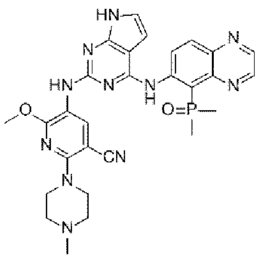 | 5-((4-((5-(dimethylphosphoryl)quinoxalin-6-yl)amino)-7H-pyrrolo[2,3-d]pyrimidin-2-yl)amino)-6-methoxy-2-(4-methylpiperazin-1-yl)nicotinon | MS(ESI): m/z 584 $[M+H]^+$ | | |

| | | | | | |
|---|---|---|---|---|---|
| | | itrile | | | |
| 81 | 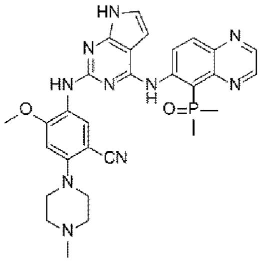 | 5-((4-((5-(dimethylphosphoryl)quinoxalin-6-yl)amino)-7H-pyrrolo[2,3-d]pyrimidin-2-yl)amino)-4-methoxy-2-(4-methylpiperazin-1-yl)benzonitrile | $^1$H NMR (400 MHz, TFA salt, Methanol-$d_4$) δ 9.66 (dd, *J* = 9.6, 4.2 Hz, 1H), 8.83 (d, *J* = 1.9 Hz, 1H), 8.81 (s, 1H), 8.75 (d, *J* = 1.8 Hz, 1H), 8.24 (d, *J* = 9.6 Hz, 1H), 7.02 (d, *J* = 3.5 Hz, 1H), 6.86 (s, 1H), 6.63 (d, *J* = 3.5 Hz, 1H), 4.07 (s, 4H), 3.43 (s, 8H), 2.95 (s, 3H), 2.21 (s, 3H), 2.17 (s, 3H); MS(ESI): m/z 583 [M+H]$^+$ | 60 | 8.52, 99, (b) |
| 82 | 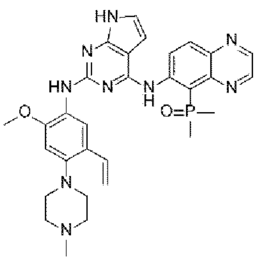 | (6-((2-((2-methoxy-4-(4-methylpiperazin-1-yl)-5-vinylphenyl)amino)-7H- | $^1$H NMR (400 MHz, TFA salt, Methanol-$d_4$) δ 9.43 (dd, *J* = 9.5, 3.7 Hz, 1H), 8.87 (d, *J* = 1.7 Hz, 1H), 8.81 (d, *J* = 1.7 Hz, 1H), 8.02 (s, 1H), 7.96 (d, *J* = | 6 | 8.31, 91, (b) |

| | | | | | |
|---|---|---|---|---|---|
| | | pyrrolo[2,3-d]pyrimidin-4-yl)amino)quinoxalin-5-yl)dimethylphosphine oxide | 9.7 Hz, 1H), 7.11 (dd, *J* = 17.8, 11.0 Hz, 1H), 7.06 (d, *J* = 3.6 Hz, 1H), 6.87 (s, 1H), 6.73 (d, *J* = 3.5 Hz, 1H), 5.56 (d, *J* = 17.4 Hz, 1H), 5.14 (d, *J* = 11.3 Hz, 1H), 3.93 (s, 3H), 3.66 (d, *J* = 12.4 Hz, 2H), 3.45 (t, *J* = 11.6 Hz, 4H), 3.19 (d, *J* = 12.0 Hz, 2H), 3.04 (s, 3H), 2.21 (s, 3H), 2.17 (s, 3H); MS(ESI): m/z 584 $[M+H]^+$ | | |
| 83 | 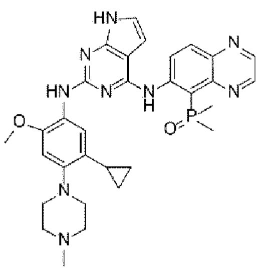 | (6-((2-((5-cyclopropyl-2-methoxy-4-(4-methylpiperazin-1-yl)phenyl)amino)-7H- | $^1$H NMR (400 MHz, TFA salt, Methanol-$d_4$) δ 9.41-9.32 (m, 1H), 8.88 (d, *J* = 1.9 Hz, 1H), 8.82 (d, *J* = 1.8 Hz, 1H), 8.00 (d, *J* = 9.4 Hz, 1H), 7.24 (s, 1H), 7.05 (d, *J* = 3.6 | 86 | 8.34, 99, (b) |

| | | | | | |
|---|---|---|---|---|---|
| | | pyrrolo[2,3-d]pyrimidin-4-yl)amino)quinoxalin-5-yl)dimethylphosphine oxide | Hz, 1H), 6.88 (s, 1H), 6.72 (d, *J* = 3.5 Hz, 1H), 3.89 (s, 3H), 3.65 (dd, *J* = 21.4, 12.9 Hz, 4H), 3.52-3.39 (m, 2H), 3.20 (t, *J* = 11.9 Hz, 2H), 3.04 (s, 3H), 2.31-2.22 (m, 1H), 2.21 (s, 3H), 2.18 (s, 3H), 0.96-0.85 (m, 2H), 0.65-0.58 (m, 2H); MS(ESI): m/z 597 $[M+H]^+$ | | |
| 84 | 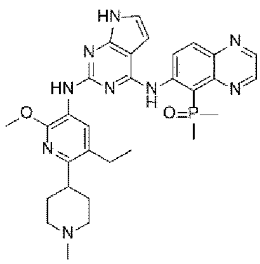 | (6-((2-((5-ethyl-2-methoxy-6-(1-methylpiperidin-4-yl)pyridin-3-yl)amino)-7H-pyrrolo[2,3- | MS(ESI): m/z 586 $[M+H]^+$ | | |

| | | | | | |
|---|---|---|---|---|---|
| | | d]pyrimidin-4-yl)amino)quinoxalin-5-yl)dimethylphosphine oxide | | | |
| 85 | 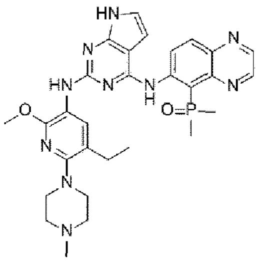 | (6-((2-((5-ethyl-2-methoxy-6-(4-methylpiperazin-1-yl)pyridin-3-yl)amino)-7H-pyrrolo[2,3-d]pyrimidin-4-yl)amino)quinoxalin-5-yl)dimethylphosphine oxide | $^1$H NMR (400 MHz, TFA salt, Methanol-$d_4$) δ 9.41-9.39 (m, 1H), 8.87 (d, *J* = 2.0 Hz, 1H), 8.81 (d, *J* = 1.6 Hz, 1H), 8.16 (d, *J* = 10.4 Hz, 1H), 8.06 (d, *J* = 9.6 Hz, 1H), 7.05 (d, *J* = 3.6 Hz, 1H), 6.70 (d, *J* = 3.2 Hz, 1H), 3.99 (s, 3H), 3.63-3.54 (m, 4H), 3.42-3.33 (m, 4H), 3.01 (s, 3H), 2.69-2.64 (m, 2H), 2.20 (s, 3H), 2.17 (s, 3H), 1.17 (t, *J* = 7.6 Hz, | 87 | 8.69, 98, (b) |

| | | | | | |
|---|---|---|---|---|---|
| | | | 3H); MS(ESI): m/z 587 [M+H]+ | | |
| 86 | 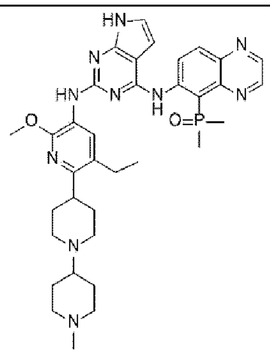 | (6-((2-((5-ethyl-2-methoxy-6-(1'-methyl-[1,4'-bipiperidin]-4-yl)pyridin-3-yl)amino)-7H-pyrrolo[2,3-d]pyrimidin-4-yl)amino)quinoxalin-5-yl)dimethylphosphine oxide | MS(ESI): m/z 669 [M+H]+ | | |

| | | | | | |
|---|---|---|---|---|---|
| 87 | 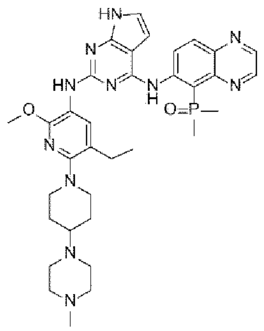 | (6-((2-((5-ethyl-2-methoxy-6-(4-(4-methylpiperazin-1-yl)piperidin-1-yl)pyridin-3-yl)amino)-7H-pyrrolo[2,3-d]pyrimidin-4-yl)amino)quinoxalin-5-yl)dimethylphosphine oxide | $^1$H NMR (400 MHz, TFA salt, Methanol-$d_4$) δ 9.42-9.40 (m, 1H), 8.90 (d, *J* = 2.0 Hz, 1H), 8.85 (d, *J* = 1.6 Hz, 1H), 8.09 (d, *J* = 9.6 Hz, 1H), 8.03 (s, 1H), 7.09 (d, *J* = 3.6 Hz, 1H), 6.76 (d, *J* = 3.6 Hz, 1H), 4.00 (s, 3H), 3.69-3.66 (m, 2H), 3.37-3.16 (m, 11H), 2.88 (s, 3H), 2.72-2.67 (m, 2H), 2.23 (s, 3H), 2.20 (s, 3H), 2.18-2.17 (m, 2H), 2.01-1.95 (m, 2), 1.22 (t, *J* = 7.6 Hz, 3H); MS(ESI): m/z 670 [M+H]$^+$ | 50 | 8.14, 99, (b) |
| 88 | 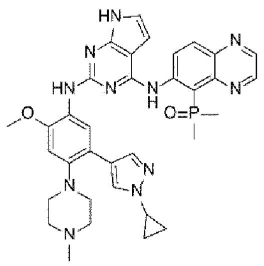 | (6-((2-((5-(1-cyclopropyl-1H-pyrazol- | $^1$H NMR (400 MHz, TFA salt, MeOD-$d_4$) δ 9.35-9.33 (m, 1H), 8.89 (d, *J* = 1.6 Hz, 1H), 8.84 | 58 | 8.19, 100, (b) |

| | | | | | |
|---|---|---|---|---|---|
| | | 4-yl)-2-methoxy-4-(4-methylpiperazin-1-yl)phenyl)amino)-7H-pyrrolo[2,3-d]pyrimidin-4-yl)amino)quinoxalin-5-yl)dimethylphosphine oxide | (d, $J$ = 1.6 Hz, 1H), 8.05 (s, 1H), 7.92 (s, 1H), 7.86 (s, 1H), 7.63-7.61 (m, 1H), 7.10 (d, $J$ = 3.6 Hz, 1H), 3.97 (s, 3H), 3.65-3.62 (m, 3H), 3.60-3.58 (m, 2H), 3.47-3.44 (m, 2H), 3.16-3.13 (m, 2H), 3.10 (s, 2H), 2.23 (s, 3H), 2.19 (s, 3H), 0.99-0.97 (m, 4H); MS(ESI): m/z 664 $[M+H]^+$ | | |
| 89 | 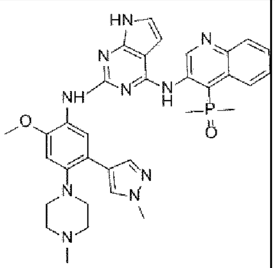 | (3-((2-((2-methoxy-5-(1-methyl-1H-pyrazol-4-yl)-4-(4-methylpiperazin-1-yl)phenyl)amino)-7H- | $^1$H NMR (400 MHz, FA salt, Methanol-$d_4$) δ 10.23 (d, $J$ = 4.6 Hz, 1H), 8.48 (s, 1H), 8.43 (s, 1H), 8.12-8.08 (m, 1H), 7.94-7.90 (m, 1H), 7.80 (s, 1H), 7.72-7.66 (m, 3H), 6.99 (d, $J$ = 3.5 | 4 | 7.49, 99, (b) |

| | | | | | |
|---|---|---|---|---|---|
| | | pyrrolo[2,3-d]pyrimidin-4-yl)amino)quinolin-4-yl)dimethylphosphine oxide | Hz, 1H), 6.83 (s, 1H), 6.53 (d, $J$ = 3.5 Hz, 1H), 3.96 (s, 3H), 3.65 (s, 3H), 3.36-3.33 (m, 2H), 3.06-2.97 (m, 4H), 2.64 (s, 3H), 2.25 (s, 3H), 2.21 (s, 3H); MS(ESI): m/z 637 $[M+H]^+$ | | |
| 90 | 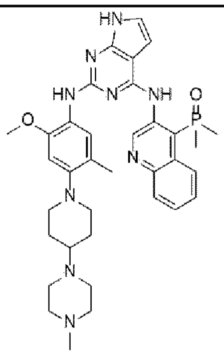 | (3-((2-((2-methoxy-5-methyl-4-(4-(4-methylpiperazin-1-yl)piperidin-1-yl)phenyl)amino)-7H-pyrrolo[2,3-d]pyrimidin-4-yl)amino)qui | $^1$H NMR (400 MHz, TFA salt, Methanol-$d_4$) δ 9.99 (d, $J$ = 4.1 Hz, 1H), 8.30 (d, $J$ = 8.5 Hz, 1H), 8.10 (d, $J$ = 7.8 Hz, 1H), 7.85-7.69 (m, 3H), 7.01 (d, $J$ = 3.5 Hz, 1H), 6.78 (s, 1H), 6.60 (d, $J$ = 3.4 Hz, 1H), 3.89 (s, 3H), 3.24 (d, $J$ = 9.9 Hz, 4H), 3.11-2.68 (m, 12H), 2.20 (s, 3H), 2.16 (s, 3H), | 81 | 7.19, 99, (b) |

| | | | | | |
|---|---|---|---|---|---|
| | | nolin-4-yl)dimethylphosphine oxide | 2.07 (d, $J$ = 11.1 Hz, 2H), 1.99 (s, 3H), 1.81 (dd, $J$ = 22.7, 11.5 Hz, 2H); MS(ESI): m/z 654 $[M+H]^+$ | | |
| 91 | 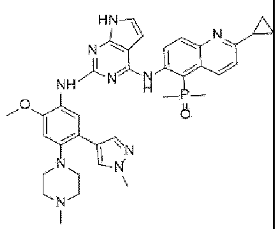 | (2-cyclopropyl-6-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)quinolin-5-yl)dimethylphosphine | $^1$H NMR (400 MHz, TFA salt, Methanol-$d_4$) δ 8.94 (dd, $J$ = 9.1, 4.0 Hz, 1H), 8.69 (d, $J$ = 9.0 Hz, 1H), 7.91 (d, $J$ = 13.4 Hz, 2H), 7.75-7.69 (m, 2H), 7.42 (d, $J$ = 9.2 Hz, 1H), 7.05 (d, $J$ = 3.6 Hz, 1H), 6.90 (s, 1H), 6.67 (d, $J$ = 3.5 Hz, 1H), 3.95 (s, 3H), 3.82 (s, 3H), 3.61-3.55 (m, 2H), 3.41-3.34 (m, 4H), 3.09-3.04 (m, 2H), 3.02 (s, 3H), 2.41-2.34 (m, 1H), 2.18 (s, 3H), | 30 | 7.59, 99, (b) |

| | | | | | |
|---|---|---|---|---|---|
| | | oxide | 2.14 (s, 3H), 1.35-1.20 (m, 4H); MS(ESI): m/z 677 $[M+H]^+$ | | |
| 92 | 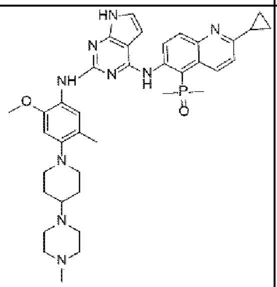 | (2-cyclopropyl-6-((2-((2-methoxy-5-methyl-4-(4-(4-methylpiperazin-1-yl)piperidin-1-yl)phenyl)amino)-7H-pyrrolo[2,3-d]pyrimidin-4-yl)amino)quinolin-5-yl)dimethylphosphine oxide | $^1H$ NMR (400 MHz, FA salt, Methanol-$d_4$) δ 8.90 (m, 1H), 8.67 (d, *J* = 9.2 Hz, 1H), 8.38 (s, 1H), 8.03 (d, *J* = 9.6 Hz, 1H), 7.87 (s, 1H), 7.33 (d, *J* = 9.2 Hz, 1H), 6.92 (d, *J* = 3.6 Hz, 1H), 6.70 (s, 1H), 6.51 (d, *J* = 3.6 Hz, 1H), 3.86 (s, 3H), 3.16-3.12 (m, 4H), 3.00 (s, 5H), 2.72 (t, *J* = 11.6 Hz, 4H), 2.63 (s, 3H), 2.30-2.25 (m, 1H), 2.10 (s, 3H), 2.06 (s, 3H), 2.02-1.95 (m, 8H), 1.80-1.71 (m, 2H), 1.16-1.13 (m, 4H); | 47 | 7.37, 98, (b) |

| | | | | | |
|---|---|---|---|---|---|
| | | | MS(ESI): m/z 695 [M+H]+ | | |
| 93 | 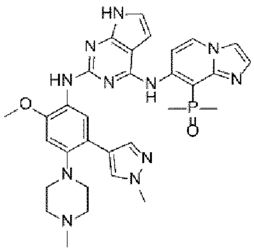 | (7-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)imidazo[1,2-a]pyridin-8-yl)dimethylphosphine oxide | $^{1}$H NMR (400 MHz, TFA salt, MeOD-$d_4$) δ 8.77 (s, 1H), 8.22 (d, *J* = 7.6 Hz, 1H), 8.02 (s, 1H), 8.01 (s, 1H), 7.93-7.90 (m, 2H), 7.77 (d, *J* = 1.6 Hz, 1H), 7.06 (d, *J* = 3.6 Hz, 1H), 6.92 (s, 1H), 6.63 (d, *J* = 3.6 Hz, 1H), 3.96 (s, 3H), 3.89 (s, 3H), 3.59-3.56 (m, 2H), 3.38-3.28 (m, 4H), 3.13-3.06 (m, 2H), 3.01 (s, 3H), 2.16 (s, 3H), 2.12 (s, 3H); MS(ESI): m/z 626 [M+H]+ | 53 | 7.16, 99, (b) |

| | | | | | |
|---|---|---|---|---|---|
| 94 | 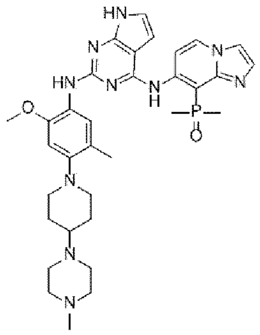 | (7-((2-((2-methoxy-5-methyl-4-(4-(4-methylpiperazin-1-yl)piperidin-1-yl)phenyl)amino)-7H-pyrrolo[2,3-d]pyrimidin-4-yl)amino)imidazo[1,2-a]pyridin-8-yl)dimethylphosphine oxide | $^1$H NMR (400 MHz, FA salt, Methanol-$d_4$) δ 8.92 (dd, $J$ = 7.62 Hz, 1H), 8.39 (dd, $J$ = 7.74 Hz, 1H), 8.31 (s, 2H), 8.07 (s, 1H), 7.70 (t, 1H), 7.45 (d, $J$ = 1.24 Hz, 1H), 6.94 (d, $J$ = 3.59 Hz 1H), 6.76 (s, 1H), 6.53 (d, $J$ = 3.57 Hz, 1H), 3.89 (s, 3H), 3.23 (d, $J$ = 11.9 Hz, 2H), 3.14-2.97 (m, 7H), 2.77 (t, 3H), 2.69 (s, 3H), 2.26 (s, 3H), 2.15 (s, 3H), 2.12 (s, 3H), 2.08 (d, $J$ = 11.13 Hz, 2H), 1.84-1.75 (m, 2H); MS(ESI): m/z 643 [M+H]$^+$ | 42 | 6.77, 99, (b) |

| | | | | | |
|---|---|---|---|---|---|
| 95 | 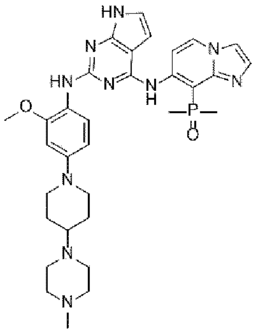 | (7-((2-((2-methoxy-4-(4-(4-methylpiperazin-1-yl)piperidin-1-yl)phenyl)amino)-7H-pyrrolo[2,3-d]pyrimidin-4-yl)amino)imidazo[1,2-a]pyridin-8-yl)dimethylphosphine oxide | MS(ESI): m/z 629 [M+H]+ | | |
| 96 | 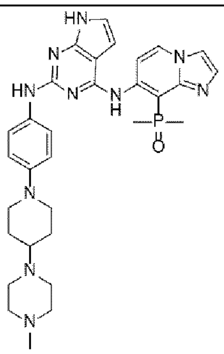 | dimethyl(7-((2-((4-(4-(4-methylpiperazin-1-yl)piperidin | MS(ESI): m/z 599 [M+H]+ | | |

| | | | | | |
|---|---|---|---|---|---|
| | | -1-yl)phenyl)amino)-7H-pyrrolo[2,3-d]pyrimidin-4-yl)amino)imidazo[1,2-a]pyridin-8-yl)phosphine oxide | | | |
| 97 | 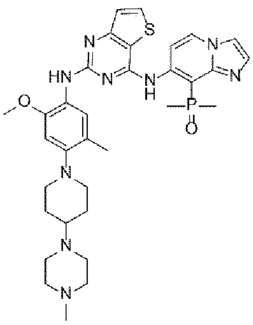 | (7-((2-((2-methoxy-5-methyl-4-(4-(4-methylpiperazin-1-yl)piperidin-1-yl)phenyl)amino)thieno[3,2-d]pyrimidin-4- | MS(ESI): m/z 660 [M+H]$^+$ | | |

| | | | | | |
|---|---|---|---|---|---|
| | | yl)amino)imidazo[1,2-a]pyridin-8-yl)dimethylphosphine oxide | | | |
| 98 | 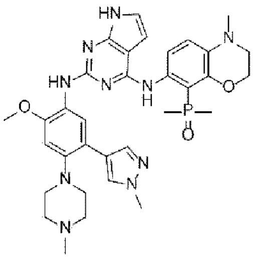 | (7-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)-4-methyl-3,4-dihydro-2H-benzo[b][1,4]oxazin-8-yl)dimethylp | MS(ESI): m/z 657 $[M+H]^+$ | | |

| | | | | | |
|---|---|---|---|---|---|
| | | hosphine oxide | | | |
| 99 | 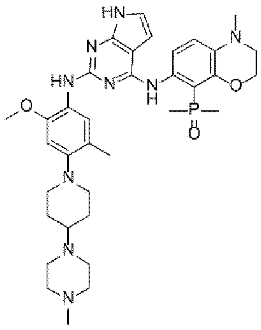 | (7-((2-((2-methoxy-5-methyl-4-(4-(4-methylpiperazin-1-yl)piperidin-1-yl)phenyl)amino)-7H-pyrrolo[2,3-d]pyrimidin-4-yl)amino)-4-methyl-3,4-dihydro-2H-benzo[b][1,4]oxazin-8-yl)dimethylphosphine oxide | MS(ESI): m/z 674 [M+H]+ | | |

| | | | | | |
|---|---|---|---|---|---|
| 100 | 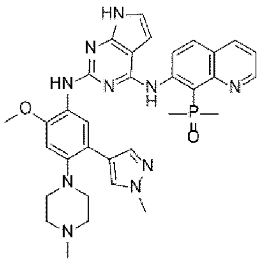 | (7-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)quinolin-8-yl)dimethylphosphine oxide | $^1$H NMR (400 MHz, TFA salt, DMSO-$d_6$) δ 13.27 (s, 1H), 11.35 (s, 1H), 9.46-9.43 (m, 1H), 8.84-8.82 (m, 1H), 8.23-8.17 (m, 3H), 8.05 (s, 1H), 7.93 (s, 1H), 7.70 (d, *J* = 8.8 Hz, 1H), 7.56 (s, 1H), 7.44-7.41 (m, 1H), 6.99-6.98 (m, 1H), 6.83 (s, 1H), 6.48-6.46 (m, 1H), 3.88 (s, 3H), 3.79 (s, 3H), 2.87-2.86 (m, 4H); MS(ESI): m/z 637 [M+H]$^+$ | 59 | 8.34, 100, (b) |
| 101 | 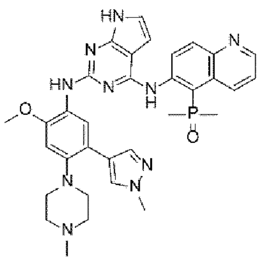 | (6-((2-((2-methoxy-5-(1-methyl-1H-pyrazol-4-yl)-4-(4-methylpiperazin-1- | $^1$H NMR (400 MHz, FA salt, Methanol-$d_4$) δ 9.30-9.27 (m, 1H), 8.79-8.77 (m, 1H), 8.58 (d, *J* = 8.8 Hz, 1H), 8.39 (s, 1H), 8.36 (s, 1H), 7.89 (s, | 36 | 7.75, 100, (b) |

| | | | | | |
|---|---|---|---|---|---|
| | | yl)phenyl)amino)-7H-pyrrolo[2,3-d]pyrimidin-4-yl)amino)quinolin-5-yl)dimethylphosphine oxide | 1H), 7.75 (s, 1H), 7.74-7.73 (m, 1H), 7.71-7.59 (m, 1H), 6.98 (d, *J* = 3.6 Hz, 1H), 6.87 (s, 1H), 6.57 (d, *J* = 3.6 Hz, 1H), 3.99 (s, 3H), 3.76 (s, 3H), 3.32-3.19 (m, 8H), 2.86 (s, 3H), 2.22 (s, 3H), 2.18 (s, 3H); m/z 667 $[M+H]^+$ | | |
| 102 | 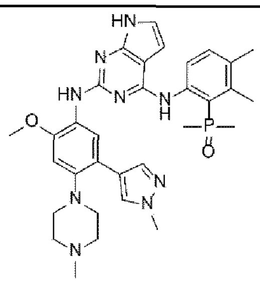 | (6-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)- | $^1H$ NMR (400 MHz, FA salt, Methanol-$d_4$) δ 8.45 (s, 1H), 8.34-8.31 (m, 1H), 7.92 (s, 1H), 7.67 (s, 1H), 6.90-6.84 (m, 3H), 6.41 (d, *J* = 3.6 Hz, 1H), 3.98 (s, 3H), 3.92 (s, 3H), 3.35-3.28 (m, 4H), 3.16-3.13 (m, 4H), 2.89 (s, 3H), 2.44 (s, 3H), | 31 | 7.69, 100, (b) |

| | | | | | |
|---|---|---|---|---|---|
| | | 2,3-dimethylphenyl)dimethylphosphine oxide | 2.23 (s, 3H), 2.03 (s, 3H), 2.01 (s, 3H); MS(ESI): m/z 614 [M+H]+ | | |
| 103 | 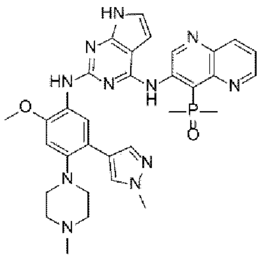 | (3-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)-1,5-naphthyridin-4-yl)dimethylphosphine oxide | $^1$H NMR (400 MHz, TFA salt, MeOD-$d_4$) δ 10.62 (d, $J$ = 4.4 Hz, 1H), 8.90-8.88 (m, 1H), 8.46 (s, 1H), 8.22-8.20 (m, 1H), 7.99 (s, 1H), 7.97 (s, 1H), 7.62-7.59 (m, 1H), 7.00 (d, $J$ = 3.6 Hz, 1H), 6.83 (s, 1H), 6.56 (d, $J$ = 3.6 Hz, 1H), 3.97 (s, 3H), 3.72 (s, 3H), 3.49-3.47 (m, 2H), 3.15-3.08 (m, 2H), 2.95 (s, 3H), 2.25 (s, 3H), 2.22 (s, 3H); MS(ESI): m/z 638 [M+H]+ | 14 | 8.15, 97, (b) |

| | | | | | |
|---|---|---|---|---|---|
| 104 | 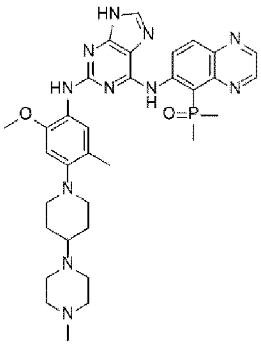 | (6-((2-((2-methoxy-5-methyl-4-(4-(4-methylpiperazin-1-yl)piperidin-1-yl)phenyl)amino)-9H-purin-6-yl)amino)quinoxalin-5-yl)dimethylphosphine oxide | $^1$H NMR (400 MHz, TFA salt, Methanol-$d_4$) δ 9.47-9.43 (m, 1H), 8.83 (d, *J* = 2.0 Hz, 1H), 8.76 (d, *J* = 1.6 Hz, 1H), 8.07 (d, *J* = 9.6 Hz, 1H), 7.94 (s, 1H), 7.89 (s, 1H), 6.76 (s, 1H), 3.87 (s, 3H), 3.21 (d, *J* = 11.6 Hz, 2H), 2.87-2.62 (m, 9H), 2.52 (s, 3H), 2.24-2.14 (m, 10H), 2.06-2.03 (m, 3H), 1.80-1.59 (m, 2H); MS(ESI): m/z 656 [M+H]$^+$ | 5 | 3.85, 100, (a) |
| 105 | 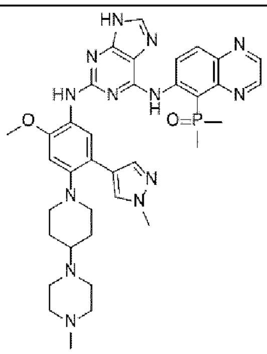 | (6-((2-((2-methoxy-5-(1-methyl-1H-pyrazol-4-yl)-4-(4-(4-methylpipera | $^1$H NMR (400 MHz, TFA salt, Methanol-$d_4$) δ 9.37-9.34 (m, 1H), 8.81 (d, *J* = 1.6 Hz, 1H), 8.75 (d, *J* = 2.0 Hz, 1H), 8.25 (s, 1H), 7.99 (s, 1H), | 4 | 3.88, 100, (a) |

| | | | | | |
|---|---|---|---|---|---|
| | | zin-1-yl)piperidin-1-yl)phenyl)amino)-9H-purin-6-yl)amino)quinoxalin-5-yl)dimethylphosphine oxide | 7.91 (s, 1H), 7.69 (s, 1H), 7.63 (d, *J* = 9.2 Hz, 1H), 6.85 (s, 1H), 3.93 (s, 3H), 3.85 (s, 3H), 3.34-3.33 (m, 2H), 2.69-2.61 (m, 5H), 2.38 (s, 3H), 2.35-2.02 (m, 12H), 1.67-1.59 (m, 4H); MS(ESI): m/z 722 $[M+H]^+$ | | |
| 106 | 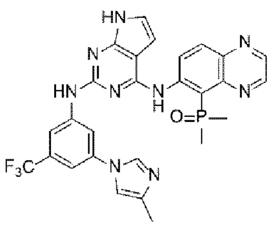 | dimethyl(6-((2-((3-(4-methyl-1H-imidazol-1-yl)-5-(trifluoromethyl)phenyl)amino)-7H-pyrrolo[2,3-d]pyrimidin-4-yl)amino)quinoxalin-5- | $^1H$ NMR (400 MHz, TFA salt, Methanol-$d_4$) δ 9.68-9.65 (m, 1H), 9.38 (d, *J* = 1.6 Hz, 1H), 8.80 (d, *J* = 2.0 Hz, 1H), 8.73 (d, *J* = 2.0 Hz, 1H), 8.68 (t, *J* = 2.0 Hz, 1H), 8.10 (s, 1H), 7.95 (d, *J* = 9.6 Hz, 1H), 7.81 (t, *J* = 1.2 Hz, 1H), 7.54 (s, 1H), 7.02 (d, *J* = 3.6 Hz, 1H), 6.59 (d, | 41 | 6.04, 100, (b) |

| | | | | | |
|---|---|---|---|---|---|
| | | yl)phosphine oxide | $J$ = 3.6 Hz, 1H), 2.39 (d, J = 1.6 Hz, 3H), 2.19 (s, 3H), 2.16 (s, 3H); MS(ESI): m/z 578 $[M+H]^+$ | | |
| 107 | 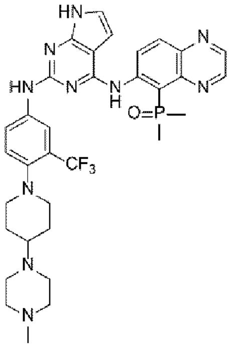 | dimethyl(6-((2-((4-(4-(4-methylpiperazin-1-yl)piperidin-1-yl)-3-(trifluoromethyl)phenyl)amino)-7H-pyrrolo[2,3-d]pyrimidin-4-yl)amino)quinoxalin-5-yl)phosphine oxide | $^1$H NMR (400 MHz, FA salt, Methanol-$d_4$) δ 9.93-9.89 (m, 1H), 8.81 (d, $J$ = 2.0 Hz, 1H), 8.73 (d, $J$ = 2.0 Hz, 1H), 8.32 (s, 1H), 8.13 (s, 1H), 8.11 (d, $J$ = 2.8 Hz, 1H), 7.93-7.90 (m, 1H), 7.43 (d, $J$ = 8.4 Hz, 1H), 6.99 (d, $J$ = 3.6 Hz, 1H), 6.61 (d, $J$ = 3.6 Hz, 1H), 3.13-3.09 (m, 4H), 3.03 (s, 5H), 2.89-2.78 (m, 4H), 2.66 (s, 3H), 2.20 (s, 3H), 2.16 (s, 3H), 2.04-2.02 (m, 2H), 1.81-1.72 (m, 2H); | 54 | 8.45, 99, (b) |

| | | | | | |
|---|---|---|---|---|---|
| | | | MS(ESI): m/z 679 [M+H]+ | | |
| 108 | 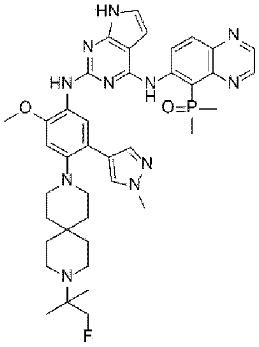 | (6-((2-((4-(9-(1-fluoro-2-methylpropan-2-yl)-3,9-diazaspiro[5.5]undecan-3-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1$H NMR (400 MHz, TFA salt, Methanol-$d_4$) δ 9.61 (dd, *J* = 9.5, 4.3 Hz, 1H), 8.80 (d, *J* = 1.9 Hz, 1H), 8.72 (d, *J* = 2.0 Hz, 1H), 8.27 (s, 1H), 7.98 (s, 1H), 7.83 (s, 1H), 7.57 (d, *J* = 9.7 Hz, 1H), 6.98 (d, *J* = 3.6 Hz, 1H), 6.91 (s, 1H), 6.60 (d, *J* = 3.6 Hz, 1H), 3.96 (s, 3H), 3.79 (s, 3H), 3.35-3.31 (m, 6H), 2.96-2.89 (m, 4H), 2.19 (s, 3H), 2.16 (s, 3H), 1.93-1.65 (m, 8H), 1.53 (s, 3H), 1.47 (s, 3H); MS(ESI): m/z 766 [M+H]+ | 36 | 8.76, 96, (b) |

| | | | | | |
|---|---|---|---|---|---|
| 109 | 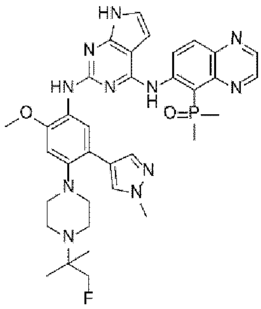 | (6-((2-((4-(4-(1-fluoro-2-methylpropan-2-yl)piperazin-1-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1$H NMR (400 MHz, FA salt, Methanol-$d_4$) δ 9.61 (dd, $J$ = 9.54 Hz, 1H), 8.77 (d, $J$ = 1.89 Hz, 1H), 8.70 (d, $J$ = 1.92 Hz, 1H), 8.30 (s, 1H), 7.99 (s, 1H), 7.79 (s, 1H), 7.57 (d, $J$ = 9.54 Hz 1H), 6.96 (d, $J$ = 3.58 Hz, 1H), 6.87 (s, 1H), 6.58 (d, $J$ = 3.56 Hz, 1H), 3.96 (s, 3H), 3.79 (s, 3H), 3.01 (t, 4H), 2.88-2.83 (m, 4H), 2.76 (d, $J$ = 22.29 Hz, 2H), 2.18 (s, 3H), 2.15 (s, 3H), 1.44 (s, 3H), 1.39 (s, 3H); MS(ESI): m/z 698 [M+H]$^+$ | 54 | 8.49, 99, (b) |

| | | | | | |
|---|---|---|---|---|---|
| 110 | 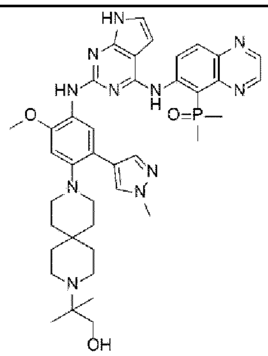 | (6-((2-((4-(9-(1-hydroxy-2-methylpropan-2-yl)-3,9-diazaspiro[5.5]undecan-3-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1$H NMR (400 MHz, TFA salt, Methanol-$d_4$) δ 9.59 (dd, $J$ = 9.5, 4.2 Hz, 1H), 8.81 (d, $J$ = 1.9 Hz, 1H), 8.73 (d, $J$ = 1.9 Hz, 1H), 8.23 (s, 1H), 7.98 (s, 1H), 7.83 (s, 1H), 7.58 (d, $J$ = 9.6 Hz, 1H), 6.99 (d, $J$ = 3.6 Hz, 1H), 6.92 (s, 1H), 6.62 (d, $J$ = 3.6 Hz, 1H), 3.96 (s, 3H), 3.79 (s, 3H), 3.67 (s, 2H), 3.46 (dd, $J$ = 6.8, 4.3 Hz, 1H), 3.33 (d, $J$ = 1.7 Hz, 2H), 3.25-3.15 (m, 3H), 2.98-2.91 (m, 4H), 2.20 (s, 3H), 2.16 (s, 3H), 2.15-2.08 (m, 2H), 1.86-1.80 (m, 2H), 1.79-1.68 (m, 2H), 1.68-1.60 (m, 2H), 1.39 (s, | 57 | 8.39, 97, (b) |

| | | | | | |
|---|---|---|---|---|---|
| | | | 6H); MS(ESI): m/z 764 [M+H]+ | | |
| 111 | 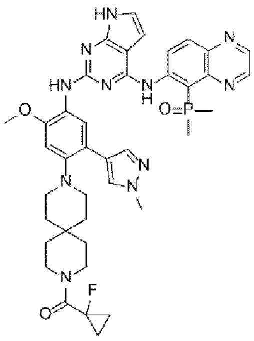 | (9-(4-((4-((5-(dimethylphosphoryl)quinoxalin-6-yl)amino)-7H-pyrrolo[2,3-d]pyrimidin-2-yl)amino)-5-methoxy-2-(1-methyl-1H-pyrazol-4-yl)phenyl)-3,9-diazaspiro[5.5]undecan-3-yl)(1-fluorocyclopropyl)methanone | $^{1}$H NMR (400 MHz, free base, Methanol-$d_4$) δ 9.57-9.54 (m, 1H), 8.73 (d, *J* = 2.0 Hz, 1H), 8.67 (d, *J* = 2.0 Hz, 1H), 8.26 (s, 1H), 7.96 (s, 1H), 7.78 (s, 1H), 7.52 (d, *J* = 9.6 Hz, 1H), 6.95 (d, *J* = 3.6 Hz, 1H), 6.85 (s, 1H), 6.55 (d, *J* = 4.0 Hz, 1H), 3.93 (s, 3H), 3.78 (s, 3H), 3.62-3.60 (m, 4H), 2.89-2.86 (m, 4H), 2.17-2.13 (m, 8H), 1.69-1.61 (m, 8H), 1.30-1.18 (m, 3H); MS(ESI): m/z 778 [M+H]+ | 31 | 11.08, 100, (b) |

| | | | | | |
|---|---|---|---|---|---|
| 112 | 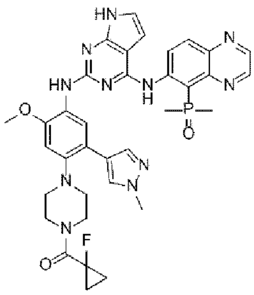 | (4-(4-((4-((5-(dimethylphosphoryl)quinoxalin-6-yl)amino)-7H-pyrrolo[2,3-d]pyrimidin-2-yl)amino)-5-methoxy-2-(1-methyl-1H-pyrazol-4-yl)phenyl)piperazin-1-yl)(1-fluorocyclopropyl)methanone | $^1$H NMR (400 MHz, TFA salt, Methanol-$d_4$) δ 9.35-9.34 (m, 1H), 8.85 (d, $J$ = 2.0 Hz, 1H), 8.82 (d, $J$ = 2.0 Hz, 1H), 8.12 (s, 1H), 7.84 (s, 1H), 7.79 (s, 1H), 7.62-7.66 (m, 1H), 7.06 (d, $J$ = 3.6 Hz, 1H), 6.93 (s, 1H), 6.74 (d, $J$ = 3.2 Hz, 1H), 3.93-3.86 (m, 10H), 3.04-3.01 (m, 4H), 2.20 (s, 3H), 2.16-2.15 (m, 4H), 1.29-1.25 (m, 4H); MS(ESI): m/z 710 [M+H]$^+$ | 10 | 10.55, 98, (b) |

| | | | | | |
|---|---|---|---|---|---|
| 113 | 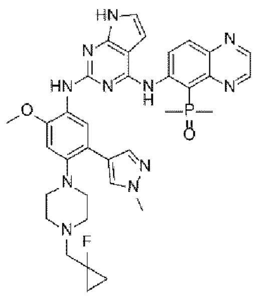 | (6-((2-((4-(4-((1-fluorocyclopropyl)methyl)piperazin-1-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1$H NMR (400 MHz, TFA salt, Methanol-$d_4$) δ 9.55-9.51 (m, 1H), 8.79 (d, *J* = 2.0 Hz, 1H), 8.72 (d, *J* = 2.0 Hz, 1H), 8.33 (s, 1H), 7.95 (s, 1H), 7.76 (s, 1H), 7.56 (d, *J* = 9.6 Hz, 1H), 6.60 (d, *J* = 3.6 Hz, 1H), 3.98 (s, 3H), 3.80-3.75 (m, 7H), 2.18-2.14 (m, 10H), 1.38-1.25 (m, 4H), 1.05-0.99 (m, 2H); MS(ESI): m/z 696 [M+H]$^+$ | 13 | 8.36, 100, (b) |

| | | | | | |
|---|---|---|---|---|---|
| 114 | 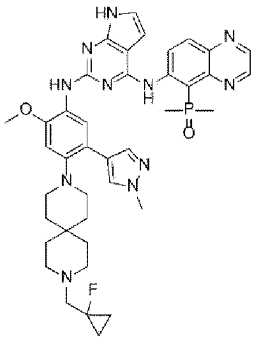 | (6-((2-((4-(9-((1-fluorocyclopropyl)methyl)-3,9-diazaspiro[5.5]undecan-3-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | $^1$H NMR (400 MHz, TFA, Methanol-$d_4$) δ 9.60-9.57 (m, 1H), 8.77 (d, $J$ = 2.0 Hz, 1H), 8.70 (d, $J$ = 2.0 Hz, 1H), 8.26 (s, 1H), 7.96 (s, 1H), 7.81 (s, 1H), 7.56 (d, $J$ = 9.6 Hz, 1H), 6.97 (d, $J$ = 3.6 Hz, 1H), 6.89 (s, 1H), 6.58 (d, $J$ = 3.6 Hz, 1H), 3.95 (s, 3H), 3.78 (s, 3H), 3.46 (d, $J$ = 21.2 Hz, 2H), 3.30-3.24 (m, 3H), 2.93-2.90 (m, 4H), 2.18-2.14 (m, 8H), 1.86 (s, 4H), 1.72 (s, 4H), 1.32-1.20 (m, 2H), 0.91-0.87 (m, 2H); MS(ESI): m/z 764 [M+H]$^+$ | 18 | 8.74, 100, (b) |

| | | | | | |
|---|---|---|---|---|---|
| 115 | 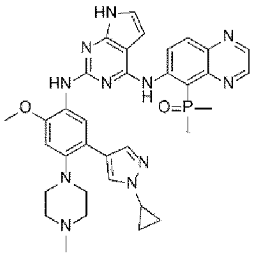 | (6-((2-((5-(1-cyclopropyl-1H-pyrazol-4-yl)-2-methoxy-4-(4-methylpiperazin-1-yl)phenyl)amino)-7H-pyrrolo[2,3-d]pyrimidin-4-yl)amino)quinoxalin-5-yl)dimethylphosphine oxide | $^1$H NMR (400 MHz, TFA salt, MeOD-$d_4$) δ 9.35-9.33 (m, 1H), 8.89 (d, $J$ = 1.6 Hz, 1H), 8.84 (d, $J$ = 1.6 Hz, 1H), 8.05 (s, 1H), 7.92 (s, 1H), 7.86 (s, 1H), 7.63-7.61 (m, 1H), 7.10 (d, $J$ = 3.6 Hz, 1H), 3.97 (s, 3H), 3.65-3.62 (m, 3H), 3.60-3.58 (m, 2H), 3.47-3.44 (m, 2H), 3.16-3.13 (m, 2H), 3.10 (s, 2H), 2.23 (s, 3H), 2.19 (s, 3H), 0.99-0.97 (m, 4H); MS(ESI): m/z 664 [M+H]$^+$ | 58 | 8.19, 100, (b) |
| 116 | 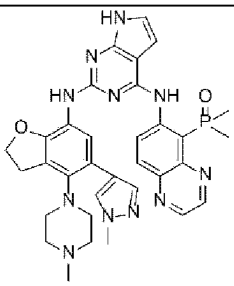 | dimethyl(6-((2-((5-(1-methyl-1H-pyrazol-4-yl)-4-(4- | $^1$H NMR (400 MHz, TFA salt, MeOD-$d_4$) δ 9.42-9.30 (m, 1H), 8.89 (d, $J$ = 2 Hz, 1H), 8.84 (d, $J$ = 2 Hz, 1H), | 58 | 100* |

| | | | | | |
|---|---|---|---|---|---|
| | | methylpiperazin-1-yl)-2,3-dihydrobenzofuran-7-yl)amino)-7H-pyrrolo[2,3-d]pyrimidin-4-yl)amino)quinoxalin-5-yl)phosphine oxide | 7.91 (s, 1H), 7.80-7.70 (m, 3H), 7.09 (d, $J$ =3.6 Hz, 1H), 6.76 (d, $J$ = 3.6 Hz, 1H), 4.69 (t, $J$ = 8.8 Hz, 2H), 3.62-3.14 (m, 12H), 3.01 (s, 3H), 2.30-2.10 (m, 7H); m/z 650 $[M+H]^+$ | | |
| 117 | 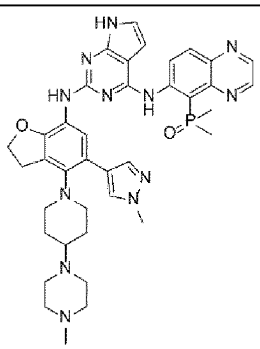 | dimethyl(6-((2-((5-(1-methyl-1H-pyrazol-4-yl)-4-(4-(4-methylpiperazin-1-yl)piperidin-1-yl)-2,3-dihydrobenzo | $^1H$ NMR (400 MHz, FA salt, MeOD-$d_4$) δ 9.66-9.63 (m, 1H), 8.78 (d, $J$ = 2 Hz, 1H), 8.71 (d, $J$ = 1.6 Hz, 1H), 7.97 (s, 1H), 7.83 (s, 1H), 7.73 (s, 1H), 7.64 (d, $J$ = 9.6 Hz, 1H), 6.96 (d, $J$ = 3.6 Hz, 1H), 6.59 (d, $J$ = | 28 | 7.50, 98, (b) |

| | | | | | |
|---|---|---|---|---|---|
| | | furan-7-yl)amino)-7H-pyrrolo[2,3-d]pyrimidin-4-yl)amino)quinoxalin-5-yl)phosphine oxide | 3.6 Hz, 1H), 4.61 (tr, *J* = 8.4 Hz, 2H), 3.81 (s, 3H), 3.45 (tr, *J* = 8.4 Hz, 2H), 3.17-3.14 (m, 2H), 3.06-3.00 (m, 9H), 2.65 (s, 4H), 2.18 (s, 3H), 2.15 (s, 3H), 1.95 (d, *J* = 10.8 Hz, 2H), 1.71-1.62 (m, 2H); MS(ESI): m/z 733 $[M+H]^+$ | | |
| 118 | 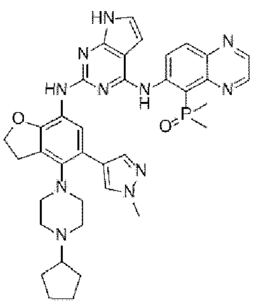 | (6-((2-((4-(4-cyclopentylpiperazin-1-yl)-5-(1-methyl-1H-pyrazol-4-yl)-2,3-dihydrobenzofuran-7-yl)amino)-7H- | MS(ESI): m/z 704 $[M+H]^+$ | | |

| | | | | | |
|---|---|---|---|---|---|
| | | pyrrolo[2,3-d]pyrimidin-4-yl)amino)quinoxalin-5-yl)dimethylphosphine oxide | | | |
| 119 | 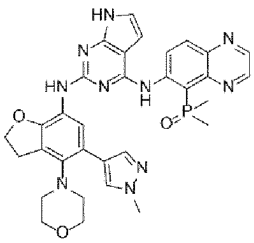 | dimethyl(6-((2-((5-(1-methyl-1H-pyrazol-4-yl)-4-morpholino-2,3-dihydrobenzofuran-7-yl)amino)-7H-pyrrolo[2,3-d]pyrimidin-4-yl)amino)quinoxalin-5- | MS(ESI): m/z 637 [M+H]$^+$ | | |

| | | | | | |
|---|---|---|---|---|---|
| | | yl)phosphine oxide | | | |
| 120 | 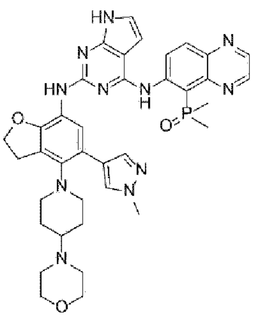 | dimethyl(6-((2-((5-(1-methyl-1H-pyrazol-4-yl)-4-(4-morpholinopiperidin-1-yl)-2,3-dihydrobenzofuran-7-yl)amino)-7H-pyrrolo[2,3-d]pyrimidin-4-yl)amino)quinoxalin-5-yl)phosphine oxide | $^1$H NMR (400 MHz, FA salt, MeOD-$d_4$) δ 9.62-9.59 (m, 1H), 8.75 (d, *J* = 2.0 Hz, 1H), 8.69 (d, *J* = 2.0 Hz, 1H), 8.26 (s, 1H), 8.02 (s, 1H), 7.78 (s, 1H), 7.66 (s, 1H), 7.60 (d, *J* = 9.2 Hz, 1H), 6.97 (d, *J* = 3.6 Hz, 1H), 6.57 (d, *J* = 3.6 Hz, 1H), 4.58 (t, *J* = 8.4, 8.4 Hz, 2H), 3.90-3.88 (m, 4H), 3.82 (s, 3H), 3.39-3.34 (m, 2H), 3.33-3.11 (m, 6H), 2.99-2.96 (m, 3H), 2.17 (s, 3H), 2.14 (s, 3H), 2.08-2.05 (m, 2H), 1.71-1.68 (m, 2H); MS(ESI): m/z 720 | 42 | 8.02, 96, (b) |

| | | | | | |
|---|---|---|---|---|---|
| | | | [M+H]+ | | |
| 121 | 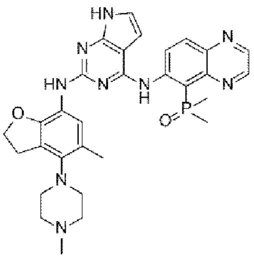 | dimethyl(6-((2-((5-methyl-4-(4-methylpiperazin-1-yl)-2,3-dihydrobenzofuran-7-yl)amino)-7H-pyrrolo[2,3-d]pyrimidin-4-yl)amino)quinoxalin-5-yl)phosphine oxide | $^1$H NMR (400 MHz, FA salt, MeOD-$d_4$) δ 9.75-9.71 (m, 1H), 8.80 (d, *J* = 2 Hz, 1H), 8.71 (d, *J* = 1.6 Hz, 1H), 8.02 (d, *J* = 9.2 Hz, 1H), 7.75 (s, 1H), 6.69 (d, *J* = 3.6 Hz, 1H), 6.58 (d, *J* = 3.6 Hz, 1H), 4.60 (tr, *J* = 8.4 Hz, 2H), 3.43-3.37 (m, 5H), 2.94 (s, 3H), 2.29 (s, 3H), 2.19 (s, 3H), 2.16 (s, 3H); MS(ESI): m/z 584 [M+H]+ | 57 | 7.95, 98, (b) |
| 122 | 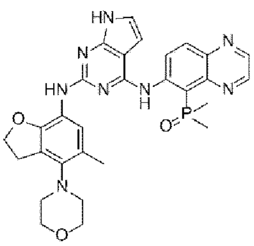 | dimethyl(6-((2-((5-methyl-4-morpholino-2,3-dihydrobenzo | $^1$H NMR (400 MHz, TFA salt, DMSO-$d_4$) δ 13.24 (s, 1H), 11.31 (S, 1H), 9.80-9.76 (m, 1H), 8.86 (d, *J* = 2.0 Hz, 1H), 8.80 (d, *J* = | 20 | 10.63, 96, (b) |

| | | | | | |
|---|---|---|---|---|---|
| | | furan-7-yl)amino)-7H-pyrrolo[2,3-d]pyrimidin-4-yl)amino)quinoxalin-5-yl)phosphine oxide | 1.6 Hz, 1H), 8.02 (d, *J* = 9.6 Hz, 1H), 7.78 (s, 1H), 7.51 (s, 1H), 6.97 (t, *J* = 2.4 Hz, 1H), 6.47 (t, *J* = 1.6 Hz, 1H), 4.52 (t, *J* = 8.4 Hz, 2H), 3.73 (s, 4H), 3.42 (t, *J* = 8.8 Hz, 2H), 2.98 (s, 4H), 2.25 (s, 3H), 2.10 (s, 3H), 2.06 (s, 3H); MS(ESI): m/z 571 $[M+H]^+$ | | |
| 123 | 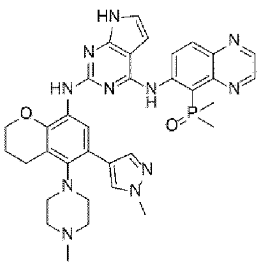 | dimethyl(6-((2-((6-(1-methyl-1H-pyrazol-4-yl)-5-(4-methylpiperazin-1-yl)chroman-8-yl)amino)-7H-pyrrolo[2,3- | MS(ESI): m/z 664 $[M+H]^+$ | | |

| | | | | | |
|---|---|---|---|---|---|
| | | d]pyrimidin-4-yl)amino)quinoxalin-5-yl)phosphine oxide | | | |
| 124 | 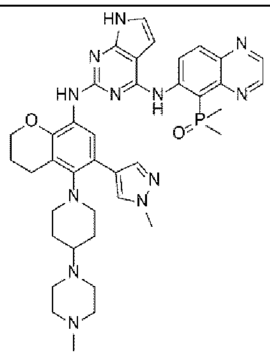 | dimethyl(6-((2-((6-(1-methyl-1H-pyrazol-4-yl)-5-(4-(4-methylpiperazin-1-yl)piperidin-1-yl)chroman-8-yl)amino)-7H-pyrrolo[2,3-d]pyrimidin-4-yl)amino)quinoxalin-5-yl)phosphine | MS(ESI): m/z 747 [M+H]$^+$ | | |

| | | | | | |
|---|---|---|---|---|---|
| | | oxide | | | |
| 125 | 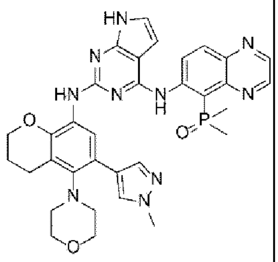 | dimethyl(6-((2-((6-(1-methyl-1H-pyrazol-4-yl)-5-morpholinochroman-8-yl)amino)-7H-pyrrolo[2,3-d]pyrimidin-4-yl)amino)quinoxalin-5-yl)phosphine oxide | MS(ESI): m/z 651 $[M+H]^+$ | | |
| 126 | 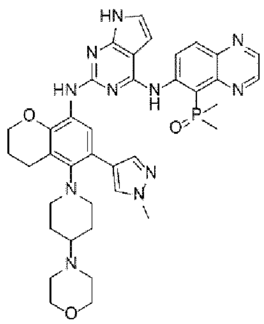 | dimethyl(6-((2-((6-(1-methyl-1H-pyrazol-4-yl)-5-(4-morpholinopiperidin-1- | MS(ESI): m/z 734 $[M+H]^+$ | | |

| | | | | | |
|---|---|---|---|---|---|
| | | yl)chroman-8-yl)amino)-7H-pyrrolo[2,3-d]pyrimidin-4-yl)amino)quinoxalin-5-yl)phosphine oxide | | | |
| 127 | 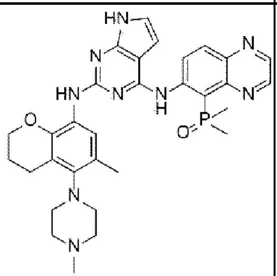 | dimethyl(6-((2-((6-methyl-5-(4-methylpiperazin-1-yl)chroman-8-yl)amino)-7H-pyrrolo[2,3-d]pyrimidin-4-yl)amino)quinoxalin-5-yl)phosphine | MS(ESI): m/z 598 $[M+H]^+$ | | |

| | | | | | |
|---|---|---|---|---|---|
| | | oxide | | | |
| 128 | 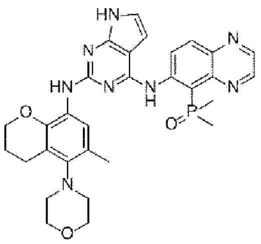 | dimethyl(6-((2-((6-methyl-5-morpholinochroman-8-yl)amino)-7H-pyrrolo[2,3-d]pyrimidin-4-yl)amino)quinoxalin-5-yl)phosphine oxide | MS(ESI): m/z 585 $[M+H]^+$ | | |
| 129 | 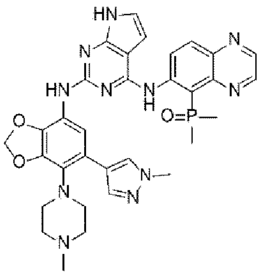 | dimethyl(6-((2-((6-(1-methyl-1H-pyrazol-4-yl)-7-(4-methylpiperazin-1-yl)benzo[d][1,3]dioxol- | $^1H$ NMR (400 MHz, TFA salt, Methanol-$d_4$) δ 9.49 (dd, *J* = 9.5, 4.2 Hz, 1H), 8.86 (d, *J* = 1.8 Hz, 1H), 8.79 (d, *J* = 1.8 Hz, 1H), 7.98 (s, 1H), 7.82-7.69 (m, 2H), 7.40 (s, 1H), 7.07 (d, *J* = 3.6 Hz, | 32 | 7.89, 99, (b) |

| | | | | | |
|---|---|---|---|---|---|
| | | 4-yl)amino)-7H-pyrrolo[2,3-d]pyrimidin-4-yl)amino)quinoxalin-5-yl)phosphine oxide | 1H), 6.71 (d, *J* = 3.5 Hz, 1H), 6.04 (s, 2H), 3.88 (s, 3H), 3.62-3.51 (m, 4H), 3.01 (s, 3H), 2.66 (s, 4H), 2.20 (s, 3H), 2.17 (s, 3H); MS(ESI): m/z 652 $[M+H]^+$ | | |
| 130 | 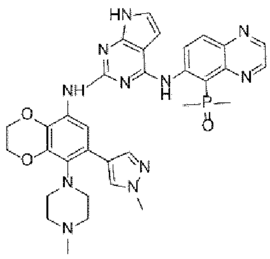 | dimethyl(6-((2-((7-(1-methyl-1H-pyrazol-4-yl)-8-(4-methylpiperazin-1-yl)-2,3-dihydrobenzo[b][1,4]dioxin-5-yl)amino)-7H-pyrrolo[2,3-d]pyrimidin- | $^1$H NMR (400 MHz, TFA salt, Methanol-$d_4$) δ 9.48 (dd, *J* = 9.4, 4.0 Hz, 1H), 8.85 (d, *J* = 1.9 Hz, 1H), 8.79 (d, *J* = 1.9 Hz, 1H), 7.89 (s, 1H), 7.83 (s, 1H), 7.64 (s, 1H), 7.61 (s, 1H), 7.06 (d, *J* = 3.6 Hz, 1H), 6.70 (d, *J* = 3.6 Hz, 1H), 4.40 (s, 4H), 3.85 (s, 3H), 3.54-3.49 (m, 2H), 3.30-3.20 (m, 6H), 2.99 (s, 3H), 2.21 (s, | 16 | 8.10, 100, (b) |

| | | | | | |
|---|---|---|---|---|---|
| | | 4-yl)amino)quinoxalin-5-yl)phosphine oxide | 3H), 2.17 (s, 3H); MS(ESI): m/z 665 [M+H]$^+$ | | |
| 131 | 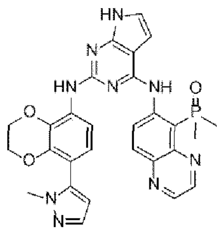 | dimethyl(6-((2-((8-(1-methyl-1H-pyrazol-5-yl)-2,3-dihydrobenzo[b][1,4]dioxin-5-yl)amino)-7H-pyrrolo[2,3-d]pyrimidin-4-yl)amino)quinoxalin-5-yl)phosphine oxide | $^1$H NMR (400 MHz, FA salt, DMSO-$d_6$) δ 13.30 (s, 1H), 11.55 (s, 1H), 9.81 (dd, *J* = 9.55 Hz, 1H), 8.87 (d, *J* = 1.84 Hz, 1H), 8.82 (d, *J* = 1.85 Hz, 1H), 8.41 (s, 0.3H), 8.19 (d, *J* = 9.52 Hz, 1H), 8.06 (d, *J* = 8.48 Hz, 1H), 7.84 (s, 1H), 7.44 (d, *J* = 1.76 Hz, 1H), 7.06 (q, 1H), 6.87 (d, *J* = 8.44 Hz, 1H), 6.53 (q, 1H), 6.26 (d, *J* = 1.80 Hz, 1H), 4.41 (t, 2H), 4.33 (t, 2H), 3.71 (s, 3H), 2.11 (s, 3H), | 32 | 10.63, 99, (b) |

| | | | | | |
|---|---|---|---|---|---|
| | | | 2.08 (s, 3H); MS(ESI): m/z 568 $[M+H]^+$ | | |
| 132 | 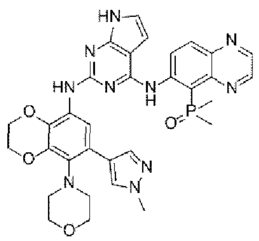 | dimethyl(6-((2-((7-(1-methyl-1H-pyrazol-4-yl)-8-morpholino-2,3-dihydrobenzo[b][1,4]dioxin-5-yl)amino)-7H-pyrrolo[2,3-d]pyrimidin-4-yl)amino)quinoxalin-5-yl)phosphine oxide | $^1H$ NMR (400 MHz, TFA salt, Methanol-$d_4$) δ 9.40-9.38 (m, 1H), 8.86 (d, $J$ = 2.0 Hz, 1H), 8.82 (d, $J$ = 2.0 Hz, 1H), 8.05 (s, 1H), 7.72 (s, 1H), 7.70 (s, 1H), 7.50 (s, 1H), 7.08 (d, $J$ = 3.6 Hz, 1H), 6.76 (d, $J$ = 3.2 Hz, 1H), 4.35 (s, 4H), 3.85 (s, 3H), 3.80 (s, 4H), 3.34-3.32 (m, 4H), 2.20 (s, 3H), 2.17 (s, 3H); MS(ESI): m/z 653 $[M+H]^+$ | 83 | 10.07, 99, (b) |

| | | | | | |
|---|---|---|---|---|---|
| 133 | 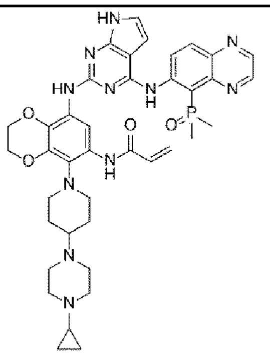 | N-(5-(4-(4-cyclopropylpiperazin-1-yl)piperidin-1-yl)-8-((4-((5-(dimethylphosphoryl)quinoxalin-6-yl)amino)-7H-pyrrolo[2,3-d]pyrimidin-2-yl)amino)-2,3-dihydrobenzo[b][1,4]dioxin-6-yl)acrylamide | $^1$H NMR (400 MHz, FA salt, MeOD-$d_4$) δ 9.73-9.69 (m, 1H), 8.94 (s, 1H), 8.80 (d, $J$ = 2 Hz, 1H), 8.71 (d, $J$ = 1.6 Hz, 1H), 8.05 (d, $J$ = 9.6 Hz, 1H), 7.01 (d, $J$ = 3.6 Hz, 1H), 6.60 (d, $J$ = 3.6 Hz, 1H), 6.45-6.38 (m, 1H), 6.11 (d, $J$ = 16.8 Hz, 1H), 5.67 (d, $J$ = 10.8 Hz, 1H), 4.64 (s, 4H), 4.38-4.33 (m, 4H), 3.49-3.47 (m, 3H), 3.22-2.92 (m, 12H), 2.20-2.14 (m, 8H), 1.86-1.82 (m, 3H), 0.57-0.56 (m, 2H), 0.47-0.46 (m, 2H); MS(ESI): m/z 764 [M+H]$^+$ | 20 | 8.51, 98, (b) |

| | | | | | |
|---|---|---|---|---|---|
| 134 | 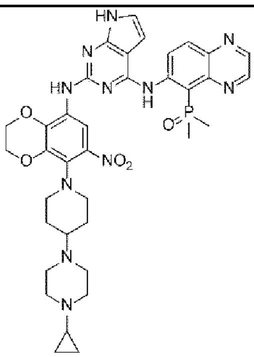 | (6-((2-((8-(4-(4-cyclopropylpiperazin-1-yl)piperidin-1-yl)-7-nitro-2,3-dihydrobenzo[b][1,4]dioxin-5-yl)amino)-7H-pyrrolo[2,3-d]pyrimidin-4-yl)amino)quinoxalin-5-yl)dimethylphosphine oxide | $^1$H NMR (400 MHz, FA salt, MeOD-$d_4$) δ 9.66-9.62 (m, 1H), 8.79 (d, $J$ = 2 Hz, 1H), 8.71 (d, $J$ = 1.6 Hz, 1H), 8.44 (s, 1H), 8.12 (d, $J$ = 9.6 Hz, 1H), 7.00 (d, $J$ = 3.6 Hz, 1H), 6.58 (d, $J$ = 3.6 Hz, 1H), 4.48 (d, $J$ = 2.4 Hz, 2H), 4.39 (s, 2H), 3.21-3.13 (m, 5H), 2.91 (s, 4H), 2.20 (s, 3H), 2.16 (s, 3H), 2.11-2.08 (m, 2H), 1.82-1.74 (m, 3H), 0.56 (d, $J$ = 5.2 Hz, 2H), 0.46 (d, $J$ = 2.4 Hz, 2H); MS(ESI): m/z 740 [M+H]$^+$ | 26 | 9.58, 98, (b) |
| 135 | 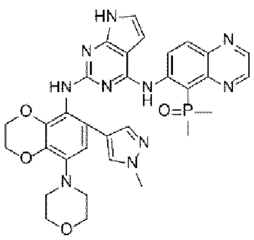 | dimethyl(6-((2-((6-(1-methyl-1H-pyrazol-4- | $^1$H NMR (400 MHz, FA salt, Methanol-$d_4$) δ 9.45 (s, 1H), 8.76 (d, $J$ = 2.0 Hz, 1H), 8.70 | 35 | 8.80, 99, (b) |

| | | | | | |
|---|---|---|---|---|---|
| | | yl)-8-morpholino-2,3-dihydrobenzo[b][1,4]dioxin-5-yl)amino)-7H-pyrrolo[2,3-d]pyrimidin-4-yl)amino)quinoxalin-5-yl)phosphine oxide | (d, *J* = 1.6 Hz, 1H), 8.23 (s, 1H), 7.85 (d, *J* = 10.0 Hz, 1H), 7.80 (s, 1H), 7.70 (s, 1H), 6.90 (d, *J* = 3.6 Hz, 1H), 6.85 (s, 1H), 6.52 (d, *J* = 3.6 Hz, 1H), 4.24 (d, *J* = 7.2 Hz, 4H), 3.92 (t, *J* = 4.4 Hz, 4H), 3.75 (s, 1H), 3.22 (t, *J* = 4.4 Hz, 4H), 2.14 (s, 3H), 2.11 (s, 3H); MS(ESI): m/z 653 $[M+H]^+$ | | |
| 136 | 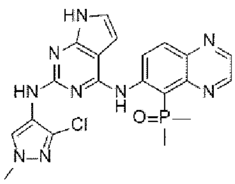 | (6-((2-((3-chloro-1-methyl-1H-pyrazol-4-yl)amino)-7H-pyrrolo[2,3-d]pyrimidin-4- | MS(ESI): m/z 468 $[M+H]^+$ | | |

| | | | | | |
|---|---|---|---|---|---|
| | | yl)amino)quinoxalin-5-yl)dimethylphosphine oxide | | | |
| 137 | 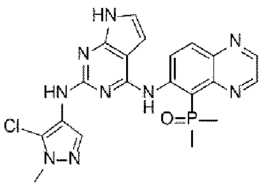 | (6-((2-((5-chloro-1-methyl-1H-pyrazol-4-yl)amino)-7H-pyrrolo[2,3-d]pyrimidin-4-yl)amino)quinoxalin-5-yl)dimethylphosphine oxide | $^{1}$H NMR (400 MHz, TFA salt, Methanol-$d_4$) δ 9.59 (s, 1H), 8.86 (d, $J$ = 2.0 Hz, 1H), 8.81 (d, $J$ = 1.6 Hz, 1H), 8.15 (d, $J$ = 9.2 Hz, 1H), 7.80 (s, 1H), 7.03 (d, $J$ = 3.2 Hz, 1H), 6.71 (d, $J$ = 3.6 Hz, 1H), 3.94 (s, 3H), 2.21 (s, 3H), 2.17 (s, 3H); MS(ESI): m/z 468 [M+H]$^+$ | 44 | 9.15, 99, (b) |
| 138 | 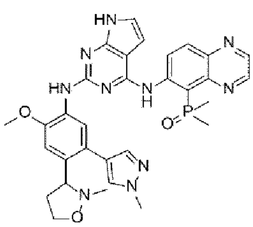 | (6-((2-((2-methoxy-5-(1-methyl-1H-pyrazol-4-yl)-4-(2- | $^{1}$H NMR (400 MHz, Methanol-$d_4$) δ 9.60-9.56 (m, 1H), 8.80 (d, $J$ = 1.9 Hz, 1H), 8.76 (d, $J$ = 1.9 Hz, 1H), | 30 | 9.29, 99, (b) |

| | | | | | |
|---|---|---|---|---|---|
| | | methylisoxazolidin-3-yl)phenyl)amino)-7H-pyrrolo[2,3-d]pyrimidin-4-yl)amino)quinoxalin-5-yl)dimethylphosphine oxide | 8.44 (s, 1H), 7.70-7.62 (m, 2H), 7.42 (s, 1H), 7.23 (s, 1H), 6.99 (d, *J* = 3.6 Hz, 1H), 6.61 (d, *J* = 3.6 Hz, 1H), 4.10 (t, *J* = 7.2 Hz, 2H), 4.01 (s, 3H), 3.96-3.93 (m, 1H), 3.90 (s, 3H), 2.79-2.71 (m, 1H), 2.51 (s, 3H), 2.36-2.29 (m, 1H), 2.19 (s, 3H), 2.16 (s, 3H); MS(ESI): m/z 625 $[M+H]^+$ | | |
| 139 | 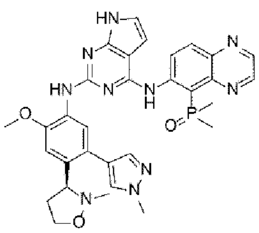 | (S)-(6-((2-((2-methoxy-5-(1-methyl-1H-pyrazol-4-yl)-4-(2-methylisoxazolidin-3-yl)phenyl)amino)-7H- | MS(ESI): m/z 625 $[M+H]^+$ | | |

| | | | | | |
|---|---|---|---|---|---|
| | | pyrrolo[2,3-d]pyrimidin-4-yl)amino)quinoxalin-5-yl)dimethylphosphine oxide | | | |
| 140 | 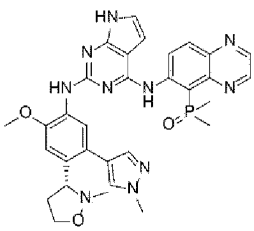 | (R)-(6-((2-((2-methoxy-5-(1-methyl-1H-pyrazol-4-yl)-4-(2-methylisoxazolidin-3-yl)phenyl)amino)-7H-pyrrolo[2,3-d]pyrimidin-4-yl)amino)quinoxalin-5-yl)dimethylphosphine | MS(ESI): m/z 625 $[M+H]^+$ | | |

| | | | | | |
|---|---|---|---|---|---|
| | | oxide | | | |
| 141 | 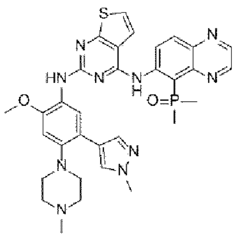 | (6-((2-((2-methoxy-5-(1-methyl-1H-pyrazol-4-yl)-4-(4-methylpiperazin-1-yl)phenyl)amino)thieno[2,3-d]pyrimidin-4-yl)amino)quinoxalin-5-yl)dimethylphosphine oxide | $^1$H NMR (400 MHz, TFA salt, Methanol-$d_4$) δ 9.51 (dd, $J$ = 9.6, 4.3 Hz, 1H), 8.81 (d, $J$ = 1.9 Hz, 1H), 8.76 (d, $J$ = 1.9 Hz, 1H), 8.10 (s, 1H), 7.99 (s, 1H), 7.76 (s, 1H), 7.56 (d, $J$ = 9.2 Hz, 1H), 7.49 (d, $J$ = 6.0 Hz, 1H), 7.19 (d, $J$ = 6.0 Hz, 1H), 6.89 (s, 1H), 3.95 (s, 3H), 3.81 (s, 3H), 3.01 (s, 4H), 2.68 (s, 4H), 2.42 (s, 3H), 2.19 (s, 3H), 2.15 (s, 3H); MS(ESI): m/z 655 [M+H]$^+$ | 8 | 9.38, 97, (b) |
| 142 | 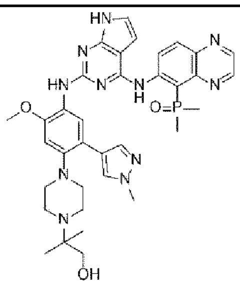 | (6-((2-((4-(4-(1-hydroxy-2-methylpropan | MS(ESI): m/z 696 [M+H]$^+$ | 68 | 8.04, 99, (b) |

| | | | | | |
|---|---|---|---|---|---|
| | | -2-yl)piperazin-1-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide | | | |

(*: When "*" is marked to the right side of the numerical value described in the last column, the numerical value means NMR purity)

**Experimental Example 1: Evaluation of various kinase inhibitory activities of compounds according to the present invention**

The following experiments were performed to evaluate the inhibitory activity of compounds according to the present invention against various enzymes. Specifically, with respect to the compound of Example 2 selected from the Example Compounds of the present invention, the enzyme (kinase) selectivity was measured by requesting DiscoverX company, and the experiment was conducted using a scanMAX™ Kinase analysis panel. Here, the concentration of the drug treated with the enzyme was 1 μM in DMSO, and the control percentage (% control) was determined by the following Equation 1.

[Equation 1]

(Example Compound - positive control) / (negative control - positive control) X 100

Here, the positive control indicates a compound showing the control percentage of 0%, and the negative control indicates DMSO showing the control percentage of 100%. Further, regarding the enzyme selectivity of the present invention, when the control percentage for each enzyme was <30% (i.e., less than 30%), the compound was determined to have the activity against the corresponding enzyme.

As a result, it could be confirmed that the compound of Example 54 had the control percentage of <30% (i.e., less than 30%) to the following enzymes, thereby having the activity against the following enzymes: EGFR, EGFR(E746-A750del), EGFR(G719C), EGFR(G719S), EGFR(L747-E749del, A750P), EGFR(L747-S752del, P753S), EGFR(L747-T751del, Sins), EGFR(L858R), EGFR(L858R, T790M), EGFR(L861Q), EGFR(S752-I759del), and EGFR(T790M).

**Experimental Example 2: Evaluation of Inhibitory activity on Ba/F3 cell proliferation**

The following experiments were performed to evaluate the inhibitory activity of the compound represented by Chemical Formula 1 according to the present invention on the proliferation of Ba/F3 cells expressing EGFR mutants.

BaF3-EGFR (WT) and BaF3-EGFR (Del19/T790M/C797S) cells were seeded in a clear bottom white 96-well plate at a density of $3 \times 10^3$/100 μl/well, and then 0.5 μl/well of the culture medium, containing the compounds at 12 concentrations (0.00001 - 2 mM) serially diluted in 3 folds and the DMSO control, was added to a final concentration of 0.00005 - 10 μM, followed by culturing in a 37°C $CO_2$ incubator for 72 hours. After 72 hours, the plates treated with the compounds were taken out, treated with 100 μl/well of CellTiter-Glo® 2.0 Assay (Promega) solution, and mixed well. After mixing well at room temperature for about 10 minutes, fluorescence was measured using a microplate reader. As to Data, $GI_{50}$ (μM) values were calculated using GraphPad Prism 8.3.0 (GraphPad software Inc., San Diego) as percentages in proportion to vehicle-based treated cells. The result values were calculated as a cell growth rate (%) compared to the control group. A graph was created using the GraphPad Prism version 5.0 program, and $GI_{50}$ (μM) values were calculated.

Table 2 below shows the evaluation results of the inhibitory activity on the proliferation of Ba/F3 cells expressing EGFR mutants.

[Table 2]

| Example | Enzyme ($IC_{50}$, μM) | Ba/F3 ($GI_{50}$, μM) | |
|---|---|---|---|
| | EGFR Del19/T790M/C797S | EGFR WT | EGFR Del19/T790M/C797S |
| 1 | A | C | B |
| 2 | A | C | A |
| 3 | A | C | C |
| 4 | A | C | B |
| 5 | B | B | C |
| 6 | A | C | A |
| 7 | - | C | C |
| 8 | A | C | A |
| 9 | A | C | C |
| 10 | - | C | A |
| 11 | - | C | C |
| 12 | - | C | C |
| 13 | A | C | C |
| 14 | - | C | C |

(continued)

| Example | Enzyme ($IC_{50}$, μM) | Ba/F3 ($GI_{50}$, μM) | |
|---|---|---|---|
| | EGFR Del19/T790M/C797S | EGFR WT | EGFR Del19/T790M/C797S |
| 21 | A | C | A |
| 22 | A | C | A |
| 23 | A | C | A |
| 24 | A | C | A |
| 25 | A | C | A |
| 26 | A | C | A |
| 27 | A | C | A |
| 28 | A | C | A |
| 29 | A | C | C |
| 30 | A | C | A |
| 31 | A | C | C |
| 32 | A | C | A |
| 33 | A | C | C |
| 34 | A | C | A |
| 35 | A | C | A |
| 36 | A | C | A |
| 40 | - | B | A |
| 41 | A | C | A |
| 42 | - | C | A |
| 43 | A | B | A |
| 44 | A | C | A |
| 47 | A | C | A |
| 48 | A | C | C |
| 49 | - | C | C |
| 50 | A | C | A |
| 51 | - | C | B |
| 52 | - | C | C |
| 53 | - | C | C |
| 54 | A | C | A |
| 55 | A | C | A |
| 56 | A | C | A |
| 57 | - | B | B |
| 58 | - | C | B |
| 59 | - | C | C |
| 60 | A | C | A |
| 61 | A | C | B |
| 62 | A | C | B |

(continued)

| Example | Enzyme ($IC_{50}$, $\mu M$) | Ba/F3 ($GI_{50}$, $\mu M$) | |
|---|---|---|---|
| | EGFR Del19/T790M/C797S | EGFR WT | EGFR Del19/T790M/C797S |
| 63 | - | C | C |
| 64 | - | C | C |
| 65 | - | C | C |
| 66 | - | C | C |
| 67 | - | C | C |
| 68 | A | C | C |
| 69 | C | C | C |
| 70 | A | C | C |
| 71 | A | C | C |
| 72 | A | C | C |
| 73 | A | B | C |
| 75 | A | C | C |
| 76 | A | C | C |
| 81 | A | C | C |
| 82 | A | C | C |
| 83 | A | C | C |
| 89 | A | C | C |
| 90 | | C | C |
| 104 | A | C | C |
| 105 | A | C | C |
| 106 | A | C | C |
| 109 | - | C | A |
| 111 | C | C | A |
| 112 | B | C | A |
| 113 | A | C | A |
| 114 | A | C | A |
| 115 | A | C | B |
| 116 | A | C | A |
| 117 | A | C | A |
| 120 | A | B | A |
| 121 | - | C | C |
| 122 | A | C | C |
| 129 | - | C | A |
| 130 | - | C | A |
| 131 | C | C | C |
| 133 | A | A | C |
| 134 | A | B | C |

(continued)

| Example | Enzyme ($IC_{50}$, μM) | Ba/F3 ($GI_{50}$, μM) | |
|---|---|---|---|
| | EGFR Del19/T790M/C797S | EGFR WT | EGFR Del19/T790M/C797S |
| 135 | C | C | C |
| 137 | A | C | C |

(A: $GI_{50} \leq 0.05$ μM; B: 0.05 μM < $GI_{50} \leq 0.1$ μM; C: 0.1 μM < $GI_{50}$)

## Claims

**1.** A compound represented by the following Chemical Formula 1, an optical isomer thereof, or a pharmaceutically acceptable salt thereof:

[Chemical Formula 1]

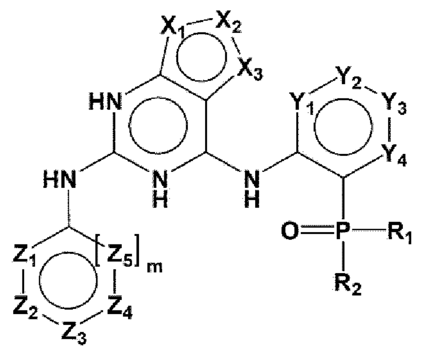

in Chemical Formula 1 above,

$X_1$ to $X_3$ are each independently $CR_aR_b$, $NR_c$, O, or S;

$R_a$ to $R_c$ are each independently -H, -$C_{1-6}$alkyl, -$C_{1-6}$aminoalkyl, -$C_{1-6}$hydroxyalkyl, -$C_{1-6}$haloalkyl, -CN, -$NH_2$, -OH, -O-$C_{1-6}$alkyl, -halo, -$C_{3-6}$cycloalkyl, or null;

$Y_1$ to $Y_4$ are each independently $CR_d$ or $NR_e$ {wherein any one of $Y_1$-$Y_2$, $Y_2$-$Y_3$, and $Y_3$-$Y_4$ may be linked to each other to form a 5-6 membered aryl, a 5-6 membered heteroaryl, or a 5-6 membered heterocycloalkyl [wherein at least one H of the aryl, heteroaryl, or heterocycloalkyl may be substituted with -$C_{1-6}$alkyl, -$C_{1-6}$aminoalkyl, -$C_{1-6}$hydroxyalkyl, -$C_{1-6}$haloalkyl, -CN, -$NR_3R_4$, -$OR_5$, -halo, or -$C_{3-6}$cycloalkyl]};

$R_d$ and $R_e$ are each independently -H, -$C_{1-6}$alkyl, -$C_{1-6}$aminoalkyl, -$C_{1-6}$hydroxyalkyl, -$C_{1-6}$haloalkyl, -CN, -$NR_3R_4$,-$OR_5$, -halo, -$C_{3-6}$cycloalkyl, or null;

$Z_1$ to $Z_5$ are each independently $CR_f$, $NR_g$ or N {wherein any one of $Z_1$-$Z_2$, $Z_2$-$Z_3$, $Z_3$-$Z_4$, and $Z_4$-$Z_5$ may be linked to each other to form a 5-6 membered aryl, a 5-6 membered heteroaryl or a 5-6 membered heterocycloalkyl [wherein at least one H of the aryl, heteroaryl, or heterocycloalkyl may be substituted with -$C_{1-6}$alkyl, -$C_{1-6}$aminoalkyl, -$C_{1-6}$hydroxyalkyl, -$C_{1-6}$haloalkyl, -CN, -$NR_3R_4$, -$OR_5$, -halo, or -$C_{3-6}$cycloalkyl]};

$R_f$ and $R_g$ are each independently -H, -$C_{1-6}$alkyl, -$C_{2-6}$alkenyl, -$C_{2-6}$alkynyl, -$C_{1-6}$aminoalkyl, -$C_{1-6}$hydroxyalkyl, - $C_{1-6}$haloalkyl, -CN, -$NR_3R_4$, -$NO_2$, -$OR_5$, -halo, aryl, heteroaryl, -$C_{3-6}$cycloalkyl, or heterocycloalkyl {wherein at least one H of the aryl or heteroaryl may be substituted with -$C_{1-6}$alkyl, -$C_{2-6}$alkenyl, -$C_{2-6}$alkynyl, -$C_{1-6}$aminoalkyl, -$C_{1-6}$hydroxyalkyl, -$C_{1-6}$haloalkyl, -$C_{3-6}$cycloalkyl, heterocycloalkyl, phenyl, or heteroaryl, and at least one H of the -$C_{3-6}$cycloalkyl or heterocycloalkyl may be substituted with -$C_{1-6}$alkyl, -$C_{1-6}$aminoalkyl, -$C_{1-6}$hydroxyalkyl, -$C_{1-6}$haloalkyl, -$C_{3-6}$cycloalkyl, -$C_{1-6}$alkyl-$C_{3-6}$cycloalkyl, -C(=O)-$C_{3-6}$cycloalkyl, or heterocycloalkyl (wherein at least one H of the -$C_{3-6}$cycloalkyl, -$C_{1-6}$alkyl-$C_{3-6}$cycloalkyl, -C (=O) -$C_{3-6}$cycloalkyl, or heterocycloalkyl ring may be substituted with -$C_{1-6}$alkyl, -$C_{1-6}$aminoalkyl, -$C_{1-6}$hydroxyalkyl, -$C_{1-6}$haloalkyl, -$C_{3-6}$cycloalkyl, or -halo)]};

m is 0 or 1;

$R_1$ and $R_2$ may each independently be -H, -$C_{1-6}$alkyl, -$C_{1-6}$alkoxy, -$C_{1-6}$alkylamino, -$C_{2-12}$dialkylamino, or $C_{3-6}$cycloalkyl, or the $R_1$ and $R_2$ may be linked to each other to form a 4- to 7-membered ring together with the P atom;

$R_3$ and $R_4$ are each independently -H, -$C_{1-6}$alkyl, -$C_{1-6}$alkyl-$NR_6R_7$, -$C_{1-6}$alkyl-O-$C_{1-6}$alkyl, -C(=O)-$C_{1-6}$alkyl, or - C (=O) -$C_{2-6}$alkenyl;
$R_5$ is -H, -$C_{1-6}$alkyl, -$C_{1-6}$alkyl-$NR_6R_7$, -$C_{1-6}$aminoalkyl, - $C_{1-6}$hydroxyalkyl, or -$C_{1-6}$haloalkyl; and
$R_6$ and $R_7$ are each independently -H or -$C_{1-6}$alkyl.

**2.** The compound, the optical isomer thereof, or the pharmaceutically acceptable salt thereof according to claim 1, wherein

$X_1$ to $X_3$ are each independently $CR_aR_b$, $NR_c$, or S;
$R_a$ to $R_c$ are each independently -H, -$C_{1-6}$alkyl, -$C_{1-6}$haloalkyl, -CN, -halo, -$C_{3-6}$cycloalkyl, or null;
$Y_1$ to $Y_4$ are each independently $CR_d$ or $NR_e$ {wherein any one of $Y_1$-$Y_2$, $Y_2$-$Y_3$, and $Y_3$-$Y_4$ may be linked to each other to form a 5-6 membered aryl, a 5-6 membered heteroaryl, or a 5-6 membered heterocycloalkyl [wherein at least one H of the aryl, heteroaryl, or heterocycloalkyl may be substituted with -$C_{1-6}$alkyl, -$C_{1-6}$haloalkyl, -halo, or -$C_{3-6}$cycloalkyl]};
$R_d$ and $R_e$ are each independently -H, -$C_{1-6}$alkyl, or null;
$Z_1$ to $Z_5$ are each independently $CR_f$, $NR_g$ or N {wherein any one of $Z_1$-$Z_2$, $Z_2$-$Z_3$, $Z_3$-$Z_4$, and $Z_4$-$Z_5$ may be linked to each other to form a 5-6 membered heterocycloalkyl [wherein at least one H of the heterocycloalkyl may be substituted with -$C_{1-6}$alkyl, -$C_{1-6}$haloalkyl, -halo, or -$C_{3-6}$cycloalkyl]};
$R_f$ and $R_g$ are each independently -H, -$C_{1-6}$alkyl, -$C_{2-6}$alkenyl, -$C_{2-6}$alkynyl, -$C_{1-6}$haloalkyl, -CN, -$NR_3R_4$, -$NO_2$, -$OR_5$, -halo, aryl, heteroaryl, -$C_{3-6}$cycloalkyl, or heterocycloalkyl {wherein at least one H of the aryl or heteroaryl may be substituted with -$C_{1-6}$alkyl, -$C_{1-6}$haloalkyl, or -$C_{3-6}$cycloalkyl, and at least one H of the -$C_{3-6}$cycloalkyl or heterocycloalkyl may be substituted with -$C_{1-6}$alkyl, -$C_{1-6}$hydroxyalkyl, -$C_{1-6}$haloalkyl, -$C_{3-6}$cycloalkyl, -$C_{1-6}$alkyl-$C_{3-6}$cycloalkyl, -C(=O)-$C_{3-6}$cycloalkyl, or heterocycloalkyl (wherein at least one H of the -$C_{3-6}$cycloalkyl, -$C_{1-6}$alkyl-$C_{3-6}$cycloalkyl, -C(=O)-$C_{3-6}$cycloalkyl, or heterocycloalkyl ring may be substituted with -$C_{1-6}$alkyl, -$C_{3-6}$cycloalkyl, or -halo)]};
m is 0 or 1;
$R_1$ and $R_2$ are each independently -$C_{1-6}$alkyl;
$R_3$ and $R_4$ are each independently -H, -$C_{1-6}$alkyl, -$C_{1-6}$alkyl-$NR_6R_7$, -$C_{1-6}$alkyl-O-$C_{1-6}$alkyl, -C(=O)-$C_{1-6}$alkyl, or - C (=O) -$C_{2-6}$alkenyl;
$R_5$ is -H, -$C_{1-6}$alkyl, -$C_{1-6}$alkyl-$NR_6R_7$, or -$C_{1-6}$haloalkyl; and
$R_6$ and $R_7$ are each independently -H or -$C_{1-6}$alkyl.

**3.** The compound, the optical isomer thereof, or the pharmaceutically acceptable salt thereof according to claim 1, wherein

$X_1$ is $CR_aR_b$, $NR_c$, or S;
$X_2$ is $CR_aR_b$;
$X_3$ is $CR_aR_b$, $NR_c$, or S; and
$R_a$ to $R_c$ are each independently -H, -$C_{1-6}$haloalkyl, -CN, -halo, -$C_{3-6}$cycloalkyl, or null.

**4.** The compound, the optical isomer thereof, or the pharmaceutically acceptable salt thereof according to claim 1, wherein

$Y_1$ to $Y_4$ are each independently $CR_d$ or $NR_e$ {wherein any one of $Y_1$-$Y_2$, $Y_2$-$Y_3$, and $Y_3$-$Y_4$ may be linked to each other to form a 5-6 membered aryl, a 5-6 membered heteroaryl, or a 5-6 membered heterocycloalkyl [wherein at least one H of the aryl, heteroaryl, or heterocycloalkyl may be substituted with -$C_{1-6}$alkyl, -$C_{1-6}$haloalkyl, -halo, or -$C_{3-6}$cycloalkyl]}; and
$R_d$ and $R_e$ are each independently -H, -$C_{1-6}$alkyl, or null.

**5.** The compound, the optical isomer thereof, or the pharmaceutically acceptable salt thereof according to claim 1, wherein

$Z_1$ to $Z_5$ are each independently $CR_f$, $NR_g$ or N {wherein $Z_1$-$Z_2$ may be linked to each other to form a 5-6 membered heterocycloalkyl [wherein at least one H of the heterocycloalkyl may be substituted with -$C_{1-6}$alkyl, -$C_{1-6}$haloalkyl, or -halo]};
$R_f$ and $R_g$ are each independently -H, -$C_{1-6}$alkyl, -$C_{2-6}$alkenyl, -$C_{2-6}$alkynyl, -$C_{1-6}$haloalkyl, -CN, -$NR_3R_4$, -$NO_2$, -$OR_5$, -halo, aryl, heteroaryl, -$C_{3-6}$cycloalkyl, or heterocycloalkyl {wherein at least one H of the aryl or heteroaryl may be substituted with -$C_{1-6}$alkyl, -$C_{1-6}$haloalkyl, -$C_{3-6}$cycloalkyl, heterocycloalkyl, phenyl, or heteroaryl, and

at least one H of the -$C_{3-6}$cycloalkyl or heterocycloalkyl may be substituted with -$C_{1-6}$alkyl, -$C_{1-6}$hydroxyalkyl, -$C_{1-6}$haloalkyl, -$C_{3-6}$cycloalkyl, -$C_{1-6}$alkyl-$C_{3-6}$cycloalkyl, -C(=O)-$C_{3-6}$cycloalkyl, or heterocycloalkyl (wherein at least one H of the -$C_{3-6}$cycloalkyl, -$C_{1-6}$alkyl-$C_{3-6}$cycloalkyl, -C (=O) -$C_{3-6}$cycloalkyl, or heterocycloalkyl ring may be substituted with -$C_{1-6}$alkyl, -$C_{3-6}$cycloalkyl, or -halo)]};
$R_3$ and $R_4$ are each independently -H, -$C_{1-6}$alkyl, -$C_{1-6}$alkyl-$NR_6R_7$, -$C_{1-6}$alkyl-O-$C_{1-6}$alkyl, -C(=O)-$C_{1-6}$alkyl, or - C (=O) -$C_{2-6}$alkenyl;
$R_5$ is -$C_{1-6}$alkyl, -$C_{1-6}$alkyl-$NR_6R_7$, or -$C_{1-6}$haloalkyl; and
$R_6$ and $R_7$ are each independently -H or -$C_{1-6}$alkyl.

**6.** The compound, the optical isomer thereof, or the pharmaceutically acceptable salt thereof according to claim 1, wherein
$R_1$ and $R_2$ are each independently -$C_{1-6}$alkyl.

**7.** The compound, the optical isomer thereof, or the pharmaceutically acceptable salt thereof according to claim 1, wherein
the compound represented by Chemical Formula 1 above is selected from the group consisting of the following compounds:

(1) dimethyl(6-((2-((5-(1-methyl-1H-pyrazol-4-yl)-4-(4-methylpiperazin-1-yl)-2-(2,2,2-trifluoroethoxy)phenyl)amino)-7H-pyrrolo[2,3-d]pyrimidin-4-yl)amino)quinoxalin-5-yl)phosphine oxide;
(2) dimethyl(6-((2-((5-(1-methyl-1H-pyrazol-4-yl)-4-morpholino-2-(2,2,2-trifluoroethoxy)phenyl)amino)-7H-pyrrolo[2,3-d]pyrimidin-4-yl) amino)quinoxalin-5-yl)phosphine oxide;
(3) (6-((2-((4-fluoro-5-(1-methyl-1H-pyrazol-4-yl)-2-(2,2,2-trifluoroethoxy)phenyl)amino)-7H-pyrrolo[2,3-d]pyrimidin-4-yl)amino)quinoxalin-5-yl)dimethylphosphine oxide;
(4) dimethyl(6-((2-((5-(1-methyl-1H-pyrazol-4-yl)-4-(4-(4-methylpiperazin-1-yl)piperidin-1-yl)-2-(2,2,2-trifluoroethoxy)phenyl)amino)-7H-pyrrolo[2,3-d]pyrimidin-4-yl)amino)quinoxalin-5-yl)phosphine oxide;
(5) dimethyl(6-((2-((5-(1-methyl-1H-pyrazol-4-yl)-2-(2,2,2-trifluoroethoxy)phenyl)amino)-7H-pyrrolo[2,3-d]pyrimidin-4-yl)amino)quinoxalin-5-yl)phosphine oxide;
(6) dimethyl(6-((2-((5-(1-methyl-1H-pyrazol-4-yl)-4-(4-morpholinopiperidin-1-yl)-2-(2,2,2-trifluoroethoxy)phenyl)amino)-7H-pyrrolo[2,3-d]pyrimidin-4-yl)amino)quinoxalin-5-yl)phosphine oxide;
(7) dimethyl(6-((2-((4-(4-methylpiperazin-1-yl)-2-(2,2,2-trifluoroethoxy)phenyl)amino)-7H-pyrrolo[2,3-d]pyrimidin-4-yl)amino)quinoxalin-5-yl)phosphine oxide;
(8) dimethyl(6-((2-((5-methyl-4-(4-(4-methylpiperazin-1-yl)piperidin-1-yl)-2-(2,2,2-trifluoroethoxy)phenyl)amino)-7H-pyrrolo[2,3-d]pyrimidin-4-yl)amino)quinoxalin-5-yl)phosphine oxide;
(9) dimethyl(6-((2-((5-methyl-4-(4-methylpiperazin-1-yl)-2-(2,2,2-trifluoroethoxy)phenyl)amino)-7H-pyrrolo[2,3-d]pyrimidin-4-yl) amino)quinoxalin-5-yl)phosphine oxide;
(10) (6-((2-((4-((2R,6S)-2,6-dimethylmorpholino)-5-(1-methyl-1H-pyrazol-4-yl)-2-(2,2,2-trifluoroethoxy)phenyl)amino)-7H-pyrrolo[2,3-d]pyrimidin-4-yl)amino)quinoxalin-5-yl)dimethylphosphine oxide;
(11) dimethyl(6-((2-((5-methyl-4-morpholino-2-(2,2,2-trifluoroethoxy)phenyl)amino)-7H-pyrrolo[2,3-d]pyrimidin-4-yl)amino)quinoxalin-5-yl)phosphine oxide;
(12) dimethyl(6-((2-((5-methyl-4-(4-morpholinopiperidin-1-yl)-2-(2,2,2-trifluoroethoxy)phenyl)amino)-7H-pyrrolo[2,3-d]pyrimidin-4-yl)amino)quinoxalin-5-yl)phosphine oxide;
(13) dimethyl(3-((2-((5-methyl-4-(4-(4-methylpiperazin-1-yl)piperidin-1-yl)-2-(2,2,2-trifluoroethoxy)phenyl)amino)-7H-pyrrolo[2,3-d]pyrimidin-4-yl)amino)quinolin-4-yl)phosphine oxide;
(14) dimethyl(3-((2-((5-(1-methyl-1H-pyrazol-4-yl)-4-(4-methylpiperazin-1-yl)-2-(2,2,2-trifluoroethoxy)phenyl)amino)-7H-pyrrolo[2,3-d]pyrimidin-4-yl)amino)quinolin-4-yl)phosphine oxide;
(15) (2-cyclopropyl-6-((2-((5-(1-methyl-1H-pyrazol-4-yl)-4-(4-methylpiperazin-1-yl)-2-(2,2,2-trifluoroethoxy)phenyl)amino)-7H-pyrrolo[2,3-d]pyrimidin-4-yl)amino)quinolin-5-yl)dimethylphosphine oxide;
(16) (2-cyclopropyl-6-((2-((5-methyl-4-(4-(4-methylpiperazin-1-yl)piperidin-1-yl)-2-(2,2,2-trifluoroethoxy)phenyl)amino)-7H-pyrrolo[2,3-d]pyrimidin-4-yl)amino)quinolin-5-yl)dimethylphosphine oxide;
(17) dimethyl(7-((2-((5-(1-methyl-1H-pyrazol-4-yl)-4-(4-methylpiperazin-1-yl)-2-(2,2,2-trifluoroethoxy)phenyl)amino)-7H-pyrrolo[2,3-d]pyrimidin-4-yl)amino)imidazo[1,2-a]pyridin-8-yl)phosphine oxide;
(18) dimethyl(7-((2-((5-methyl-4-(4-(4-methylpiperazin-1-yl)piperidin-1-yl)-2-(2,2,2-trifluoroethoxy)phenyl)amino)-7H-pyrrolo[2,3-d]pyrimidin-4-yl)amino)imidazo[1,2-a]pyridin-8-yl)phosphine oxide;
(19) dimethyl(4-methyl-7-((2-((5-(1-methyl-1H-pyrazol-4-yl)-4-(4-methylpiperazin-1-yl)-2-(2,2,2-trifluoroethoxy)phenyl)amino)-7H-pyrrolo[2,3-d]pyrimidin-4-yl)amino)-3,4-dihydro-2H-benzo[b] [1,4]oxazin-8-yl)phosphine oxide;
(20) dimethyl(4-methyl-7-((2-((5-methyl-4-(4-(4-methylpiperazin-1-yl)piperidin-1-yl)-2-(2,2,2-trifluoroethoxy)phenyl)amino)-7H-pyrrolo[2,3-d]pyrimidin-4-yl)amino)-3,4-dihydro-2H-benzo[b] [1,4]oxazin-8-yl)phosphine oxide;

(21) (6-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)quinoxalin-5-yl)dimethylphosphine oxide;

(22) (6-((2-((4-(4-cyclopentylpiperazin-1-yl)-2-methoxy-5-(1-methyl-1H-pyrazol-4-yl)phenyl)amino)-7H-pyrrolo[2,3-d]pyrimidin-4-yl) amino)quinoxalin-5-yl)dimethylphosphine oxide;

(23) (6-((2-((4-((2S,6R)-2,6-dimethylmorpholino)-2-methoxy-5-(1-methyl-1H-pyrazol-4-yl)phenyl)amino)-7H-pyrrolo[2,3-d]pyrimidin-4-yl) amino)quinoxalin-5-yl)dimethylphosphine oxide;

(24) (6-((2-((2-methoxy-5-(1-methyl-1H-pyrazol-4-yl)-4-(4-(4-methylpiperazin-1-yl)piperidin-1-yl)phenyl)amino)-7H-pyrrolo[2,3-d]pyrimidin-4-yl)amino)quinoxalin-5-yl)dimethylphosphine oxide;

(25) (6-((2-((2-methoxy-5-(1-methyl-1H-pyrazol-4-yl)-4-morpholinophenyl)amino)-7H-pyrrolo[2,3-d]pyrimidin-4-yl)amino)quinoxalin-5-yl)dimethylphosphine oxide;

(26) (6-((2-((4-((1S,4S)-2-oxa-5-azabicyclo[2.2.1]heptan-5-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(27) (6-((2-((4-((1R,4R))-2-oxa-5-azabicyclo[2.2.1]heptan-5-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(28) (6-((2-((2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(29) (6-((2-((2-methoxy-5-(1-methyl-1H-pyrazol-4-yl)-4-(2-oxa-6-azaspiro[3.3]heptan-6-yl)phenyl)amino)-7H-pyrrolo[2,3-d]pyrimidin-4-yl) amino)quinoxalin-5-yl)dimethylphosphine oxide;

(30) (6-((2-((4-((1R,5S)-8-oxa-3-azabicyclo[3.2.1]octan-3-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(31) (6-((2-((4-fluoro-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(32) (6-((2-((2-methoxy-5-(1-methyl-1H-pyrazol-4-yl)-4-(2-oxa-7-azaspiro[3.5]nonane-7-yl)phenyl)amino)-7H-pyrrolo[2,3-d]pyrimidin-4-yl) amino)quinoxalin-5-yl)dimethylphosphine oxide;

(33) (6-((2-((2-methoxy-5-(1-methyl-1H-pyrazol-4-yl)-4-(7-oxa-2-azaspiro[3.5]nonane-2-yl)phenyl)amino)-7H-pyrrolo[2,3-d]pyrimidin-4-yl) amino)quinoxalin-5-yl)dimethylphosphine oxide;

(34) dimethyl(6-((2-((3-(1-methyl-1H-pyrazol-4-yl)-4-morpholinophenyl)amino)-7H-pyrrolo[2,3-d]pyrimidin-4-yl)amino)quinoxalin-5-yl)phosphine oxide;

(35) (6-((2-((4-(2-(dimethylamino)ethoxy)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(36) (6-((2-((4-((2-(dimethylamino)ethyl)(methyl)amino)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(37) (6-((2-((2-methoxy-4-((2-methoxyethyl)(methyl)amino)-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(38) (6-((2-((2-methoxy-5-(1-methyl-1H-pyrazol-4-yl)-4-((3S,5R)-3,4,5-trimethylpiperazin-1-yl)phenyl)amino)-7H-pyrrolo[2,3-d]pyrimidin-4-yl) amino)quinoxalin-5-yl)dimethylphosphine oxide;

(39) (6-((2-((2-methoxy-5-(1-methyl-1H-pyrazol-4-yl)-4-(4-morpholinopiperidin-1-yl)phenyl)amino)-7H-pyrrolo[2,3-d]pyrimidin-4-yl) amino)quinoxalin-5-yl)dimethylphosphine oxide;

(40) dimethyl(6-((2-((3-(1-methyl-1H-pyrazol-4-yl)-4-(4-morpholinopiperidin-1-yl)phenyl)amino)-7H-pyrrolo[2,3-d]pyrimidin-4-yl)amino)quinoxalin-5-yl)phosphine oxide;

(41) (6-((2-((2-methoxy-5-(1-methyl-1H-pyrazol-4-yl)-4-((3aR,6aS)-tetrahydro-1H-furo[3,4-c]pyrrole-5(3H)-yl)phenyl)amino)-7H-pyrrolo[2,3-d]pyrimidin-4-yl)amino)quinoxalin-5-yl)dimethylphosphine oxide;

(42) (6-((2-((4-(4-cyclopropylpiperazin-1-yl)-2-methoxy-5-(1-methyl-1H-pyrazol-4-yl)phenyl)amino)-7H-pyrrolo[2,3-d]pyrimidin-4-yl) amino)quinoxalin-5-yl)dimethylphosphine oxide;

(43) (R)-(6-((2-((2-methoxy-5-(1-methyl-1H-pyrazol-4-yl)-4-(octahydro-2H-pyrido[1,2-a]pyrazin)-2-yl)phenyl)amino)-7H-pyrrolo[2,3-d]pyrimidin-4-yl)amino)quinoxalin-5-yl)dimethylphosphine oxide;

(44) (6-((2-((2-methoxy-5-(1-methyl-1H-pyrazol-4-yl)-6-(4-methylpiperazin-1-yl)pyridin-3-yl)amino)-7H-pyrrolo[2,3-d]pyrimidin-4-yl) amino)quinoxalin-5-yl)dimethylphosphine oxide;

(45) (6-((2-((3-methoxy-6-(1-methyl-1H-pyrazol-4-yl)-5-(4-methylpiperazin-1-yl)pyridin-2-yl)amino)-7H-pyrrolo[2,3-d]pyrimidin-4-yl) amino)quinoxalin-5-yl)dimethylphosphine oxide;

(46) (6-((2-((2-methoxy-5-(1-methyl-1H-pyrazol-4-yl)-6-(1-methylpiperidin-4-yl)pyridin-3-yl)amino)-7H-pyrrolo[2,3-d]pyrimidin-4-yl) amino)quinoxalin-5-yl)dimethylphosphine oxide;

(47) (6-((2-((5-(1-ethyl-1H-pyrazol-4-yl)-2-methoxy-4-(4-methylpiperazin-1-yl)phenyl)amino)-7H-pyrrolo[2,3-d]pyrimidin-4-yl)amino)quinoxalin-5-yl)dimethylphosphine oxide;

(48) (6-((2-((4-(4-ethylpiperazin-1-yl)-5-(1-isopropyl-1H-pyrazol-4-yl)-2-methoxyphenyl)amino)-7H-pyrrolo[2,3-d]pyrimidin-4-yl) amino)quinoxalin-5-yl)dimethylphosphine oxide;

(49) (6-((2-((5-(1-isopropyl-1H-pyrazol-4-yl)-2-methoxy-4-(4-methylpiperazin-1-yl)phenyl)amino)-7H-pyrrolo[2,3-d]pyrimidin-4-yl)amino)quinoxalin-5-yl)dimethylphosphine oxide;
(50) (6-((2-((5-(1-(difluoromethyl)-1H-pyrazol-4-yl)-2-methoxy-4-(4-methylpiperazin-1-yl)phenyl)amino)-7H-pyrrolo[2,3-d]pyrimidin-4-yl) amino)quinoxalin-5-yl)dimethylphosphine oxide;
(51) (6-((5-chloro-2-((2-methoxy-5-(1-methyl-1H-pyrazol-4-yl)-4-(4-methylpiperazin-1-yl)phenyl)amino)-7H-pyrrolo[2,3-d]pyrimidin-4-yl) amino)quinoxalin-5-yl)dimethylphosphine oxide;
(52) 4-((5-(dimethylphosphoryl)quinoxalin-6-yl)amino)-2-((2-methoxy-5-(1-methyl-1H-pyrazol-4-yl)-4-(4-methylpiperazin-1)-yl)phenyl)amino)-7H-pyrrolo[2,3-d]pyrimidin-5-carbonitrile;
(53) (6-((2-((2-methoxy-5-(1-methyl-1H-pyrazol-4-yl)-4-(4-methylpiperazin-1-yl)phenyl)amino)-5-(trifluoromethyl)-7H-pyrrolo[2,3-d]pyrimidin-4-yl)amino)quinoxalin-5-yl)dimethylphosphine oxide;
(54) (6-((2-((2-methoxy-5-methyl-4-(4-(4-methylpiperazin-1-yl)piperidin-1-yl)phenyl)amino)-7H-pyrrolo[2,3-d]pyrimidin-4-yl) amino)quinoxalin-5-yl)dimethylphosphine oxide;
(55) (6-((2-((5-ethyl-2-methoxy-4-(4-(4-methylpiperazin-1-yl)piperidin-1-yl)phenyl)amino)-7H-pyrrolo[2,3-d]pyrimidin-4-yl) amino)quinoxalin-5-yl)dimethylphosphine oxide;
(56) (6-((2-((5-chloro-2-methoxy-4-(4-(4-methylpiperazin-1-yl)piperidin-1-yl)phenyl)amino)-7H-pyrrolo[2,3-d]pyrimidin-4-yl) amino)quinoxalin-5-yl)dimethylphosphine oxide;
(57) (6-((2-((2-methoxy-4-(4-(4-methylpiperazin-1-yl)piperidin-1-yl)phenyl)amino)-7H-pyrrolo[2,3-d]pyrimidin-4-yl)amino)quinoxalin-5-yl)dimethylphosphine oxide;
(58) dimethyl(6-((2-((4-(4-(4-methylpiperazin-1-yl)piperidin-1-yl)phenyl)amino)-7H-pyrrolo[2,3-d]pyrimidin-4-yl)amino)quinoxalin-5-yl)phosphine oxide;
(59) (6-((2-((2-methoxy-5-methyl-4-(4-(4-methylpiperazin-1-yl)piperidin-1-yl)phenyl) amino)thieno[3,2-d]pyrimidin-4-yl)amino)quinoxalin-5-yl)dimethylphosphine oxide;
(60) (6-((2-((4-(4-(4-cyclopropylpiperazin-1-yl)piperidin-1-yl)-2-methoxy-5-methylphenyl)amino)-7H-pyrrolo[2,3-d]pyrimidin-4-yl) amino)quinoxalin-5-yl)dimethylphosphine oxide;
(61) (6-((2-((4-(4-(4-ethylpiperazin-1-yl)piperidin-1-yl)-2-methoxy-5-methylphenyl)amino)-7H-pyrrolo[2,3-d]pyrimidin-4-yl)amino)quinoxalin-5-yl)dimethylphosphine oxide;
(62) (6-((2-((4-(4-(4-isopropylpiperazin-1-yl)piperidin-1-yl)-2-methoxy-5-methylphenyl)amino)-7H-pyrrolo[2,3-d]pyrimidin-4-yl) amino)quinoxalin-5-yl)dimethylphosphine oxide;
(63) (6-((6-((2-methoxy-5-methyl-4-(4-(4-methylpiperazin-1-yl)piperidin-1-yl)phenyl)amino)-1H-pyrrolo[2,3-b]pyridin-4-yl) amino)quinoxalin-5-yl)dimethylphosphine oxide;
(64) (6-((6-((2-methoxy-4-(4-(4-methylpiperazin-1-yl)piperidin-1-yl)phenyl)amino)-1H-pyrrolo[2,3-b]pyridin-4-yl)amino)quinoxalin-5-yl)dimethylphosphine oxide;
(65) (6-((6-((3-methoxy-5-(4-(4-methylpiperazin-1-yl)piperidin-1-yl)pyridin-2-yl)amino)-1H-pyrrolo[2,3-b]pyridin-4-yl) amino)quinoxalin-5-yl)dimethylphosphine oxide;
(66) (6-((2-((5-fluoro-2-methoxy-4-(4-(4-methylpiperazin-1-yl)piperidin-1-yl)phenyl)amino)-7H-pyrrolo[2,3-d]pyrimidin-4-yl) amino)quinoxalin-5-yl)dimethylphosphine oxide;
(67) (6-((2-((5-isopropyl-2-methoxy-4-(4-(4-methylpiperazin-1-yl)piperidin-1-yl)phenyl)amino)-7H-pyrrolo[2,3-d]pyrimidin-4-yl) amino)quinoxalin-5-yl)dimethylphosphine oxide;
(68) (6-((2-((2-methoxy-5-methylphenyl)amino)-7H-pyrrolo[2,3-d]pyrimidin-4-yl) amino)quinoxalin-5-yl)dimethylphosphine oxide;
(69) (6-((2-((2-ethoxy-5-(trifluoromethyl)phenyl)amino)-7H-pyrrolo[2,3-d]pyrimidin-4-yl)amino)quinoxalin-5-yl)dimethylphosphine oxide;
(70) (6-((2-((2-methoxy-4-(4-methylpiperazin-1-yl)-5-(pyridin-4-yl)phenyl)amino)-7H-pyrrolo[2,3-d]pyrimidin-4-yl)amino)quinoxalin-5-yl)dimethylphosphine oxide;
(71) (6-((2-((2-methoxy-4-(4-methylpiperazin-1-yl)-5-(quinolin-3-yl)phenyl)amino)-7H-pyrrolo[2,3-d]pyrimidin-4-yl)amino)quinoxalin-5-yl)dimethylphosphine oxide;
(72) (6-((2-((4-methoxy-6-(4-methylpiperazin-1-yl)-[1,1'-biphenyl]-3-yl)amino)-7H-pyrrolo[2,3-d]pyrimidin-4-yl)amino)quinoxalin-5-yl)dimethylphosphine oxide;
(73) N-(5-((4-((5-(dimethylphosphoryl)quinoxalin-6-yl)amino)-7H-pyrrolo[2,3-d]pyrimidin-2-yl)amino)-4-methoxy-2-(4-methylpiperazin-1-yl)phenyl)acrylamide;
(74) N-(5-((4-((5-(dimethylphosphoryl)quinoxalin-6-yl)amino)-7H-pyrrolo[2,3-d]pyrimidin-2-yl)amino)-4-methoxy-2-(4-methylpiperazin-1-yl)phenyl)propionamide;
(75) (6-((2-((5-(furan-3-yl)-2-methoxy-4-(4-methylpiperazin-1-yl)phenyl)amino)-7H-pyrrolo[2,3-d]pyrimidin-4-yl)amino)quinoxalin-5-yl)dimethylphosphine oxide;
(76) (6-((2-((2-methoxy-4-(4-methylpiperazin-1-yl)-5-(thiophen-3-yl)phenyl)amino)-7H-pyrrolo[2,3-d]pyrimidin-4-yl)amino)quinoxalin-5-yl)dimethylphosphine oxide;
(77) (6-((2-((5-(isoxazol-4-yl)-2-methoxy-6-(4-methylpiperazin-1-yl)pyridin-3-yl)amino)-7H-pyrrolo[2,3-d]pyrimidin-4-yl)amino)quinoxalin-5-yl)dimethylphosphine oxide;

(78) (6-((2-((2-methoxy-6-(4-methylpiperazin-1-yl)-5-(oxazol-4-yl)pyridin-3-yl)amino)-7H-pyrrolo[2,3-d]pyrimidin-4-yl)amino)quinoxalin-5-yl)dimethylphosphine oxide;

(79) (6-((2-((5-ethynyl-2-methoxy-4-(4-methylpiperazin-1-yl)phenyl)amino)-7H-pyrrolo[2,3-d]pyrimidin-4-yl)amino)quinoxalin-5-yl)dimethylphosphine oxide;

(80) 5-((4-((5-(dimethylphosphoryl)quinoxalin-6-yl)amino)-7H-pyrrolo[2,3-d]pyrimidin-2-yl)amino)-6-methoxy-2-(4-methylpiperazin-1-yl)nicotinonitrile;

(81) 5-((4-((5-(dimethylphosphoryl)quinoxalin-6-yl)amino)-7H-pyrrolo[2,3-d]pyrimidin-2-yl)amino)-4-methoxy-2-(4-methylpiperazin-1-yl)benzonitrile;

(82) (6-((2-((2-methoxy-4-(4-methylpiperazin-1-yl)-5-vinylphenyl)amino)-7H-pyrrolo[2,3-d]pyrimidin-4-yl)amino)quinoxalin-5-yl)dimethylphosphine oxide;

(83) (6-((2-((5-cyclopropyl-2-methoxy-4-(4-methylpiperazin-1-yl)phenyl)amino)-7H-pyrrolo[2,3-d]pyrimidin-4-yl)amino)quinoxalin-5-yl)dimethylphosphine oxide;

(84) (6-((2-((5-ethyl-2-methoxy-6-(1-methylpiperidin-4-yl)pyridin-3-yl)amino)-7H-pyrrolo[2,3-d]pyrimidin-4-yl)amino)quinoxalin-5-yl)dimethylphosphine oxide;

(85) (6-((2-((5-ethyl-2-methoxy-6-(4-methylpiperazin-1-yl)pyridin-3-yl)amino)-7H-pyrrolo[2,3-d]pyrimidin-4-yl)amino)quinoxalin-5-yl)dimethylphosphine oxide;

(86) (6-((2-((5-ethyl-2-methoxy-6-(1'-methyl-[1,4'-bipiperidin]-4-yl)pyridin-3-yl)amino)-7H-pyrrolo[2,3-d]pyrimidin-4-yl)amino)quinoxalin-5-yl)dimethylphosphine oxide;

(87) (6-((2-((5-ethyl-2-methoxy-6-(4-(4-methylpiperazin-1-yl)piperidin-1-yl)pyridin-3-yl)amino)-7H-pyrrolo[2,3-d]pyrimidin-4-yl) amino)quinoxalin-5-yl)dimethylphosphine oxide;

(88) (6-((2-((5-(1-cyclopropyl-1H-pyrazol-4-yl)-2-methoxy-4-(4-methylpiperazin-1-yl)phenyl)amino)-7H-pyrrolo[2,3-d]pyrimidin-4-yl) amino)quinoxalin-5-yl)dimethylphosphine oxide;

(89) (3-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl) amino)quinolin-4-yl)dimethylphosphine oxide;

(90) (3-((2-((2-methoxy-5-methyl-4-(4-(4-methylpiperazin-1-yl)piperidin-1-yl)phenyl)amino)-7H-pyrrolo[2,3-d]pyrimidin-4-yl) amino)quinolin-4-yl)dimethylphosphine oxide;

(91) (2-cyclopropyl-6-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl) amino)quinolin-5-yl)dimethylphosphine oxide;

(92) (2-cyclopropyl-6-((2-((2-methoxy-5-methyl-4-(4-(4-methylpiperazin-1-yl)piperidin-1-yl)phenyl)amino)-7H-pyrrolo[2,3-d]pyrimidin-4-yl) amino)quinolin-5-yl)dimethylphosphine oxide;

(93) (7-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)imidazo[1,2-a]pyridin-8-yl)dimethylphosphine oxide;

(94) (7-((2-((2-methoxy-5-methyl-4-(4-(4-methylpiperazin-1-yl)piperidin-1-yl)phenyl)amino)-7H-pyrrolo[2,3-d]pyrimidin-4-yl)amino)imidazo[1,2-a]pyridin-8-yl)dimethylphosphine oxide;

(95) (7-((2-((2-methoxy-4-(4-(4-methylpiperazin-1-yl)piperidin-1-yl)phenyl)amino)-7H-pyrrolo[2,3-d]pyrimidin-4-yl)amino)imidazo[1,2-a]pyridin-8-yl)dimethylphosphine oxide;

(96) dimethyl(7-((2-((4-(4-(4-methylpiperazin-1-yl)piperidin-1-yl)phenyl)amino)-7H-pyrrolo[2,3-d]pyrimidin-4-yl)amino)imidazo[1,2-a]pyridin-8-yl)phosphine oxide;

(97) (7-((2-((2-methoxy-5-methyl-4-(4-(4-methylpiperazin-1-yl)piperidin-1-yl)phenyl) amino)thieno[3,2-d]pyrimidin-4-yl)amino)imidazo[1,2-a]pyridin-8-yl)dimethylphosphine oxide;

(98) (7-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)-4-methyl-3,4-dihydro-2H-benzo[b][1,4]oxazin-8-yl)dimethylphosphine oxide;

(99) (7-((2-((2-methoxy-5-methyl-4-(4-(4-methylpiperazin-1-yl)piperidin-1-yl)phenyl)amino)-7H-pyrrolo[2,3-d]pyrimidin-4-yl)amino)-4-methyl-3,4-dihydro-2H-benzo[b][1,4]oxazin-8-yl)dimethylphosphine oxide;

(100) (7-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl) amino)quinolin-8-yl)dimethylphosphine oxide;

(101) (6-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl) amino)quinolin-5-yl)dimethylphosphine oxide;

(102) (6-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)-2,3-dimethylphenyl)dimethylphosphine oxide;

(103) (3-((2-((2-methoxy-5-(1-methyl-1H-pyrazol-4-yl)-4-(4-methylpiperazin-1-yl)phenyl)amino)-7H-pyrrolo[2,3-d]pyrimidin-4-yl)amino)-1,5-naphthyridin-4-yl)dimethylphosphine oxide;

(104) (6-((2-((2-methoxy-5-methyl-4-(4-(4-methylpiperazin-1-yl)piperidin-1-yl)phenyl)amino)-9H-purin-6-yl)amino)quinoxalin-5-yl)dimethylphosphine oxide;

(105) (6-((2-((2-methoxy-5-(1-methyl-1H-pyrazol-4-yl)-4-(4-(4-methylpiperazin-1-yl)piperidin-1-yl)phenyl) amino)-9H-purin-6-yl) amino)quinoxalin-5-yl)dimethylphosphine oxide;

(106) dimethyl(6-((2-((3-(4-methyl-1H-imidazol-1-yl)-5-(trifluoromethyl)phenyl)amino)-7H-pyrrolo[2,3-d]pyrimidin-4-yl)amino)quinoxalin-5-yl)phosphine oxide;

(107) dimethyl(6-((2-((4-(4-(4-methylpiperazin-1-yl)piperidin-1-yl)-3-(trifluoromethyl)phenyl)amino)-7H-pyrrolo[2,3-d]pyrimidin-4-yl) amino)quinoxalin-5-yl)phosphine oxide;

(108) (6-((2-((4-(9-(1-fluoro-2-methylpropan-2-yl)-3,9-diazaspiro[5.5]undecan-3-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(109) (6-((2-((4-(4-(1-fluoro-2-methylpropan-2-yl)piperazin-1-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(110) (6-((2-((4-(9-(1-hydroxy-2-methylpropan-2-yl)-3,9-diazaspiro[5.5]undecan-3-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(111) (9-(4-((4-((5-(dimethylphosphoryl)quinoxalin-6-yl)amino)-7H-pyrrolo[2,3-d]pyrimidin-2-yl)amino)-5-methoxy-2-(1-methyl-1H-pyrazol-4-yl)phenyl)-3,9-diazaspiro[5.5]undecan-3-yl)(1-fluorocyclopropyl)methanone;

(112) (4-(4-((4-((5-(dimethylphosphoryl)quinoxalin-6-yl)amino)-7H-pyrrolo[2,3-d]pyrimidin-2-yl)amino)-5-methoxy-2-(1-methyl-1H-pyrazol-4-yl)phenyl)piperazin-1-yl) (1-fluorocyclopropyl)methanone;

(113) (6-((2-((4-(4-((1-fluorocyclopropyl)methyl)piperazin-1-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide;

(114) (6-((2-((4-(9-((1-fluorocyclopropyl)methyl)-3,9-diazaspiro[5.5]undecan-3-yl)-2-methoxy-5-(1-methyl-1H-pyrazol-4-yl)phenyl)amino)-7H-pyrrolo[2,3-d]pyrimidin-4-yl) amino)quinoxalin-5-yl)dimethylphosphine oxide;

(115) (6-((2-((5-(1-cyclopropyl-1H-pyrazol-4-yl)-2-methoxy-4-(4-methylpiperazin-1-yl)phenyl)amino)-7H-pyrrolo[2,3-d]pyrimidin-4-yl) amino)quinoxalin-5-yl)dimethylphosphine oxide;

(116) dimethyl(6-((2-((5-(1-methyl-1H-pyrazol-4-yl)-4-(4-methylpiperazin-1-yl)-2,3-dihydrobenzofuran-7-yl)amino)-7H-pyrrolo[2,3-d]pyrimidin-4-yl)amino)quinoxalin-5-yl)phosphine oxide;

(117) dimethyl(6-((2-((5-(1-methyl-1H-pyrazol-4-yl)-4-(4-(4-methylpiperazin-1-yl)piperidin-1-yl)-2,3-dihydrobenzofuran-7-yl)amino)-7H-pyrrolo[2,3-d]pyrimidin-4-yl)amino)quinoxalin-5-yl)phosphine oxide;

(118) (6-((2-((4-(4-cyclopentylpiperazin-1-yl)-5-(1-methyl-1H-pyrazol-4-yl)-2,3-dihydrobenzofuran-7-yl)amino)-7H-pyrrolo[2,3-d]pyrimidin-4-yl)amino)quinoxalin-5-yl)dimethylphosphine oxide;

(119) dimethyl(6-((2-((5-(1-methyl-1H-pyrazol-4-yl)-4-morpholino-2,3-dihydrobenzofuran-7-yl)amino)-7H-pyrrolo[2,3-d]pyrimidin-4-yl) amino)quinoxalin-5-yl)phosphine oxide;

(120) dimethyl(6-((2-((5-(1-methyl-1H-pyrazol-4-yl)-4-(4-morpholinopiperidin-1-yl)-2,3-dihydrobenzofuran-7-yl)amino)-7H-pyrrolo[2,3-d]pyrimidin-4-yl)amino)quinoxalin-5-yl)phosphine oxide;

(121) dimethyl(6-((2-((5-methyl-4-(4-methylpiperazin-1-yl)-2,3-dihydrobenzofuran-7-yl) amino)-7H-pyrrolo[2,3-d]pyrimidin-4-yl) amino)quinoxalin-5-yl)phosphine oxide;

(122) dimethyl(6-((2-((5-methyl-4-morpholino-2,3-dihydrobenzofuran-7-yl)amino)-7H-pyrrolo[2,3-d]pyrimidin-4-yl)amino)quinoxalin-5-yl)phosphine oxide;

(123) dimethyl(6-((2-((6-(1-methyl-1H-pyrazol-4-yl)-5-(4-methylpiperazin-1-yl)chroman-8-yl)amino)-7H-pyrrolo[2,3-d]pyrimidin-4-yl) amino)quinoxalin-5-yl)phosphine oxide;

(124) dimethyl(6-((2-((6-(1-methyl-1H-pyrazol-4-yl)-5-(4-(4-methylpiperazin-1-yl)piperidin-1-yl)chroman-8-yl)amino)-7H-pyrrolo[2,3-d]pyrimidin-4-yl)amino)quinoxalin-5-yl)phosphine oxide;

(125) dimethyl(6-((2-((6-(1-methyl-1H-pyrazol-4-yl)-5-morpholinochroman-8-yl)amino)-7H-pyrrolo[2,3-d]pyrimidin-4-yl)amino)quinoxalin-5-yl)phosphine oxide;

(126) dimethyl(6-((2-((6-(1-methyl-1H-pyrazol-4-yl)-5-(4-morpholinopiperidin-1-yl)chroman-8-yl)amino)-7H-pyrrolo[2,3-d]pyrimidin-4-yl) amino)quinoxalin-5-yl)phosphine oxide;

(127) dimethyl(6-((2-((6-methyl-5-(4-methylpiperazin-1-yl)chroman-8-yl)amino)-7H-pyrrolo[2,3-d]pyrimidin-4-yl)amino)quinoxalin-5-yl)phosphine oxide;

(128) dimethyl(6-((2-((6-methyl-5-morpholinochroman-8-yl)amino)-7H-pyrrolo[2,3-d]pyrimidin-4-yl)amino)quinoxalin-5-yl)phosphine oxide;

(129) dimethyl(6-((2-((6-(1-methyl-1H-pyrazol-4-yl)-7-(4-methylpiperazin-1-yl)benzo[d] [1,3]dioxol-4-yl)amino)-7H-pyrrolo[2,3-d]pyrimidin-4-yl)amino)quinoxalin-5-yl)phosphine oxide;

(130) dimethyl(6-((2-((7-(1-methyl-1H-pyrazol-4-yl)-8-(4-methylpiperazin-1-yl)-2,3-dihydrobenzo[b] [1,4]dioxin-5-yl)amino)-7H-pyrrolo[2,3-d]pyrimidin-4-yl)amino)quinoxalin-5-yl)phosphine oxide;

(131) dimethyl(6-((2-((8-(1-methyl-1H-pyrazol-5-yl)-2,3-dihydrobenzo[b][1,4]dioxin-5-yl)amino)-7H-pyrrolo[2,3-d]pyrimidin-4-yl) amino)quinoxalin-5-yl)phosphine oxide;

(132) dimethyl(6-((2-((7-(1-methyl-1H-pyrazol-4-yl)-8-morpholino-2,3-dihydrobenzo[b] [1,4]dioxin-5-yl)amino)-7H-pyrrolo[2,3-d]pyrimidin-4-yl)amino)quinoxalin-5-yl)phosphine oxide;

(133) N-(5-(4-(4-cyclopropylpiperazin-1-yl)piperidin-1-yl)-8-((4-((5-(dimethylphosphoryl)quinoxalin-6-yl)amino)-7H-pyrrolo[2,3-d]pyrimidin-2-yl)amino)-2,3-dihydrobenzo[b] [1,4]dioxin-6-yl)acrylamide;

(134) (6-((2-((8-(4-(4-cyclopropylpiperazin-1-yl)piperidin-1-yl)-7-nitro-2,3-dihydrobenzo[b] [1,4]dioxin-5-yl)amino)-7H-pyrrolo[2,3-d]pyrimidin-4-yl)amino)quinoxalin-5-yl)dimethylphosphine oxide;

(135) dimethyl(6-((2-((6-(1-methyl-1H-pyrazol-4-yl)-8-morpholino-2,3-dihydrobenzo[b][1,4]dioxin-5-yl)amino)-7H-pyrrolo[2,3-d]pyrimidin-4-yl)amino)quinoxalin-5-yl)phosphine oxide;
(136) (6-((2-((3-chloro-1-methyl-1H-pyrazol-4-yl)amino)-7H-pyrrolo[2,3-d]pyrimidin-4-yl)amino)quinoxalin-5-yl)dimethylphosphine oxide;
(137) (6-((2-((5-chloro-1-methyl-1H-pyrazol-4-yl)amino)-7H-pyrrolo[2,3-d]pyrimidin-4-yl)amino)quinoxalin-5-yl)dimethylphosphine oxide;
(138) (6-((2-((2-methoxy-5-(1-methyl-1H-pyrazol-4-yl)-4-(2-methylisoxazolidin-3-yl)phenyl)amino)-7H-pyrrolo[2,3-d]pyrimidin-4-yl) amino)quinoxalin-5-yl)dimethylphosphine oxide;
(139) (S)-(6-((2-((2-methoxy-5-(1-methyl-1H-pyrazol-4-yl)-4-(2-methylisoxazolidin-3-yl)phenyl)amino)-7H-pyrrolo[2,3-d]pyrimidin-4-yl) amino)quinoxalin-5-yl)dimethylphosphine oxide;
(140) (R)-(6-((2-((2-methoxy-5-(1-methyl-1H-pyrazol-4-yl)-4-(2-methylisoxazolidin-3-yl)phenyl)amino)-7H-pyrrolo[2,3-d]pyrimidin-4-yl) amino)quinoxalin-5-yl)dimethylphosphine oxide;
(141) (6-((2-((2-methoxy-5-(1-methyl-1H-pyrazol-4-yl)-4-(4-methylpiperazin-1-yl)phenyl)amino)thieno[2,3-d]pyrimidin-4-yl)amino)quinoxalin-5-yl)dimethylphosphine oxide; and
(142) (6-((2-((4-(4-(1-hydroxy-2-methylpropan-2-yl)piperazin-1-yl)-2-methoxy-5-(1-methyl-1H-pyrazol-4-yl)phenyl)amino)-7H-pyrrolo[2,3-d]pyrimidin-4-yl)amino)quinoxalin-5-yl)dimethylphosphine oxide.

**8.** A pharmaceutical composition for treating or preventing cancer, comprising the compound, the optical isomer thereof, or the pharmaceutically acceptable salt thereof according to any one of claims 1 to 7 as an active ingredient.

**9.** The pharmaceutical composition of claim 8, wherein
the pharmaceutical composition inhibits EGFR.

**10.** The pharmaceutical composition of claim 9, wherein
the pharmaceutical composition inhibits at least one selected from the group consisting of EGFR Del19/T790M, EGFR Del19/T790M/C797S, EGFR L858R/T790M, EGFR L858R, EGFR Exon20 ins NPH, EGFR Exon20 ins SVD, EGFR Exon20 ins FQEA, EGFR Exon20 ins H, and EGFR Exon20 ins ASV.

**11.** The pharmaceutical composition of claim 8, wherein
the cancer is at least one selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of vater cancer, bladder cancer, peritoneal cancer, parathyroid cancer, adrenal cancer, sinonasal cancer, non-small cell lung cancer, tongue cancer, astrocytoma, small cell lung cancer, childhood brain cancer, childhood lymphoma, childhood leukemia, small intestine cancer, meningioma, esophageal cancer, glioma, renal pelvic cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoma, gastrointestinal stromal cancer, Wilms' cancer, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoma, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleural cancer, hematological cancer, and thymic cancer.

**12.** Use of the compound, the optical isomer thereof, or the pharmaceutically acceptable salt thereof according to any one of claims 1 to 7 for use in the manufacture of a medicament for use in the treatment or prevention of cancer.

**13.** A method for treating or preventing cancer, comprising administering to a subject in need thereof a therapeutically effective amount of the compound, the optical isomer thereof, or the pharmaceutically acceptable salt thereof according to any one of claims 1 to 7.

## INTERNATIONAL SEARCH REPORT

International application No.
**PCT/KR2021/020165**

**A. CLASSIFICATION OF SUBJECT MATTER**

**C07D 487/04**(2006.01)i; **A61K 31/519**(2006.01)i; **A61P 35/00**(2006.01)i; **C07D 495/04**(2006.01)i

According to International Patent Classification (IPC) or to both national classification and IPC

**B. FIELDS SEARCHED**

Minimum documentation searched (classification system followed by classification symbols)

C07D 487/04(2006.01); A61K 31/505(2006.01); A61K 31/519(2006.01); A61K 31/675(2006.01); C07D 401/12(2006.01); C07D 495/04(2006.01); C07F 9/6561(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 헤테로아릴(heteroaryl), EGFR, 항암(anticancer), 수용체 티로신 키나아제(receptor tyrosine kinase)

**C. DOCUMENTS CONSIDERED TO BE RELEVANT**

| Category* | Citation of document, with indication, where appropriate, of the relevant passages | Relevant to claim No. |
|---|---|---|
| X | WO 2020-253862 A1 (SHANGHAI HANSOH BIOMEDICAL CO., LTD. et al.) 24 December 2020 (2020-12-24)<br>See abstract; claims 1, 7 and 41; example 54; and page 211, lines 25-29. | 1-12 |
| X | WO 2019-223777 A1 (BEIJING SCITECH-MQ PHARMACEUTICALS LIMITED) 28 November 2019 (2019-11-28)<br>See claims 1-14; and pages 48-49. | 1-12 |
| X | KR 10-2020-0026782 A (HONGYUN BIOTECH CO., LTD.) 11 March 2020 (2020-03-11)<br>See claims 1, 24, 26 and 27; and paragraph [0009]. | 1-12 |
| X | CN 110526941 A (BEIJING SAITE MINGQIANG MEDICINE TECHNOLOGY CO., LTD.) 03 December 2019 (2019-12-03)<br>See abstract; claims 1 and 13; and examples 1, 2 and 5-18. | 1-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 April 2022** | **11 April 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2021/020165**

**C. DOCUMENTS CONSIDERED TO BE RELEVANT**

| Category* | Citation of document, with indication, where appropriate, of the relevant passages | Relevant to claim No. |
|---|---|---|
| X | CN 111825719 A (BEIJING SCITECH-MQ PHARMACEUTICALS LTD.) 27 October 2020 (2020-10-27) See claims 1-13. | 1-12 |

Form PCT/ISA/210 (second sheet) (July 2019)

**INTERNATIONAL SEARCH REPORT**

International application No.
**PCT/KR2021/020165**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:

Claim 13 pertains to a method for treatment of the human body by surgery or therapy, and thus pertains to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/KR2021/020165**

| Patent document cited in search report | Publication date (day/month/year) | Patent family member(s) | Publication date (day/month/year) |
|---|---|---|---|
| WO 2020-253862 A1 | 24 December 2020 | CN 112469713 A | 09 March 2021 |
| | | CN 112513029 A | 16 March 2021 |
| | | TW 202115042 A | 16 April 2021 |
| | | WO 2020-253860 A1 | 24 December 2020 |
| WO 2019-223777 A1 | 28 November 2019 | CN 111836819 A | 27 October 2020 |
| KR 10-2020-0026782 A | 11 March 2020 | AU 2017-311168 A1 | 28 February 2019 |
| | | AU 2017-311168 B2 | 04 March 2021 |
| | | CA 3033459 A1 | 15 February 2018 |
| | | CN 107698603 A | 16 February 2018 |
| | | EP 3498716 A1 | 19 June 2019 |
| | | EP 3498716 A4 | 25 March 2020 |
| | | JP 2019-524790 A | 05 September 2019 |
| | | JP 6963598 B2 | 10 November 2021 |
| | | US 1078213 B2 | 03 August 2021 |
| | | US 2019-0211030 A1 | 11 July 2019 |
| | | US 2020-0207779 A2 | 02 July 2020 |
| | | WO 2018-028721 A1 | 15 February 2018 |
| CN 110526941 A | 03 December 2019 | None | |
| CN 111825719 A | 27 October 2020 | None | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Clin Cancer Res,* 2015, vol. 17, 21
- *Nature Medicine,* 2015, vol. 21, 560-562